(19) Europäisches Patentamt
European Patent Office
Office européen des brevets

(11) EP 3 904 362 A1

(12) EUROPEAN PATENT APPLICATION

(43) Date of publication:
**03.11.2021 Bulletin 2021/44**

(51) Int Cl.:
***C07F 9/66*** *(2006.01)* ***C07C 67/05*** *(2006.01)*

(21) Application number: **21179435.9**

(22) Date of filing: **10.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.04.2014 US 201414249704**
**26.03.2015 US 201562138823 P**

(62) Document number(s) of the earlier application(s) in accordance with Art. 76 EPC:
**15776299.8 / 3 129 347**

(71) Applicant: **California Institute of Technology**
**Pasadena, CA 91125 (US)**

(72) Inventors:
- **Marx, Vanessa M.**
  **Los Angeles, 90012 (US)**
- **Virgil, Scott C.**
  **Pasadena, 91107 (US)**
- **Grubbs, Robert H.**
  **South Pasadena, 91030 (US)**

(74) Representative: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

Remarks:
This application was filed on 15-06-2021 as a divisional application to the application mentioned under INID code 62.

# (54) REACTIONS IN THE PRESENCE OF RUTHENIUM COMPLEXES

(57) Described herein are compounds and methods for catalyzing metathesis reactions. The compounds described herein are ruthenium complexes bearing cyclic alkyl amino carbene (CAAC) ligands. The ruthenium complexes bearing cyclic alkyl amino carbene ligands may be used for catalyzing metathesis reactions, such as ethenolysis and alkenolysis reactions.

EP 3 904 362 A1

## Description

### Technical Field

This invention relates to olefin metathesis catalyst compounds, to the preparation of such compounds, and the use of such compounds in the metathesis of olefins and olefin compounds. The invention has utility in the fields of catalysis, organic synthesis, polymer chemistry, and industrial and fine chemicals industry.

### Statement of Government Support

This invention was made with government support under Grant No. GM031332 awarded by the National Institutes of Health and Grant No. CHE1048404 awarded by the National Science Foundation. The U.S. Government has certain rights in this invention.

### Background

The transformation of chemical feedstocks into industrially relevant small molecules has been a long-standing challenge that has received a significant resurgence of interest in the chemical sciences. This will enable the inexpensive preparation of a wide variety of useful products ranging from fuels to pharmaceuticals. Many inexpensive, renewable or bio-based materials, such as fatty acids originating from seed oils and their derivatives, contain at least one unit of unsaturation, providing a synthetic handle for derivatization by olefin metathesis catalysts. However, high catalyst loadings are needed in order to achieve good selectivity for terminal olefins, making this particular system cost-prohibitive on an industrial scale. There exists a need for systems capable of catalyzing ethenolysis reactions with high activity and selectivity on an industrial scale.

The transformation of small molecule chemical feedstocks to high-value chemicals has been a long-standing challenge that has received a significant resurgence of interest in the chemical sciences. This is a result of recently introduced programs promoting the use of greener chemistry practices, as well as the rising costs associated with the production of fine chemicals from petrochemicals. Consequently, the ability to access high-demand products from renewable sources such as oleochemicals presents a cost-effective and environmentally friendly alternative (Biermann, U.; Bornscheuer, U.; Meier, M. A. R.; Metzger, J. O.; Schäfer, H. J. Angew. Chem. 2011, 123, 3938; Angew. Chem. Int. Ed. 2011, 50, 3854. Montero de Espinosa, L.; Meier, M. A. R. Eur. Polym. J. 2011, 47, 837; Marshall, A.- L.; Alaimo, P. J. Chem. Eur. J. 2010, 16, 4970; Meier, M. A. R.; Metzger, J. O.; Schubert. U. S. Chem. Soc. Rev. 2007, 36, 1788; Sheldon, R. A. Green Chem. 2007, 9, 1273; Sheldon, R. A.; Arends, I.; Hanefeld, U. (eds) Green Chemistry and Catalysis, Wiley-VCH. Weinheim: 2007; Corma, A.; Iborra, S.; Velty, A. Chem. Rev. 2007, 107, 2411.)

Olefin metathesis reactions, such as cross-metathesis (CM), ring-closing metathesis (RCM), and ring-opening metathesis polymerization (ROMP), all of which generate a new internal olefin, have enjoyed widespread popularity in both academic and industrial settings as a result of their general applicability, ease of use, and non-prohibitive costs. (Cossy, J.; Arseniyadis, S.; Meyer, C. (eds) Metathesis in Natural Product Synthesis. Wiley-VCH, Weinhem: 2010; Lozano-Vila, A. M.; Monsaert, S.; Bajek, A.; Verpoort, F. Chem. Rev. 2010, 110, 4865; Vougioukalakis, G.; Grubbs, R. H. Chem. Rev. 2010, 110, 1746; Samojlowicz, C.; Bieniek, M.; Grela, K. Chem. Rev. 2009, 109, 3708; Schrodi, Y.; Pederson, R. L. Aldrichim. Acta. 2007, 40, 45. Nicolaou, K. C.; Bulger, P. G.; Sarlah, D. Angew. Chem. 2005, 117, 4564; Angew. Chem. Int. Ed. 2005, 44, 4490; Grubbs, R. H. (ed) Handbook of Metathesis (vol 2). Wiley-VCH, Weinhem: 2003; Schrock R. R.; Hoveyda A. H. Angew. Chem. 2003, 115, 4740; Angew. Chem. Int. Ed. 2003, 42, 4592; Schrock, R. R. Chem. Rev 2002, 102, 145; Trnka T. M.; Grubbs R. H. Acc. Chem. Res. 2001, 34, 18; Fürstner A. Angew. Chem. 2000, 112, 3140; Angew. Chem. Int. Ed. 2000, 39, 3012.)

Ruthenium-based metathesis catalysts are ideal for such transformations as a result of their generally robust nature, which enables handling in air, and imparts good tolerance to a variety of functional groups and trace impurities. All of these are necessary prerequisites when subjected to raw materials or biomass.

Many renewable or bio-based materials, such as fatty acids originating from seed oils and their derivatives, contain at least one unit of unsaturation, providing a synthetic handle for derivatization by olefin metathesis catalysts. Ethenolysis, the cross metathesis reaction of an internal olefin with ethylene to generate two terminal olefin products, has long been proposed as a method for the rapid production of commodity, value-added products from inexpensive, readily-available seed oil derivatives. However, despite extensive experimentation, a catalyst has yet to be developed that displays both the high levels of activity and selectivity necessary for the sustainability of this transformation on an industrial scale. (Schrodi, Y.; Ung, T.; Vargas, A.; Mkrtumyan, G.; Lee, C. -W.; Champagne, T.; Pederson, R. L.; Hong, S. K. Clean. 2008, 36, 669 - 673. Marinescu, S. C.; Schrock, R. R.; Müller, P.; Hoveyda, A. H. J. Am. Chem. Soc. 2009, 131, 10840 - 10841. Thomas, R. M.; Keitz, B. K.; Champagne, T. M.; Grubbs, R. H. J. Am. Chem. Soc. 2011, 133, 7490 - 7496). Previously, the best result for this transformation achieved in our laboratories was with catalyst **C578** (**diEt**)

(**Scheme 5**), which provided ethenolysis products in 35% yield (83% selectivity) using catalyst loadings of 10 ppm (TON 35,000), for the ethenolysis of methyl oleate (**1**[a]) to 1-decene (**2**[a]) and methyl-10-undecenoate (**3**[a]) (**Scheme 1**[a]). (Schrodi, Y.; Ung, T.; Vargas, A.; Mkrtumyan, G.; Lee, C. -W.; Champagne, T.; Pederson, R. L.; Hong, S. K. Clean. 2008, 36, 669 - 673. Marinescu, S. C.; Schrock, R. R.; Müller, P.; Hoveyda, A. H. J. Am. Chem. Soc. 2009, 131, 10840 - 10841. Thomas, R. M.; Keitz, B. K.; Champagne, T. M.; Grubbs, R. H. J. Am. Chem. Soc. 2011, 133, 7490 - 7496; More recent optimization experiments have provided ethenolysis products in 41% yield (86% selectivity), with a TON of 44,000 (10 ppm catalyst loading)). However, for application to commodity chemicals, the TON should be at least 50,000. Herein we present ruthenium complexes such as **C578 (Me, *i*Pr)** or **C782** (**Scheme 5**), which bear cyclic alkyl amino carbene (CAAC) ligands with an *ortho* methyl substituent on the *N*-aryl ring, as potentially viable catalyst systems for ethenolysis reactions on an industrial scale.

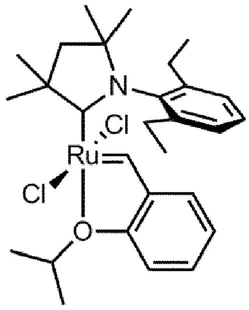

**C578 (diEt)**

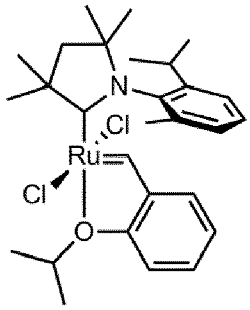

**C578 (Me, *i*Pr)**

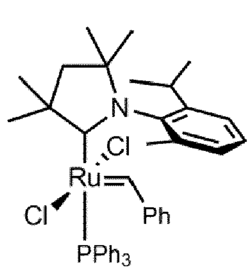

**C782**

**Scheme 5.** Ruthenium complexes bearing CAAC ligands

The CM reaction (**Scheme 6**) with ethylene (2), commonly referred to as ethenolysis, has significant potential as a clean, scalable, and sustainable solution for the production of linear alpha olefins (LAOs) (*eg.* 3 and 4) from the natural oils found in oleochemicals such as methyl oleate (MO, 1) (**Scheme 1**).

**Scheme 1.** Ethenolysis of the seed oil derivative methyl oleate (1)

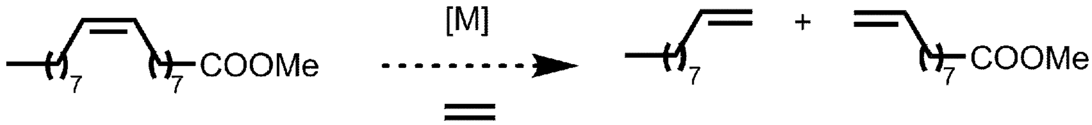


**1** **2** **3** **4**

**Scheme 6**. Selected ruthenium metathesis catalysts previously studied for ethenolysis

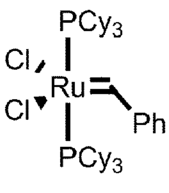

**5**

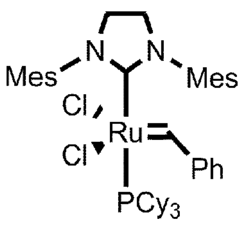

**6**

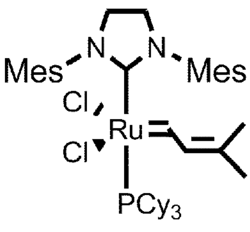

**7**

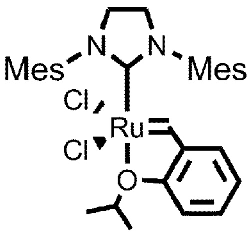

**8**

The production of linear alpha olefins (LAOs) from the ethenolysis of seed oil derivatives has been demonstrated. For example, catalyst Ru-4:

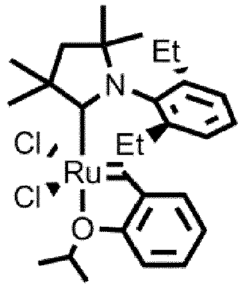

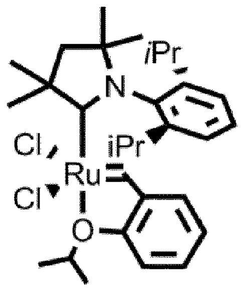

**Ru-4** **Ru-6**

provided ethenolysis products in 35% yield (83% selectivity) using catalyst loadings of 10 ppm (turnover number (TON) = 35,000), for the ethenolysis of methyl oleate (**1**$^a$) to 1-decene (**2**$^a$) and methyl-10-undecenoate (**3**$^a$) (**Scheme 1**$^a$):

Scheme 1$^a$

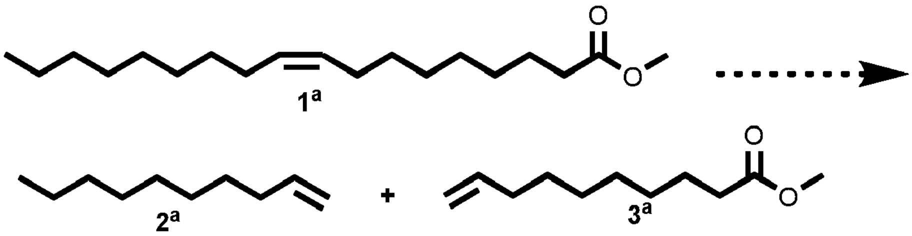

**1**$^a$

**2**$^a$ + **3**$^a$

LAOs are direct precursors to a variety of commodity chemicals with applications as fuels, surfactants, lubricants, waxes, perfumes, antimicrobial agents, and thermoplastics. In addition, LAOs can be rapidly elaborated to more expensive products such as agrochemicals, insect pheromones, and pharmaceuticals. (Chikkali, S.; Meckling, S. Angew. Chem. 2012, 124, 5902; Angew. Chem. Int. Ed. 2012, 51, 5802; Montero de Espinosa, L.; Meier, M. A. R. Top. Organomet. Chem. 2012, 39, 1; Raluca, M.; Dixneuf, P. H. In Green Metathesis Chemistry, 185. Springer Netherlands: 2010. Mol, J. C. Green Chem. 2002, 4, 5.)

The production of terminal olefins from the ethenolysis of seed oil derivatives using metathesis catalysts has been previously demonstrated. However, the high catalyst loadings required (10 - 100 ppm) to achieve an acceptable yield of terminal olefins render these reported procedures cost prohibitive on an industrial scale. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669. Anderson, D. R.; Ung, T.; Mkrtumyan. G.; Bertrand, G.; Grubbs, R. H.; Schrodi, Y. Organometallics. 2008, 27, 563. Anderson, D. R.; Lavallo, V.; O'Leary, D. J.; Bertrand, G.; Grubbs, R. H. Angew. Chem. 2007, 119, 7400; Angew. Chem. Int. Ed. 2007, 46, 7262, Zhang, J.; Song, S.; Wang, X.; Jiaoa, J.; Shi, M. Chem. Commun. 2013, 49, 9491; van der Klis, F.; Le Nôtre, J.; Blaauw, R.; van Haveren, J.; van Es, D. S. Eur. J. Lipid. Sci. Technol. 2012, 114, 911; Cohen, S. A.; Luetkens, M. L.; Balakrishnan, C.; Snyder, R (Elevance Renewable Sciences), WO2011/046872 A2, 2011; Thomas, R. M.; Keitz, B. K.; Champagne, T.; Grubbs, R. H. J. Am. Chem. Soc.2011, 133, 7490; Hagadorn, J. R.; Holtcamp, M. W.; Bedoya, M. S. (Exxonmobile), WO2011100022 A3, 2011; Mignani, G.; Pevere, V.; Olivier-Bourbigou, H.; Vallee, C.; Berthod, M.; Citadelle, C. (Rhodia Operations, IFP), WO2010/010290 A1, 2010; Marinescu, S. C.; Schrock, R. R.; Müller, P.; Hoveyda, A. H. J. Am. Chem. Soc. 2009, 131, 10840; Schrodi, Y.; Pederson, R. L.; Kaido, H.; Tupy, M. J. (Elevance Renewable Sciences), WO2008/046106 A2, 2008; Burdett, K. A.; Harris, L. D.; Margl, P.; Maughon, B. R.; Mokhtar-Zadeh, T.; Saucier, P. C.; Wasserman, E. P. Organometallics 2004, 23, 2027.)

In general, catalyst turnover numbers (TONs) of at least 35,000 and 50,000 are recommended in the manufacturing of specialty and commodity chemicals, respectively. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669.) In the ethenolysis of the benchmark substrate MO (1), standard ruthenium-based metathesis catalysts such as 5 - 8 afforded TONs of only 2,000 - 5,000. This stands in contrast to the extremely high activity normally exhibited by these catalysts in CM with terminal or internal olefins. For example, TONs as high as 470,000 have been achieved with 8 in the CM of MO and 2-butene. (Patel, J.; Mujcinovic, S.; Jackson, W. R.; Robinson, A. J.; Serelis, A. K.; Such, C. Green Chem. 2006, 8, 450). The most active catalyst for ethenolysis in the literature to date is cyclic alkyl amino carbene

(CAAC) complex 10 (**Scheme 2**), which has been previously reported to generate a TON of 35,000 in the CM of MO with ethylene. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669. Anderson, D. R.; Ung, T.; Mkrtumyan. G.; Bertrand, G.; Grubbs, R. H.; Schrodi, Y. Organometallics. 2008, 27, 563. Anderson, D. R.; Lavallo, V.; O'Leary, D. J.; Bertrand, G.; Grubbs, R. H. Angew. Chem. 2007, 119, 7400; Angew. Chem. Int. Ed. 2007, 46, 7262.)

**Scheme 2.** Synthesis of CAAC complexes under study (isolated yield in brackets).

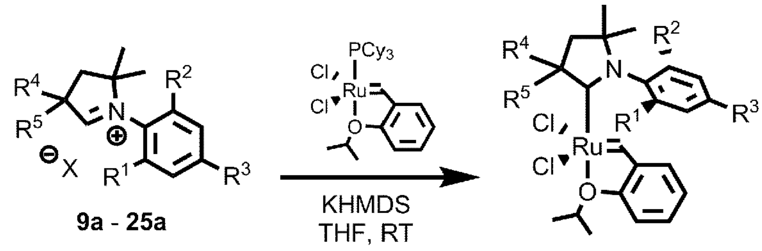


**9a** - **25a**

**9** - **25**

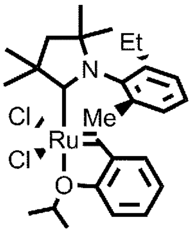

**9** *(15%)*

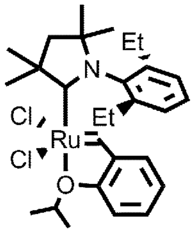

**10** *(18%)*

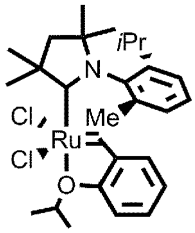

**11** *(78%)*

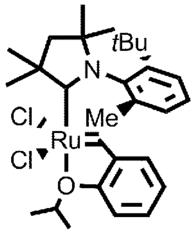

**12** *(86%)*

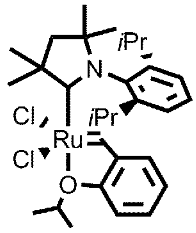

**13** *(85%)*

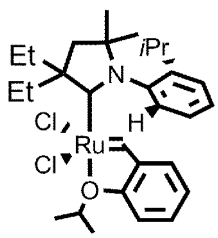

**14** *(44%)*

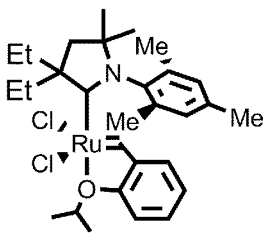

**15** *(48%)*

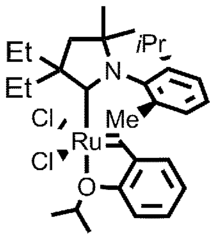

**16** *(82%)*

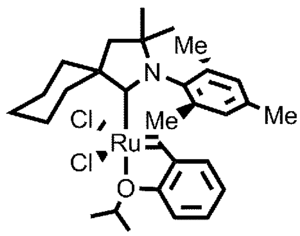

**17** *(39%)*

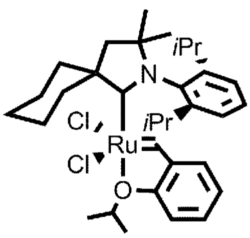

**18** *(77%)*

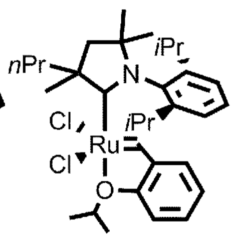

**19** *(79%)*

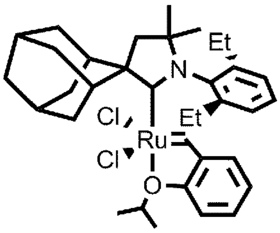

**20** *(81%)*

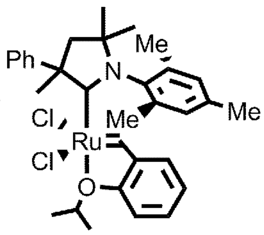

**21** *(29%)*

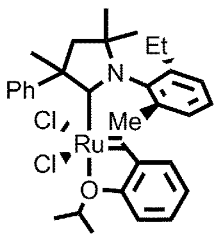

**22** *(32%)*

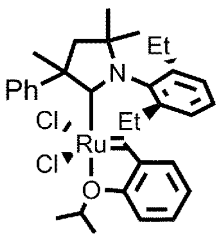

**23** *(45%)*

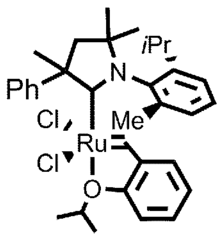

**24** *(58%)*

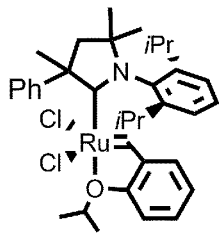

**25** *(70%)*

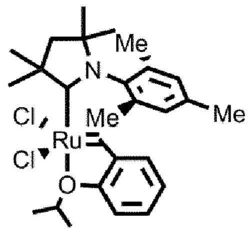
**Ru-7**

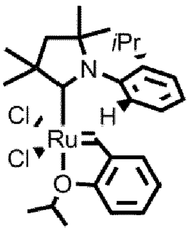
**Ru-8**

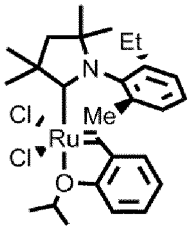
**Ru-9**

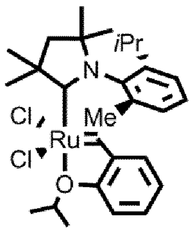
**Ru-10**

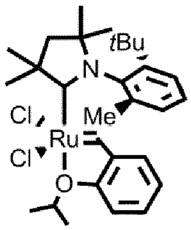
**Ru-11**

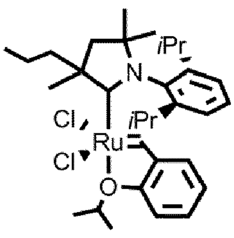
**Ru-12**

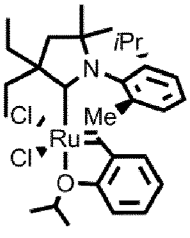
**Ru-13**

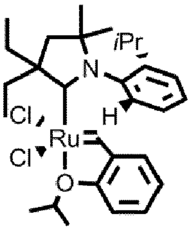
**Ru-14**

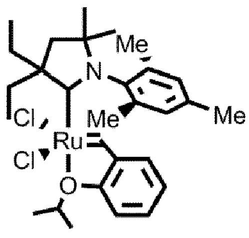
**Ru-15**

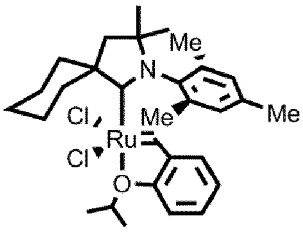
**Ru-16**

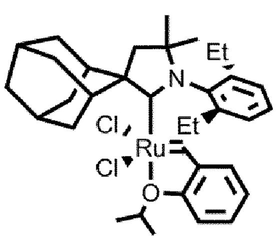
**Ru-17**

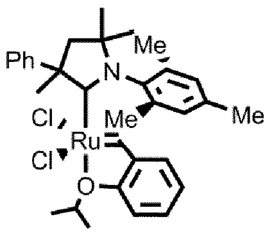
**Ru-18**

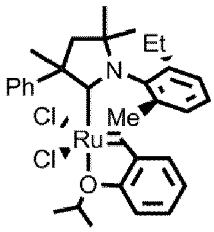
**Ru-19**

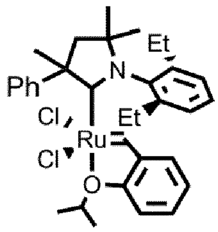
**Ru-20**

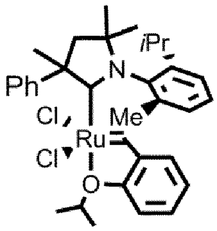
**Ru-21**

wherein: KHMDS is potassium bis(trimethylsilyl)amide , THF is tetrahydrofuran, RT is room temperature.

In the present patent application, the CAAC Ruthenium catalysts may be referred by two or more notations, as follows: **Ru-9** is **9, Ru-4** is **10, Ru-10** is **11** or **C578** or **C578 (Me, *i*Pr)**, **Ru-11** is **12, Ru-6** is **13,** Ru-14 is 14, Ru-15 is 15, Ru-13 is 16, Ru-16 is 17, Ru-12 is 19, Ru-17 is 20, Ru-18 is 21, Ru-19 is 22, Ru-20 is 23, Ru-21 is 24.

As a result of the lack of a catalyst sufficiently active to produce teminal olefins using ethylene gas, industrial scale ethenolysis is currently accomplished using higher olefins as ethylene surrogates. (van der Klis, F.; Le Nôtre, J.; Blaauw, R.; van Haveren, J.; van Es, D. S. Eur. J. Lipid. Sci. Technol. 2012, 114, 911). Catalyst 7, for example, is able to achieve the *in situ* ethenolysis of MO with a TON as high as 192,900 with propylene gas. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518). However, there is a need to develop catalysts capable of achieving high activity in ethenolysis reactions when ethylene gas is utilized directly.

Whereas CM with higher olefins necessarily results in a substantial amount of undesired internal olefins being produced as byproducts, the only products derived from CM with ethylene are terminal olefins. This intrinsic advantage promotes both increased yield and ease of purification of the desired terminal olefin products, and is a particularly important consideration for bio-refinery feedstocks in which multiple downstream products are produced. (Chikkali, S.; Meckling, S. Angew. Chem. 2012, 124, 5902; Angew. Chem. Int. Ed. 2012, 51, 5802; Montero de Espinosa, L.; Meier, M. A. R. Top. Organomet. Chem. 2012, 39, 1; Raluca, M.; Dixneuf, P. H. In Green Metathesis Chemistry, 185. Springer Netherlands: 2010. Mol, J. C. Green Chem. 2002, 4, 5.)

## Summary of the Invention

A metathesis catalyst system that is both highly active, and selective, for ethenolysis reactions is described. When applied to the ethenolysis of methyl oleate, catalyst **C578 (Me, *i*Pr)** provided 1-decene and methyl-10-undecenoate in 32% yield (92% selectivity), exhibiting turn over numbers (TON) of 106,000. This is the most active catalyst for this transformation to date, and shows promise for future application towards the transformation of seed oil derivatives into commodity materials on an industrial scale, for the first time.

Herein, we report the discovery of the most active ethenolysis catalysts to date. In many cases, TONs surpassing 100,000 were achieved for the ethenolysis of MO, using ethylene gas. Remarkably, certain catalyst systems even exhibited TONs approaching 200,000, with the highest TON achieved being 330,000. This represents the first time that

reaction conditions have been developed in order for ethenolysis to proceed sustainably on an industrial scale.

Despite the promising results previously exhibited by 10 in the ethenolysis of MO CAAC ligated ruthenium complexes have yet to be investigated in detail. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669. Anderson, D. R.; Ung, T.; Mkrtumyan. G.; Bertrand, G.; Grubbs, R. H.; Schrodi, Y. Organometallics. 2008, 27, 563. Anderson, D. R.; Lavallo, V.; O'Leary, D. J.; Bertrand, G.; Grubbs, R. H. Angew. Chem. 2007, 119, 7400; Angew. Chem. Int. Ed. 2007, 46, 7262.) In particular, it was envisioned that more in-depth structure/activity relationship (SAR) studies would facilitate the development of new, more efficient catalysts. Thus, a variety of new catalysts were prepared through modifications of exisiting literature procedures (**Scheme 2**). (Anderson, D. R.; Ung, T.; Mkrtumyan. G.; Bertrand, G.; Grubbs, R. H.; Schrodi, Y. Organometallics. 2008, 27, 563. Anderson, D. R.; Lavallo, V.; O'Leary, D. J.; Bertrand, G.; Grubbs, R. H. Angew. Chem. 2007, 119, 7400; Angew. Chem. Int. Ed. 2007, 46, 7262. Jazzar, R.; Dewhurst, R. D.; Bourg, J.- B.; Donnadieu, B.; Canac, Y.; Bertrand, G. Angew. Chem. 2007, 119, 2957; Angew. Chem Int. Ed. 2007, 46, 2899.) Known CAAC catalysts (10, 13, 18, 25) were screened alongside the new catalysts, in order to ensure accurate SAR comparisons within the series.

Initially, derivatives of the previous benchmark catalyst 10 were targeted, in which only the *ortho* substituents of the *N*-aryl ring were varied (9 - 13). It is worthy to note that the syntheses of 9 and 10 are low yielding (*ca.* 20%), and purification is cumbersome. Furthermore, complexes bearing even smaller *ortho* substitution such as *N*-mesityl or *N*-2-isopropylphenyl, were unable to be accessed in even small amounts. In contrast, 11 - 13 can be produced in high yield (78 - 86%) and isolated without difficulty. This is hypothesized to be related to the stability of the free carbene intermediate that is generated *in situ,* which might otherwise be expected to decompose rapidly in the absence of steric protection. It was envisioned that this decomposition pathway could be circumvented through the installment of larger substituents at either $R^4$ or $R^5$. Indeed, this strategy proved to be successful, and we were able to readily access a variety of new complexes in moderate to high yield (29 - 82%).

Several of the new backbone containing catalysts included *N*-aryl substitution that was previously inaccessible (14, 15, 17, 21), meanwhile others (16, 18 - 20, 22 - 25) were synthesized in order to provide a more thorough SAR study. Single-crystal X-ray diffraction of 11 and 24 revealed distorted square-pyramidal geometries, and structural parameters, including bond lengths and angles, were consistent with those found previously for 10 and 13. Moreover, catalyst 24 exhibits a CAAC ligand featuring a chirogenic center as well as two different ortho *N*-aryl substituents. Accordingly, the single crystal of 24 revealed both *N*-aryl rotamers (24a and 24b), in a ratio of 64:36 respectively. (We also observed multiple conformational and rotational isomers in the NMR spectra of each of the catalysts 21 - 24. Ratios varied with respect to *N*-aryl substitution (see experimental section for details).

The invention relates to a ruthenium complex bearing a cyclic alkyl amino carbene ligand, which is represented by Formula V,

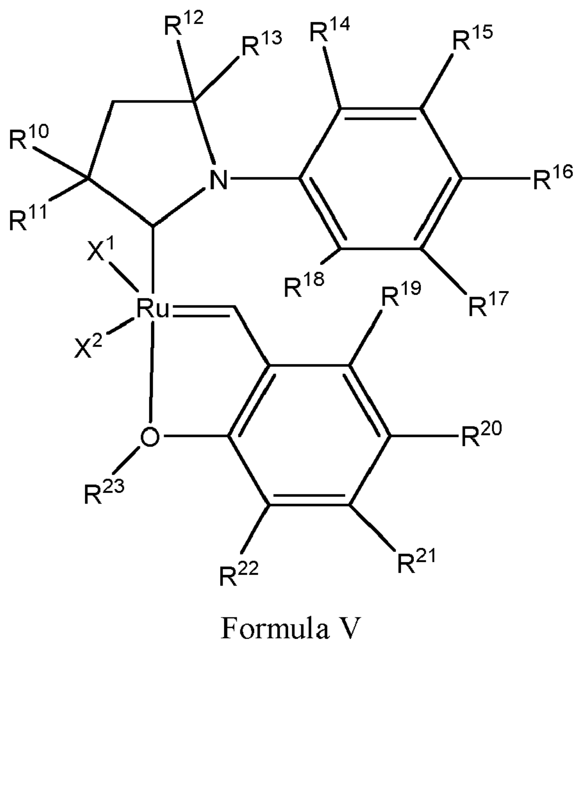

Formula V

wherein:

$X^1$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsulfonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;

$X^2$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsulfonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;

$R^{10}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{11}$ can form a form a five-, six-, or ten-membered cycloalkyl or heterocyclyl ring, with the carbon atom to which they are attached;

$R^{11}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{10}$ can form a form a five-, six-, or ten-membered cycloalkyl or heterocyclyl ring, with the carbon atom to which they are attached;

$R^{12}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{13}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{14}$ , $R^{15}$, $R^{16}$, $R^{17}$, and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N) , cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);

$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl

(-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N) , cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

Further, the ruthenium complex bearing a cyclic alkyl amino carbene ligand, represented by Formula V, can be represented by any of Formulae I, II, III or IV.

In certain embodiments, the invention relates to a compound, wherein the compound is

(A) a compound of Formula I:

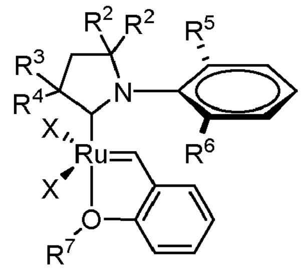

Formula I

wherein, independently for each occurrence,

X is, independently for each occurrence, a monovalent ligand, such as alkoxy or (preferably) halo, most preferably chloro;

$R^2$ is, independently for each occurrence, alkyl, preferably methyl;

$R^3$ is alkyl, preferably methyl;

$R^4$ is alkyl, aryl, heteroaryl, aralkyl, or heteroaralkyl;or $R^3$ and $R^4$, taken together with the carbon atom to which they are attached, form a five-, six-, or ten-membered cycloalkyl or heterocyclyl ring;

$R^5$ is alkyl;

$R^6$ is H or alkyl (preferably methyl or ethyl, most preferably methyl), provided that (i) $R^5$ and $R^6$ are not the same, and (ii) $R^6$ has fewer atoms than $R^5$; and

$R^7$ is alkyl, preferably a secondary or tertiary alkyl, most preferably 2-propyl;

(B) a compound of Formula II:

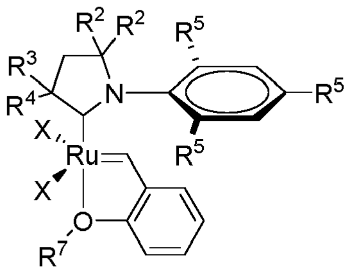

Formula II

wherein, independently for each occurrence,

X is, independently for each occurrence, a monovalent ligand, such as alkoxy or (preferably) halo, most preferably chloro;
$R^2$ is, independently for each occurrence, alkyl, preferably methyl;
$R^3$ is alkyl, preferably methyl;
$R^4$ is alkyl, aryl, heteroaryl, aralkyl, or heteroaralkyl;
or $R^3$ and $R^4$, taken together with the carbon atom to which they are attached, form a five-, six-, or ten-membered cycloalkyl or heterocyclyl ring;
$R^5$ is, independently for each occurrence, alkyl, preferably methyl; and
$R^7$ is alkyl, preferably a secondary or tertiary alkyl, most preferably 2-propyl;

(C) a compound of Formula III:

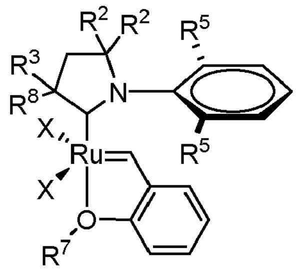

Formula III

wherein, independently for each occurrence,

X is, independently for each occurrence, a monovalent ligand, such as alkoxy or (preferably) halo, most preferably chloro;
$R^2$ is alkyl, preferably methyl;
$R^3$ is alkyl, preferably methyl;
$R^5$ is, independently for each occurrence, alkyl, preferably methyl or ethyl;
$R^7$ is alkyl, preferably a secondary or tertiary alkyl, most preferably 2-propyl; and
$R^8$ is aryl or heteroaryl, preferably substituted or unsubstituted phenyl, most preferably unsubstituted phenyl; or

(D) a compound of Formula IV:

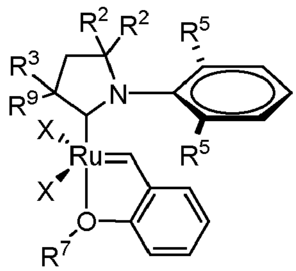

Formula IV

wherein, independently for each occurrence,

X is, independently for each occurrence, a monovalent ligand, such as alkoxy or (preferably) halo, most preferably chloro;
$R^2$ is alkyl, preferably methyl;
$R^3$ is alkyl, preferably methyl;
$R^5$ is, independently for each occurrence, alkyl, preferably methyl or ethyl;
$R^7$ is alkyl, preferably a secondary or tertiary alkyl, most preferably 2-propyl;
$R^9$ is $C_2$-$C_6$ alkyl, preferably n-propyl;
or $R^3$ and $R^9$, taken together with the carbon atom to which they are attached, form a five-, or ten-membered cycloalkyl or heterocyclyl ring.

In other embodiments, the invention relates to a compound selected from:

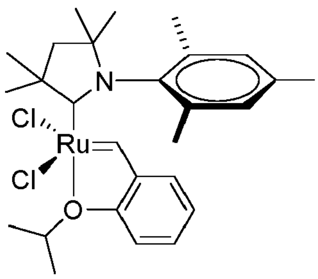 ,
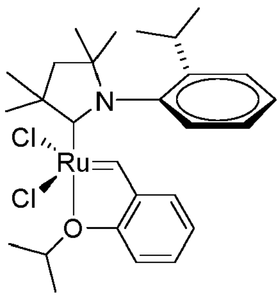 ,
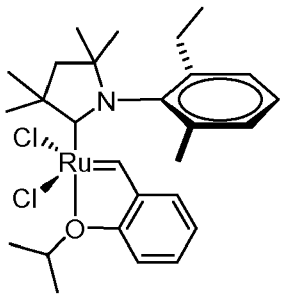 ,

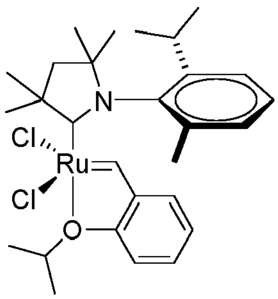 ,
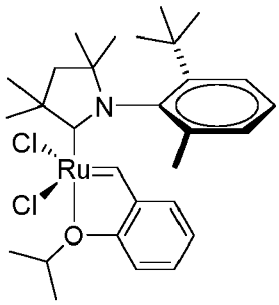 ,
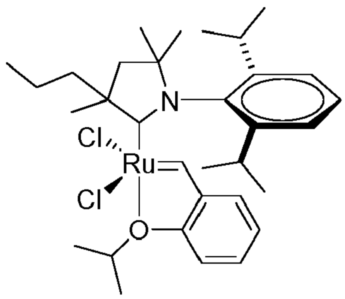 ,

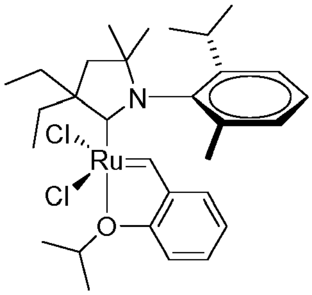
,
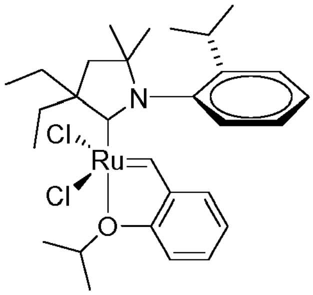
,
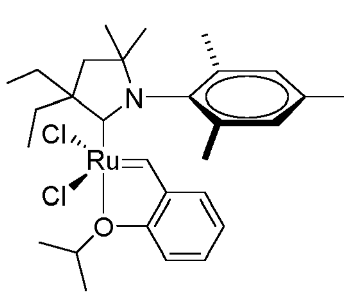
,

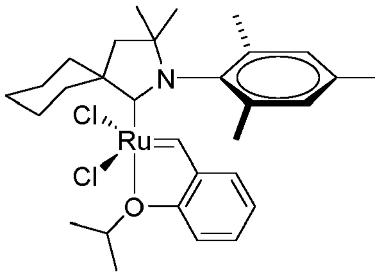
,
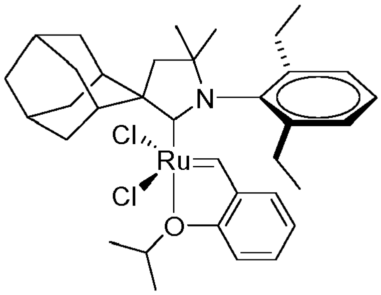
,
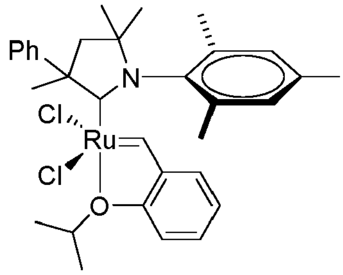
,

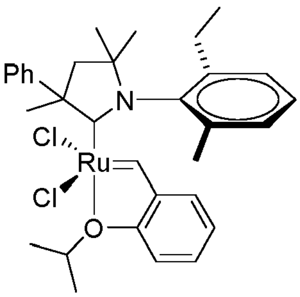
,
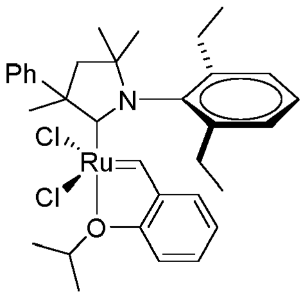
, and
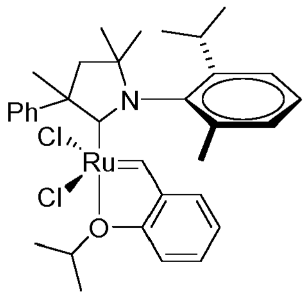
.

In still other embodiments, the invention relates to a method of producing an olefin product comprising:

providing an olefinic substrate;
providing an alpha olefin; and
contacting the olefinic substrate with the alpha olefin in the presence of any one of the compounds described herein under reaction conditions effective to allow a metathesis reaction to occur, thereby producing the olefin product.

**Brief Description of the Figures**

FIG. 1. Ethenolysis of methyl oleate using catalysts 9-25.

FIG. 2. Depicts the evolution of conversion as a function of reaction time in the ethenolysis of 1-hexene catalyzed by various compounds of the invention.

FIG. 3. Depicts the evolution of conversion as a function of reaction time at varying catalyst loading of catalyst Ru-10 (FIG. 3a), varying pressures and temperatures (FIG. 3b), and varying catalysts (FIG. 3c).

FIG. 4. Comparison of initiation rates and TON for catalysts 9 - 14.

## Detailed Description of the Invention

Overview

Unless otherwise indicated, the invention is not limited to specific reactants, substituents, catalysts, reaction conditions, or the like, as such may vary. It is also to be understood that the terminology used herein is for the purpose of describing particular embodiments only and is not intended to be limiting.

As used in the specification and the appended claims, the singular forms "a," "an," and "the" include plural referents unless the context clearly dictates otherwise. Thus, for example, reference to "an $\alpha$-olefin" includes a single $\alpha$-olefin as well as a combination or mixture of two or more $\alpha$-olefins, reference to "a substituent" encompasses a single substituent as well as two or more substituents, and the like.

As used in the specification and the appended claims, the terms "for example," "for instance," "such as," or "including" are meant to introduce examples that further clarify more general subject matter. Unless otherwise specified, these examples are provided only as an aid for understanding the invention, and are not meant to be limiting in any fashion.

In this specification and in the claims that follow, reference will be made to a number of terms, which shall be defined to have the following meanings:

The term "alkyl" as used herein refers to a linear, branched, or cyclic saturated hydrocarbon group typically although not necessarily containing 1 to about 24 carbon atoms, preferably 1 to about 12 carbon atoms, such as methyl (Me), ethyl (Et), n-propyl (Pr or n-Pr), isopropyl (i-Pr), n-butyl (Bu or n-Bu), isobutyl (i-Bu), t-butyl (t-Bu), octyl (Oct), decyl, and the like, as well as cycloalkyl groups such as cyclopentyl (Cp), cyclohexyl (Cy) and the like.

Generally, although again not necessarily, alkyl groups herein contain 1 to about 12 carbon atoms. The term "lower alkyl" refers to an alkyl group of 1 to 6 carbon atoms, and the specific term "cycloalkyl" refers to a cyclic alkyl group, typically having 4 to 8, preferably 5 to 7, carbon atoms. The term "substituted alkyl" refers to alkyl substituted with one or more substituent groups, and the terms "heteroatom-containing alkyl" and "heteroalkyl" refer to alkyl in which at least one carbon atom is replaced with a heteroatom. If not otherwise indicated, the terms "alkyl" and "lower alkyl" include linear, branched, cyclic, unsubstituted, substituted, and/or heteroatom-containing alkyl and lower alkyl, respectively.

The term "alkylene" as used herein refers to a difunctional linear, branched, or cyclic alkyl group, where "alkyl" is as defined above.

The term "alkenyl" as used herein refers to a linear, branched, or cyclic hydrocarbon group of 2 to about 24 carbon atoms containing at least one double bond, such as ethenyl, n-propenyl, isopropenyl, n-butenyl, isobutenyl, octenyl, decenyl, tetradecenyl, hexadecenyl, eicosenyl, tetracosenyl, and the like. Preferred alkenyl groups herein contain 2 to about 12 carbon atoms. The term "lower alkenyl" refers to an alkenyl group of 2 to 6 carbon atoms, and the specific term "cycloalkenyl" refers to a cyclic alkenyl group, preferably having 5 to 8 carbon atoms. The term "substituted alkenyl" refers to alkenyl substituted with one or more substituent groups, and the terms "heteroatom-containing alkenyl" and "heteroalkenyl" refer to alkenyl in which at least one carbon atom is replaced with a heteroatom. If not otherwise indicated, the terms "alkenyl" and "lower alkenyl" include linear, branched, cyclic, unsubstituted, substituted, and/or heteroatom-containing alkenyl and lower alkenyl, respectively.

The term "alkenylene" as used herein refers to a difunctional linear, branched, or cyclic alkenyl group, where "alkenyl" is as defined above.

The term "alkynyl" as used herein refers to a linear or branched hydrocarbon group of 2 to about 24 carbon atoms containing at least one triple bond, such as ethynyl, n-propynyl, and the like. Preferred alkynyl groups herein contain 2 to about 12 carbon atoms. The term "lower alkynyl" refers to an alkynyl group of 2 to 6 carbon atoms. The term "substituted alkynyl" refers to alkynyl substituted with one or more substituent groups, and the terms "heteroatom-containing alkynyl" and "heteroalkynyl" refer to alkynyl in which at least one carbon atom is replaced with a heteroatom. If not otherwise indicated, the terms "alkynyl" and "lower alkynyl" include linear, branched, unsubstituted, substituted, and/or heteroatom-containing alkynyl and lower alkynyl, respectively.

The term "alkynylene" as used herein refers to a difunctional alkynyl group, where "alkynyl" is as defined above.

The term "alkoxy" as used herein refers to an alkyl group bound through a single, terminal ether linkage; that is, an "alkoxy" group may be represented as -O-alkyl where alkyl is as defined above. A "lower alkoxy" group refers to an alkoxy group containing 1 to 6 carbon atoms. Analogously, "alkenyloxy" and "lower alkenyloxy" respectively refer to an alkenyl and lower alkenyl group bound through a single, terminal ether linkage, and "alkynyloxy" and "lower alkynyloxy" respectively refer to an alkynyl and lower alkynyl group bound through a single, terminal ether linkage.

The term "aryl" as used herein, and unless otherwise specified, refers to an aromatic substituent containing a single aromatic ring or multiple aromatic rings that are fused together, directly linked, or indirectly linked (such that the different aromatic rings are bound to a common group such as a methylene or ethylene moiety). Preferred aryl groups contain 5 to 24 carbon atoms, and particularly preferred aryl groups contain 5 to 14 carbon atoms. Exemplary aryl groups contain one aromatic ring or two fused or linked aromatic rings, e.g., phenyl (Ph), naphthyl, biphenyl, diphenylether,

diphenylamine, benzophenone, and the like. "Substituted aryl" refers to an aryl moiety substituted with one or more substituent groups, and the terms "heteroatom containing aryl" and "heteroaryl" refer to aryl substituents in which at least one carbon atom is replaced with a heteroatom, as will be described in further detail herein.

The term "aryloxy" as used herein refers to an aryl group bound through a single, terminal ether linkage, wherein "aryl" is as defined above. An "aryloxy" group may be represented as -O-aryl where aryl is as defined above. Preferred aryloxy groups contain 5 to 24 carbon atoms, and particularly preferred aryloxy groups contain 5 to 14 carbon atoms. Examples of aryloxy groups include, without limitation, phenoxy, o-halo-phenoxy, m-halo-phenoxy, p-halo-phenoxy, o-methoxy-phenoxy, m-methoxy-phenoxy, p-methoxy-phenoxy, 2,4-dimethoxy-phenoxy, 3,4,5-trimethoxy-phenoxy, and the like.

The term "alkaryl" refers to an aryl group with an alkyl substituent, and the term "aralkyl" refers to an alkyl group with an aryl substituent, wherein "aryl" and "alkyl" are as defined above. Preferred alkaryl and aralkyl groups contain 6 to 24 carbon atoms, and particularly preferred alkaryl and aralkyl groups contain 6 to 16 carbon atoms. Alkaryl groups include, without limitation, p-methylphenyl, 2,4-dimethylphenyl, p-cyclohexylphenyl, 2,7-dimethylnaphthyl, 7-cyclooctylnaphthyl, 3-ethyl-cyclopenta-1,4-diene, and the like. Examples of aralkyl groups include, without limitation, benzyl, 2-phenyl-ethyl, 3-phenylpropyl, 4-phenyl-butyl, 5-phenyl-pentyl, 4-phenylcyclohexyl, 4-benzylcyclohexyl, 4-phenylcyclohexylmethyl, 4-benzylcyclohexylmethyl, and the like. The terms "alkaryloxy" and "aralkyloxy" refer to substituents of the formula -OR wherein R is alkaryl or aralkyl, respectively, as just defined.

The term "acyl" refers to substituents having the formula -(CO)-alkyl, -(CO)-aryl, - (CO)-aralkyl, -(CO)-alkaryl, -(CO)-alkenyl, or -(CO)-alkynyl, and the term "acyloxy" refers to substituents having the formula -O(CO)-alkyl, -O(CO)-aryl, -O(CO)-aralkyl, -O(CO)-alkaryl, -O(CO)-alkenyl, or -(CO)-alkynyl wherein "alkyl," "aryl," "aralkyl," "alkaryl," "alkenyl," and "alkynyl" are as defined above. The acetoxy group (-O(CO)$CH_3$; often abbreviated as OAc) is a common example of an acyloxy group.

The terms "cyclic" and "ring" refer to alicyclic or aromatic groups that may or may not be substituted and/or heteroatom containing, and that may be monocyclic, bicyclic, or polycyclic. The term "alicyclic" is used in the conventional sense to refer to an aliphatic cyclic moiety, as opposed to an aromatic cyclic moiety, and may be monocyclic, bicyclic or polycyclic.

The terms "halo" and "halogen" are used in the conventional sense to refer to a fluoro, chloro, bromo, or iodo substituent.

"Hydrocarbyl" refers to univalent hydrocarbyl radicals containing 1 to about 30 carbon atoms, preferably 1 to about 24 carbon atoms, most preferably 1 to about 12 carbon atoms, including linear, branched, cyclic, saturated and unsaturated species, such as alkyl groups, alkenyl groups, alkynyl groups, aryl groups, and the like. The term "lower hydrocarbyl" refers to a hydrocarbyl group of 1 to 6 carbon atoms, preferably 1 to 4 carbon atoms, and the term "hydrocarbylene" refers to a divalent hydrocarbyl moiety containing 1 to about 30 carbon atoms, preferably 1 to about 24 carbon atoms, most preferably 1 to about 12 carbon atoms, including linear, branched, cyclic, saturated and unsaturated species. The term "lower hydrocarbylene" refers to a hydrocarbylene group of 1 to 6 carbon atoms. "Substituted hydrocarbyl" refers to hydrocarbyl substituted with one or more substituent groups, and the terms "heteroatom-containing hydrocarbyl" and "heterohydrocarbyl" refer to hydrocarbyl in which at least one carbon atom is replaced with a heteroatom. Similarly, "substituted hydrocarbylene" refers to hydrocarbylene substituted with one or more substituent groups, and the terms "heteroatom-containing hydrocarbylene" and heterohydrocarbylene" refer to hydrocarbylene in which at least one carbon atom is replaced with a heteroatom. Unless otherwise indicated, the term "hydrocarbyl" and "hydrocarbylene" are to be interpreted as including substituted and/or heteroatom-containing hydrocarbyl and hydrocarbylene moieties, respectively.

The term "heteroatom-containing" as in a "heteroatom-containing hydrocarbyl group" refers to a hydrocarbon molecule or a hydrocarbyl molecular fragment in which one or more carbon atoms is replaced with an atom other than carbon, e.g., nitrogen, oxygen, sulfur, phosphorus or silicon, typically nitrogen, oxygen or sulfur. Similarly, the term "heteroalkyl" refers to an alkyl substituent that is heteroatom-containing, the term "heterocyclic" refers to a cyclic substituent that is heteroatom-containing, the terms "heteroaryl" and "heteroaromatic" respectively refer to "aryl" and "aromatic" substituents that are heteroatom-containing, and the like. It should be noted that a "heterocyclic" group or compound may or may not be aromatic, and further that "heterocycles" may be monocyclic, bicyclic, or polycyclic as described above with respect to the term "aryl." Examples of heteroalkyl groups include without limitation alkoxyaryl, alkylsulfanyl-substituted alkyl, N-alkylated amino alkyl, and the like. Examples of heteroaryl substituents include without limitation pyrrolyl, pyrrolidinyl, pyridinyl, quinolinyl, indolyl, pyrimidinyl, imidazolyl, 1,2,4-triazolyl, tetrazolyl, etc., and examples of heteroatom-containing alicyclic groups include without limitation pyrrolidino, morpholino, piperazino, piperidino, etc.

By "substituted" as in "substituted hydrocarbyl," "substituted alkyl," "substituted aryl," and the like, as alluded to in some of the aforementioned definitions, is meant that in the hydrocarbyl, alkyl, aryl, or other moiety, at least one hydrogen atom bound to a carbon (or other) atom is replaced with one or more non-hydrogen substituents. Examples of such substituents include, without limitation: functional groups referred to herein as "Fn," such as halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$

alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$),mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl),N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano(-C=N), cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-($C_1$-$C_{24}$ alkyl)-substituted amino (-NH($C_1$-$C_{24}$ alkyl), di-($C_1$-$C_{24}$ alkyl)-substituted amino ((-N($C_1$-$C_{24}$ alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), phosphino (-$PH_2$), silyl (-$SiR_3$ wherein R is hydrogen or hydrocarbyl), and silyloxy (-O-silyl); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl).

By "functionalized" as in "functionalized hydrocarbyl," "functionalized alkyl," "functionalized olefin," "functionalized cyclic olefin," and the like, is meant that in the hydrocarbyl, alkyl, olefin, cyclic olefin, or other moiety, at least one hydrogen atom bound to a carbon (or other) atom is replaced with one or more functional groups such as those described hereinabove. The term "functional group" is meant to include any functional species that is suitable for the uses described herein. In particular, as used herein, a functional group would necessarily possess the ability to react with or bond to corresponding functional groups on a substrate surface.

In addition, the aforementioned functional groups may, if a particular group permits, be further substituted with one or more additional functional groups or with one or more hydrocarbyl moieties such as those specifically enumerated above. Analogously, the above mentioned hydrocarbyl moieties may be further substituted with one or more functional groups or additional hydrocarbyl moieties such as those specifically mentioned above.

Analogously, the above-mentioned hydrocarbyl moieties may be further substituted with one or more functional groups or additional hydrocarbyl moieties as noted above.

The term "ethenolysis" refers to the cross metathesis of a substrate with ethylene. For example, ethenolysis of methyl oleate produces methyl 9-decenoate and 1-decene. For ethenolysis references see Burdett, K.A.; Harris, L.D.; Margl, P.; Maughon, B.R.; Mokhtar-Zadeh, T.; Saucier, P.C.; Wasserman, E.P. Organometallics 2004, 23, 2027; Nickel, A.; Ung, T., Mkrtumyan, G., Uy, J., Lee, C.H., Stoianova, D., Papazian, J., Wei,W.-H., Mallari, A., Schrodi, Y., Pederson, R.L. Topic in Catalysis, 2012, 55, 518-523; Warwel, S.; Brüse, F.; Demes, C.; Kunz, M.; Rüsch gen. Klaas M., Chemosphere 2001, 43, 39; Anderson, D.R.; Ung, T.; Mkrtumyan, G.; Bertrand, G.; Grubbs, R.H.; Schrodi, Y. Organometallics 2008, 27, 563.; Schrodi, Y.; Ung, T.; Vargas, A.; Mkrtumyan, G.; Lee, C.W.; Champagne, T.M.; Pederson, R.L.; Hong, S.H. Clean - Soil, Air, Water 2008, 36, 669.

The term "alkenolysis" refers to a cross metathesis reaction where a terminal olefin is used in a cross metathesis reaction with an internal double bond to produce different terminal olefins, where the initial terminal olefin cannot be ethylene. For example alkenolysis of methyl oleate with 1-butene produces methyl 9-decenoate, 1-decene, methyl-9-dodecenoate and 3-dodecene. For alkenolysis references see Schrodi, Y.; Pederson, R.L.; Kaido, H.; Tupy, M.J. US Pat. App. 2010/0145086, assigned to Elevance Renewable Sciences, Inc; and Nickel, A.; Ung, T., Mkrtumyan, G., Uy, J., Lee, C.H., Stoianova, D., Papazian, J., Wei,W.-H., Mallari, A., Schrodi, Y., Pederson, R.L. Topic in Catalysis, 2012, 55, 518-523, are incorporated by reference.

"Optional" or "optionally" means that the subsequently described circumstance may or may not occur, so that the description includes instances where the circumstance occurs and instances where it does not. For example, the phrase "optionally substituted" means that a nonhydrogen substituent may or may not be present on a given atom, and,

thus, the description includes structures wherein a non-hydrogen substituent is present and structures wherein a non-hydrogen substituent is not present.

The invention relates to a ruthenium complex bearing a cyclic alkyl amino carbene ligand, which is represented by Formula V,

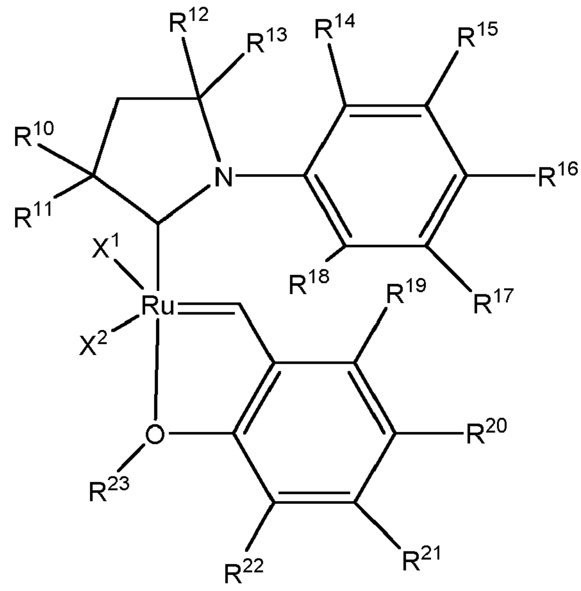

Formula V

wherein:

$X^1$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsulfonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;

$X^2$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsulfonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;

$R^{10}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{11}$ can form a form a five-, six-, or ten-membered cycloalkyl or heterocyclyl ring, with the carbon atom to which they are attached;

$R^{11}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{10}$ can form a form a five-, six-, or ten-membered cycloalkyl or heterocyclyl ring, with the carbon atom to which they are attached;

$R^{12}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{13}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{14}$ , $R^{15}$, $R^{16}$, $R^{17}$ and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido

(-NH-(CO)-$NH_2$), cyano (-C≡N) , cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);

$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato (-$COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

In another embodiment, the ruthenium complex bearing a cyclic alkyl amino carbene ligand, is represented by Formula V, wherein:

$X^1$ is halo;

$X^2$ is halo;

$R^{10}$ is $C_1$-$C_{12}$ alkyl, $C_4$-$C_{12}$ cycloalkyl, $C_5$-$C_{24}$ aryl, or together with $R^{11}$ can form a form a five-, six-, or ten-membered cycloalkyl or heterocyclyl ring, with the carbon atom to which they are attached;

$R^{11}$ is $C_1$-$C_{12}$ alkyl, $C_4$-$C_{12}$ cycloalkyl, $C_5$-$C_{24}$ aryl, , or together with $R^{10}$ can form a form a five-, six-, or ten-membered cycloalkyl or heterocyclyl ring, with the carbon atom to which they are attached;
$R^{12}$ is $C_1$-$C_{12}$ alkyl or $C_4$-$C_{12}$ cycloalkyl;
$R^{13}$ is $C_1$-$C_{12}$ alkyl or $C_4$-$C_{12}$ cycloalkyl;
$R^{14}$ , $R^{15}$, $R^{16}$, $R^{17}$ and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C=N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-(Cs-C24 aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);
$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-($C_1$-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation

hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro ($-NO_2$), nitroso (-NO), sulfo ($-SO_2$-OH), sulfonato ($-SO_2-O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl ($-SO_2$-alkyl), $C_1$-$C_{24}$ monoalkylaminosulfonyl ($-SO_2$-N(H) alkyl), $C_1$-$C_{24}$ dialkylaminosulfonyl ($-SO_2$-N(alkyl)$_2$), $C_5$-$C_{24}$ arylsulfonyl ($-SO_2$-aryl), boryl ($-BH_2$), borono ($-B(OH)_2$), boronato ($-B(OR)_2$ where R includes without limitation alkyl or other hydrocarbyl), phosphono ($-P(O)(OH)_2$), phosphonato ($-P(O)(O^-)_2$), phosphinato ($-P(O)(O^-)$), phospho ($-PO_2$), and phosphino ($-PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

In another embodiment, the ruthenium complex bearing a cyclic alkyl amino carbene ligand, is represented by Formula V, wherein:

$X^1$ is $C_1$-$C_{20}$ alkoxy, halo or $C_1$-$C_{20}$ alkyl;

$X^2$ is $C_1$-$C_{20}$ alkoxy, halo or $C_1$-$C_{20}$ alkyl;

$R^{10}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{11}$ can form a form a five-, six-, or ten-membered cycloalkyl or heterocyclyl ring, with the carbon atom to which they are attached;

$R^{11}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{10}$ can form a form a five-, six-, or ten-membered cycloalkyl or heterocyclyl ring, with the carbon atom to which they are attached;

$R^{12}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{13}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{14}$, $R^{15}$, $R^{16}$, $R^{17}$ and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino ($-NH_2$), mono-($C_1$-$C_{24}$ alkyl)-substituted amino (-NH($C_1$-$C_{24}$ alkyl), di-($C_1$-$C_{24}$ alkyl)-substituted amino (-N($C_1$-$C_{24}$ alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro ($-NO_2$), nitroso (-NO), sulfo ($-SO_2$-OH), sulfonato ($-SO_2-O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl ($-SO_2$-alkyl), $C_1$-$C_{24}$ monoalkylaminosulfonyl ($-SO_2$-N(H) alkyl), $C_1$-$C_{24}$ dialkylaminosulfonyl ($-SO_2$-N(alkyl)$_2$), $C_5$-$C_{24}$ arylsulfonyl ($-SO_2$-aryl), boryl ($-BH_2$), borono ($-B(OH)_2$), boronato ($-B(OR)_2$ where R includes without limitation alkyl or other hydrocarbyl), phosphono ($-P(O)(OH)_2$), phosphonato ($-P(O)(O^-)_2$), phosphinato ($-P(O)(O^-)$), phospho ($-PO_2$), and phosphino ($-PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);

$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$

heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-(Cs-C24 aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

In another embodiment, the ruthenium complex bearing a cyclic alkyl amino carbene ligand, is represented by Formula V, wherein:

$X^1$ is halo;

$X^2$ is halo;

$R^{10}$ is $C_1$-$C_{12}$ alkyl, $C_4$-$C_{12}$ cycloalkyl, $C_5$-$C_{24}$ aryl, or together with $R^{11}$ can form a form a five-, six-, or ten-membered cycloalkyl or heterocyclyl ring, with the carbon atom to which they are attached;

$R^{11}$ is $C_1$-$C_{12}$ alkyl, $C_4$-$C_{12}$ cycloalkyl, $C_5$-$C_{24}$ aryl, , or together with $R^{10}$ can form a form a five-, six-, or ten-membered cycloalkyl or heterocyclyl ring, with the carbon atom to which they are attached;

$R^{12}$ is $C_1$-$C_{12}$ alkyl or $C_4$-$C_{12}$ cycloalkyl;

$R^{13}$ is $C_1$-$C_{12}$ alkyl or $C_4$-$C_{12}$ cycloalkyl;

$R^{14}$, $R^{15}$, $R^{16}$, $R^{17}$ and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$),

di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B$(OH)_2$), boronato (-B$(OR)_2$ where R includes without limitation alkyl or other hydrocarbyl), phosphono (-P(O)$(OH)_2$), phosphonato (-P(O)$(O^-)_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);

$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato (-$COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-$C_{24}$ alkyl)$_2$), mono-($C_5$-$C_{24}$ aryl)-substituted amino (-NH($C_5$-$C_{24}$ aryl), di-($C_6$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B$(OH)_2$), boronato (-B$(OR)_2$ where R includes without limitation alkyl or other hydrocarbyl), phosphono (-P(O)$(OH)_2$), phosphonato (-P(O)$(O^-)_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

In another embodiment, the ruthenium complex bearing a cyclic alkyl amino carbene ligand, is represented by Formula V, wherein:

$X^1$ is halo;

$X^2$ is halo;

$R^{10}$ is $C_1$-$C_{12}$ alkyl, or together with $R^{11}$ can form a form a five-, six-, or ten-membered cycloalkyl or heterocyclyl

ring, with the carbon atom to which they are attached;
$R^{11}$ is $C_1$-$C_{12}$ alkyl, or together with $R^{10}$ can form a form a five-, six-, or ten-membered cycloalkyl or heterocyclyl ring, with the carbon atom to which they are attached;
$R^{12}$ is $C_1$-$C_{12}$ alkyl;
$R^{13}$ is $C_1$-$C_{12}$ alkyl;
$R^{14}$, $R^{15}$, $R^{16}$, $R^{17}$ and $R^{18}$ are independently selected from H, $C_1$-$C_{12}$ alkyl.
$R^{19}$, $R^{20}$, $R^{21}$ and $R^{22}$ are independently selected from H, $C_1$-$C_{12}$ alkyl.
$R^{23}$ is $C_1$-$C_{12}$ alkyl.

In another embodiment the CAAC Ru catalyst is represented by Formula V, wherein:

$X^1$ is halo;
$X^2$ is halo;
$R^{10}$ is $C_1$-$C_{12}$ alkyl or $C_5$-$C_{24}$ aryl, or together with $R^{11}$ can form a form a five-, six-, or ten-membered cycloalkyl with the carbon atom to which they are attached;
$R^{11}$ is $C_1$-$C_{12}$ alkyl or $C_5$-$C_{24}$ aryl, or together with $R^{10}$ can form a form a five-, six-, or ten-membered cycloalkyl with the carbon atom to which they are attached;
$R^{12}$ is $C_1$-$C_{12}$ alkyl;
$R^{13}$ is $C_1$-$C_{12}$ alkyl;
$R^{14}$, $R^{15}$, $R^{16}$, $R^{17}$ and $R^{18}$ are independently selected from H, $C_1$-$C_{12}$ alkyl.
$R^{19}$, $R^{20}$, $R^{21}$ and $R^{22}$ are independently selected from H, $C_1$-$C_{12}$ alkyl.
$R^{23}$ is $C_1$-$C_{12}$ alkyl.

In another embodiment, the ruthenium complex bearing a cyclic alkyl amino carbene ligand, is represented by Formula V, wherein:

$X^1$ is chloride;
$X^2$ is chloride;
$R^{10}$ is methyl, n-propyl, ethyl, phenyl or together with $R^{11}$ can form a six-, or ten-membered cycloalkyl with the carbon atom to which they are attached;
$R^{11}$ is methyl, n-propyl, ethyl, phenyl or together with $R^{10}$ can form a six-, or ten-membered cycloalkyl with the carbon atom to which they are attached;
$R^{12}$ is methyl;
$R^{13}$ is methyl;
$R^{14}$is methyl, ethyl, iso-propyl or tert-butyl;
$R^{15}$ is H;
$R^{16}$ is H or methyl;
$R^{17}$ is H;
$R^{18}$ is H, methyl, ethyl or iso-propyl;
$R^{19}$ is H;
$R^{20}$ is H;
$R^{21}$ is H;
$R^{22}$ is H; and
$R^{23}$ is iso-propyl.

Further, the ruthenium complex bearing a cyclic alkyl amino carbene ligand, represented by Formula V, can be represented by any of Formulae I, II, III or IV.

In one aspect, the invention relates to ruthenium complexes, such as **C578** (Me, *i*Pr) (i.e., **Ru-10**) or **C782**

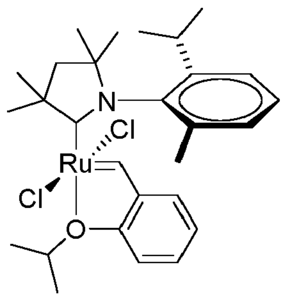

**C578 (Me, *i*Pr)**

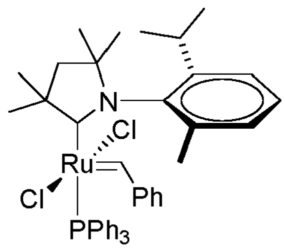

**C782**

which, bear cyclic alkyl amino carbene (CAAC) ligands with an *ortho* methyl substituent on the *N*-aryl ring. These complexes are viable catalyst systems for ethenolysis reactions on an industrial scale.

In certain embodiments, the catalyst systems are both highly active and selective for ethenolysis reactions. As an example, when applied to the ethenolysis of methyl oleate, catalyst **Ru-10** provided 1-decene and methyl-10-undecenoate in 32% yield (92% selectivity), exhibiting turn over numbers (TON) of 106,000. In one embodiment, the catalysts are used in the transformation of seed oil derivatives into commodity materials on an industrial scale.

Exemplary Compounds

In certain embodiments, the invention relates to a compound, wherein the compound is

(A) a compound of Formula I:

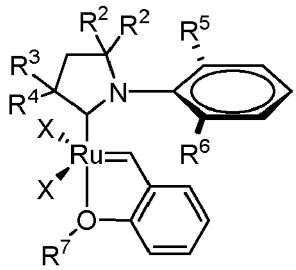

Formula I

wherein, independently for each occurrence,

X is, independently for each occurrence, a monovalent ligand, such as alkoxy or (preferably) halo, most preferably chloro;
$R^2$ is, independently for each occurrence, alkyl;
$R^3$ is alkyl, preferably methyl;
$R^4$ is alkyl, aryl, heteroaryl, aralkyl, or heteroaralkyl;
or $R^3$ and $R^4$, taken together with the carbon atom to which they are attached, form a five-, six-, or ten-membered cycloalkyl or heterocyclyl ring;
$R^5$ is alkyl;
$R^6$ is H or alkyl (preferably methyl or ethyl, most preferably methyl), provided that (i) $R^5$ and $R^6$ are not the same, and (ii) $R^6$ has fewer atoms than $R^5$; and
$R^7$ is alkyl, preferably a secondary or tertiary alkyl, most preferably 2-propyl;

(B) a compound of Formula II:

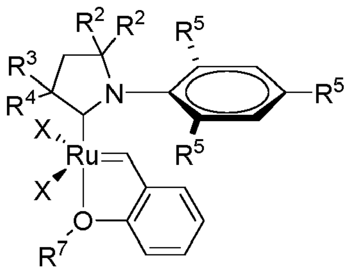

Formula II

wherein, independently for each occurrence,

X is, independently for each occurrence, a monovalent ligand, such as alkoxy or (preferably) halo, most preferably chloro;
$R^2$ is, independently for each occurrence, alkyl, preferably methyl;
$R^3$ is alkyl, preferably methyl;
$R^4$ is alkyl, aryl, heteroaryl, aralkyl, or heteroaralkyl;
or $R^3$ and $R^4$, taken together with the carbon atom to which they are attached, form a five-, six-, or ten-membered cycloalkyl or heterocyclyl ring;
$R^5$ is, independently for each occurrence, alkyl, preferably methyl; and
$R^7$ is alkyl, preferably a secondary or tertiary alkyl, most preferably 2-propyl;

(C) a compound of Formula III:

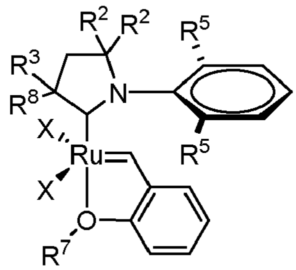

Formula III

wherein, independently for each occurrence,

X is, independently for each occurrence, a monovalent ligand, such as alkoxy or (preferably) halo, most preferably chloro;
$R^2$ is alkyl, preferably methyl;
$R^3$ is alkyl, preferably methyl;
$R^5$ is, independently for each occurrence, alkyl, preferably methyl or ethyl;
$R^7$ is alkyl, preferably a secondary or tertiary alkyl, most preferably 2-propyl; and
$R^8$ is aryl or heteroaryl, preferably substituted or unsubstituted phenyl, most preferably unsubstituted phenyl; or

(D) a compound of Formula IV:

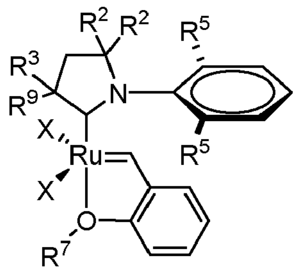

Formula IV

wherein, independently for each occurrence,

X is, independently for each occurrence, a monovalent ligand, such as alkoxy or (preferably) halo, most preferably chloro;
$R^2$ is alkyl, preferably methyl;
$R^3$ is alkyl, preferably methyl;
$R^5$ is, independently for each occurrence, alkyl, preferably methyl or ethyl;
$R^7$ is alkyl, preferably a secondary or tertiary alkyl, most preferably 2-propyl;
$R^9$ is $C_2$-$C_6$ alkyl, preferably n-propyl;
or $R^3$ and $R^9$, taken together with the carbon atom to which they are attached, form a five-, or ten-membered cycloalkyl or heterocyclyl ring.

In certain embodiments, the invention relates to any one of the compounds mentioned herein, wherein X is halo, such as chloro.

In certain embodiments, the invention relates to any one of the compounds mentioned herein, wherein $R^2$ is lower alkyl, such as methyl.

In certain embodiments, the invention relates to any one of the compounds mentioned herein, wherein $R^3$ is lower alkyl. In certain embodiments, the invention relates to any one of the compounds mentioned herein, wherein $R^3$ is methyl, or, in other embodiments, ethyl.

In certain embodiments, the invention relates to any one of the compounds mentioned herein, wherein $R^4$ is lower alkyl. In certain embodiments, the invention relates to any one of the compounds mentioned herein, wherein $R^4$ is methyl, or, in other embodiments, ethyl.

In certain embodiments, the invention relates to any one of the compounds mentioned herein, wherein $R^4$ is aryl, such as phenyl.

In certain embodiments, the invention relates to any one of the compounds mentioned herein, wherein $R^3$ and $R^4$, taken together with the carbon atom to which they are attached, form a cyclohexyl ring.

In certain embodiments, the invention relates to any one of the compounds mentioned herein, wherein $R^5$ is lower alkyl, such as methyl, ethyl, propyl, or butyl. In certain embodiments, the invention relates to any one of the compounds mentioned herein, wherein $R^5$ is methyl. In certain embodiments, the invention relates to any one of the compounds mentioned herein, wherein $R^5$ is ethyl. In certain embodiments, the invention relates to any one of the compounds mentioned herein, wherein $R^5$ is *iso*-propyl. In certain embodiments, the invention relates to any one of the compounds mentioned herein, wherein $R^5$ is tert-butyl.

In certain embodiments, the invention relates to any one of the compounds mentioned herein, wherein $R^6$ is H or lower alkyl. In certain embodiments, the invention relates to any one of the compounds mentioned herein, wherein $R^6$ is H. In certain embodiments, the invention relates to any one of the compounds mentioned herein, wherein $R^6$ is methyl.

In certain embodiments, the invention relates to any one of the compounds mentioned herein, wherein $R^7$ is lower alkyl. In certain embodiments, the invention relates to any one of the compounds mentioned herein, wherein $R^7$ is propyl, such as *iso*-propyl.

In certain embodiments, the invention relates to any one of the compounds mentioned herein, wherein $R^8$ is aryl, such as phenyl.

In certain embodiments, the invention relates to any one of the compounds mentioned herein, wherein $R^9$ is n-propyl.

In certain embodiments, the invention relates to any one of the compounds mentioned herein, wherein $R^3$ and $R^9$, taken together with the carbon atom to which they are attached, form an adamantyl ring.

In certain embodiments, the invention relates to any one of the compounds mentioned herein, provided the compound is not

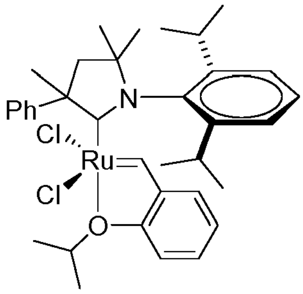

.

In other embodiments, the invention relates to a compound selected from:

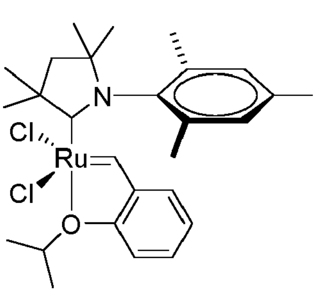

,

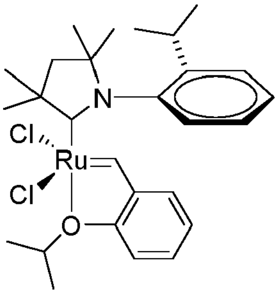

,

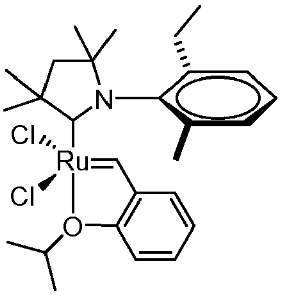

,

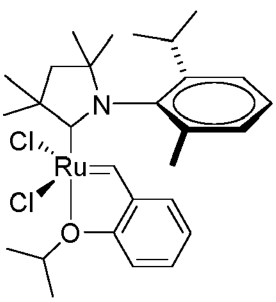

,

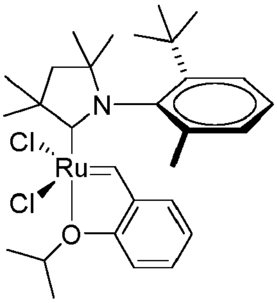

,

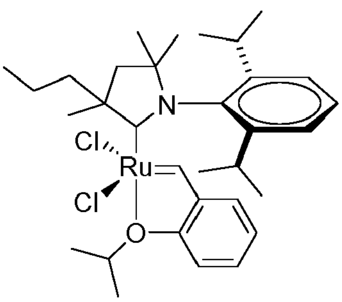

,

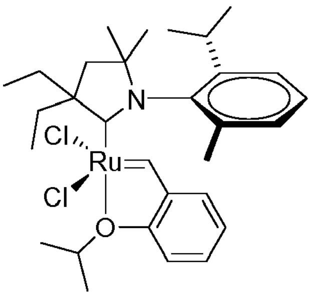

,

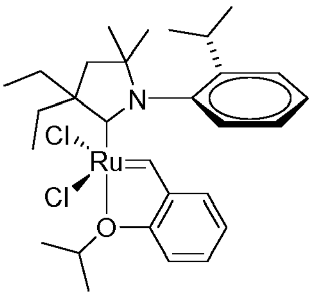

,

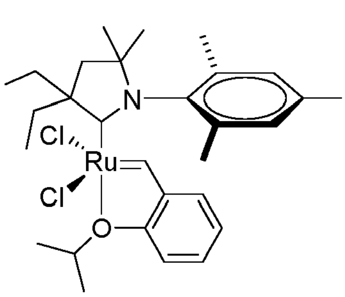

,

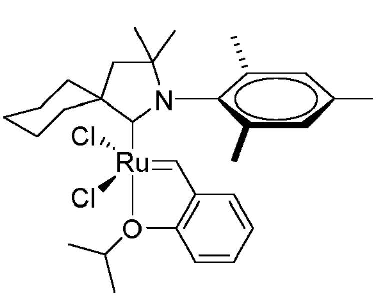

,

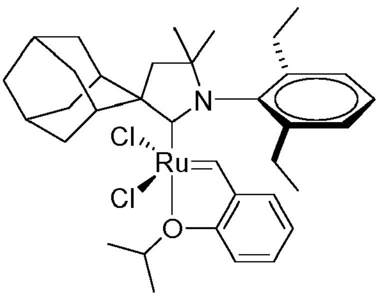

,

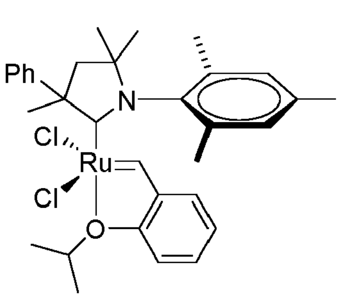

,

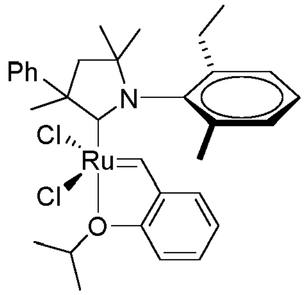, 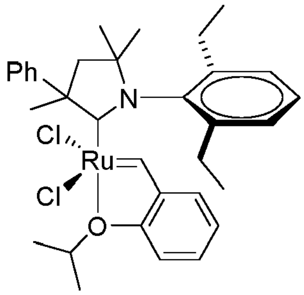, and 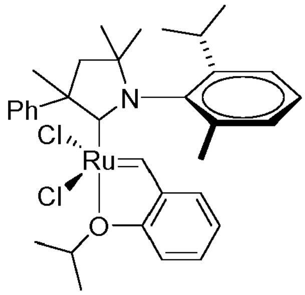.

In other embodiments, the invention relates to a compound (i.e., ruthenium complex bearing a cyclic alkyl amino carbene ligand) selected from:

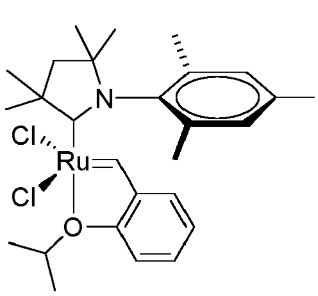, 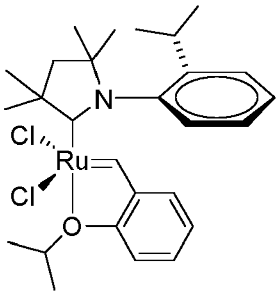, 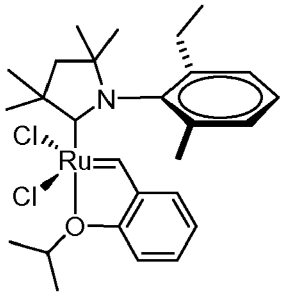,

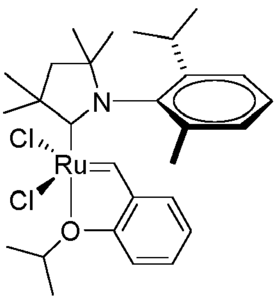, 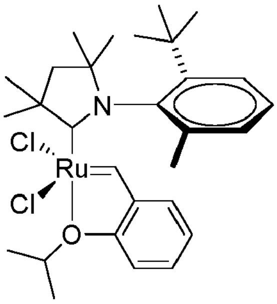, 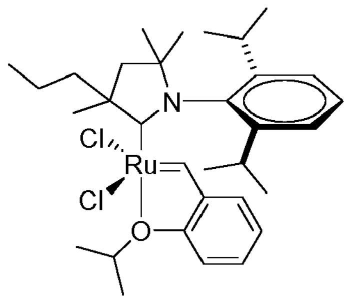,

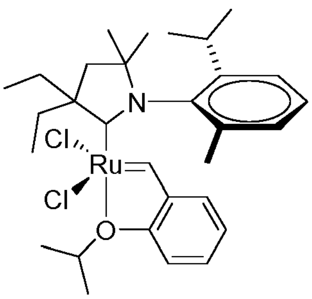, 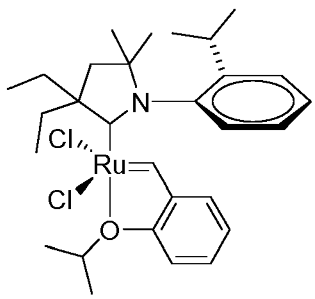, 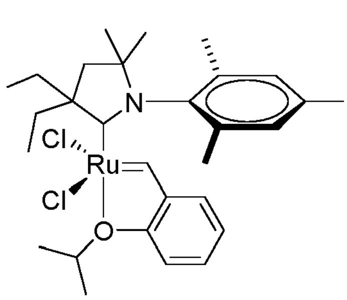,

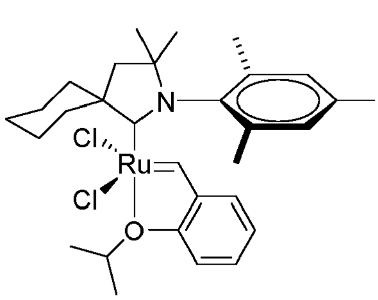, 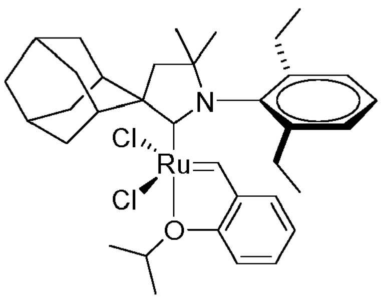, 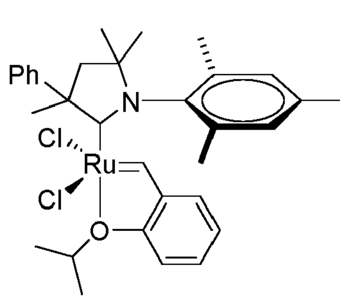,

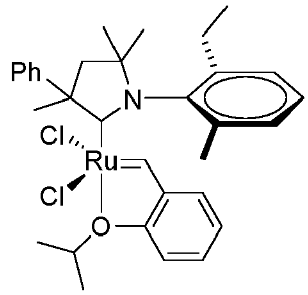, 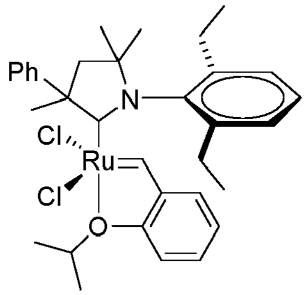, 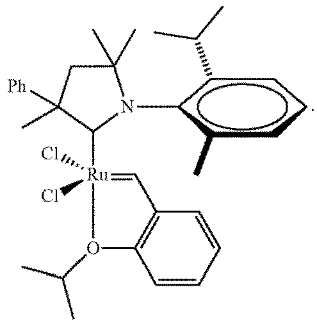,

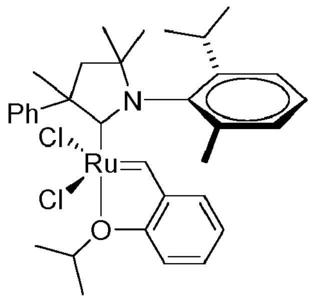.

In another embodiment, the invention relates to a compound (i.e., ruthenium complex bearing a cyclic alkyl amino carbene ligand) selected from:

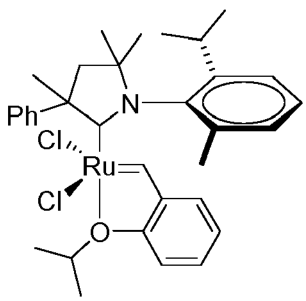, 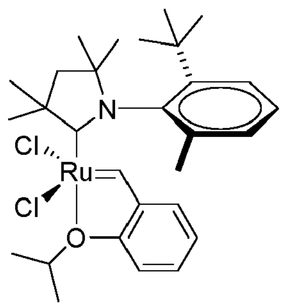,

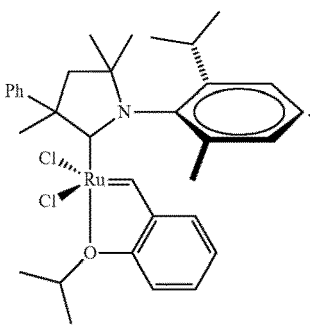, and 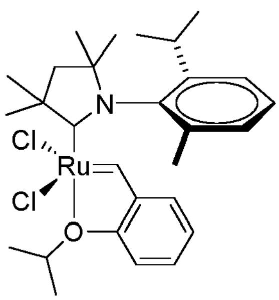.

In another embodiment, the invention relates to a compound (i.e., ruthenium complex bearing a cyclic alkyl amino carbene ligand) selected from:

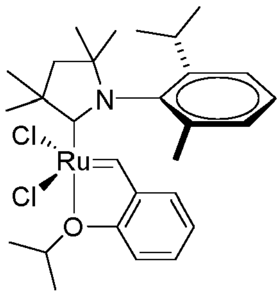

.

Exemplary Methods

In certain embodiments, the invention relates to a method of producing an olefin product comprising:

providing an olefinic substrate;
providing an alpha olefin;
contacting the olefinic substrate with the alpha olefin in the presence of any one of the compounds described herein (i.e., catalysts of the invention) under reaction conditions effective to allow a metathesis reaction to occur, thereby producing the olefin product.

In certain embodiments, the invention relates to any one of the methods described herein, wherein the olefin product is a terminal olefin product.

In certain embodiments, the olefinic substrate comprises at least one internal olefin, and may have 2 or more internal olefins. For example, the olefinic substrate may comprise in the range of 2 to about 15, 2 to about 10, or 2 to about 5 internal olefins. By "internal olefin" is meant an olefin wherein each of the olefinic carbons is substituted by at least one non-hydrogen substituent preferably a hydrocarbyl substituent. The non-hydrogen substituents are selected from unsubstituted hydrocarbyl, substituted hydrocarbyl, and functional groups. The internal olefin is therefore at least disubstituted, and may further include additional non-hydrogen substituents such that the internal olefin is tri- or tetra-substituted. Each of the substituents on the internal olefinic carbons may be further substituted as described herein. The internal olefin may be in the Z- or E-configuration. When the olefinic substrate comprises a plurality of internal olefins, the olefinic substrate may comprise a mixture of internal olefins (varying in stereochemistry and/or substituent identity), or may comprise a plurality of internal olefins.

The olefinic substrate may be a single compound or a mixture of compounds. The olefinic substrate may be hydrophobic or hydrophilic, although in a preferred embodiment, the olefinic substrate is hydrophobic. In certain embodiments, the olefinic substrate is a cyclic olefin.

As another example, the olefinic substrate is an ester of glycerol (a "glyceride"). In a preferred embodiment, the olefinic substrate comprises glycerol esterified with 1, 2, or 3 fatty acids, such that the olefinic substrate is a monoacylglycerol, diacylglycerol, or triacylglycerol (i.e., a monoglyceride, diglyceride, or triglyceride, respectively), or a mixture thereof. Each fatty acid-derived fragment of the olefinic substrate may independently be saturated, monounsaturated, or polyunsaturated, and may furthermore derive (or be derivable) from naturally-occurring fatty acids or from synthetic fatty acids. For example, the olefinic substrate may comprise glycerol esterified with one, two, or three fatty acids that are independently selected from $CH_3(CH_2)_nCOOH$, where n is 2, 3, 4, 5, 6, 7, 8, 9, 10, 11, 12, 13, 14, 15, 16, 17, 18, 19, 20, 21, or 22, palmitoleic acid, vaccenic acid, erucic acid, oleic acid, alpha linolenic acid, gamma-linolenic acid, linoleic acid, gadoleic acid, arachidonic acid, docosahexaenoic acid (i.e., DHA), and eicosapentaenoic acid (i.e., EPA). The olefinic substrate may be solid (e.g., a fat) or liquid (e.g., an oil).

Preferred glycerides that may be used as the olefinic substrate are seed oils, or are compounds that derive from seed oils. Preferred seed oil sources include soybean oil, sunflower oil, canola oil, safflower oil, cottonseed oil, castor oil, rape seed oil, peanut oil, corn oil, olive oil, palm oil, sesame oil, grape seed oil, algae oil, mustard oil, tung oil, perilla oil, linseed oil, pumpkin oil, cucumber oil, poppyseed oil, flax seed oil, walnut oil, and sesame oil.

Examples of seed oil sources include palm oil, grape seed oil, sunflower oil, canola oil, soybean oil. Examples of seed oil sources include palm oil, canola oil, and soybean oil. An example of a seed oil is soybean oil. An example of a seed oil is canola oil. An example of a seed oil is palm oil.

The olefinic substrate may be a compound or mixture of compounds that is derived from a glyceride using any one or combination of methods well known in the chemical arts. Such methods include saponification, esterification, hydrogenation, isomerization, oxidation, and reduction. For example, the olefinic substrate may the carboxylic acid or mixture of carboxylic acids that result from the saponification of a monoacylglycerol, diacylglycerol, triacylglycerol, or mixture thereof. In a preferred embodiment, the olefinic substrate is a fatty acid methyl ester (FAME), i.e., the methyl

ester of a carboxylic acid that is derived from a glyceride. Sunflower FAME, safflower FAME, soy FAME (i.e., methyl soyate), algal FAME, and canola FAME are examples of such olefinic substrates. In addition, in some embodiments the olefinic substrates include seed oil-derived compounds such as methyl oleate. In another embodiment, the invention comprises olefinic substrates such as sunflower FAME, safflower FAME, soy FAME (i.e., methyl soyate), algal FAME, canola FAME, myristoleic acid methyl ester, palmitoleic acid methyl ester, sapienic acid methyl ester, oleic acid methyl ester, elaidic acid methyl ester, vaccenic acid methyl ester, linoleic acid methyl ester, linoelaidic acid methyl ester, α-linolenic acid methyl ester, arachidonic acid methyl ester, eicosapentaenoic acid methyl ester, erucic acid methyl ester, docosahexaenoic acid methyl ester, methyl 9-decenoate acid methyl ester, methyl 9-undecenoate acid methyl ester, methyl 9-dodecenoate acid methyl ester, petroselinic acid methyl ester, sapienic acid methyl ester and methyl 9-tridecenoate acid methyl ester. In another embodiment, the invention comprises olefinic substrates such as myristoleic acid methyl ester, palmitoleic acid methyl ester, sapienic acid methyl ester, oleic acid methyl ester, elaidic acid methyl ester, vaccenic acid methyl ester, linoleic acid methyl ester, linoelaidic acid methyl ester, α-linolenic acid methyl ester, arachidonic acid methyl ester, eicosapentaenoic acid methyl ester, erucic acid methyl ester, docosahexaenoic acid methyl ester, methyl 9-decenoate acid methyl ester, methyl 9-undecenoate acid methyl ester, methyl 9-dodecenoate acid methyl ester, petroselinic acid methyl ester, sapienic acid methyl ester and methyl 9-tridecenoate acid methyl ester.

An example of olefinic substrates is palmitoleic acid methyl ester, soy fatty acid methyl ester (soy FAME), canola fatty acid methyl ester (canola FAME), sunflower fatty acid methyl ester (sunflower FAME).

An example of an olefinic substrate is sunflower fatty acid methyl ester (sunflower FAME). An example of an olefinic substrate is safflower fatty acid methyl ester (safflower FAME). An example of an olefinic substrate is soy fatty acid methyl ester (soy FAME).

An example of an olefinic substrate is algal fatty acid methyl ester (algal FAME).

An example of an olefinic substrate is canola fatty acid methyl ester (canola FAME).

An example of an olefinic substrate is palmitoleic acid methyl ester.

An example of an olefinic substrate is palmitoleic fatty acid methyl ester (palm FAME).

Soy FAME is a commondity feedstock, comprised of:

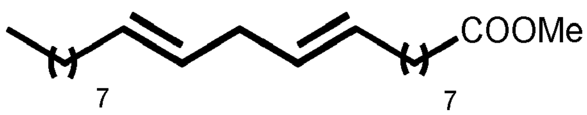

**methyl linoleate**

*56%*

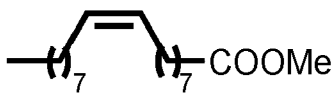

**methyl oleate**

*27%*

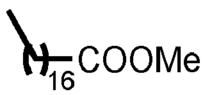

methyl stearate

*(6%)*

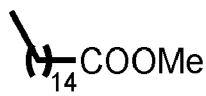

methyl palmitate

*(12%)*

The cross-metathesis partner that is reacted with the at least one internal olefin may be any olefinic compound that is capable of undergoing a metathesis reaction with the olefinic substrate to generate a terminal alkene product. The cross metathesis partner comprises an alpha olefin, wherein one olefinic carbon is unsubstituted and the other olefinic carbon is substituted with one or two non-hydrogen substituents. The substituted olefinic carbon may therefore be mono-substituted or di-substituted. The cross-metathesis partner may comprise a plurality of alpha olefins. A mixture of alpha olefins may be used.

Examples of monosubstituted alpha-olefins that may be used for the cross-metathesis partner include 1-propene, 1-butene, 1-pentene, 1-hexene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene and larger alpha olefins, 2-propenol, 3-butenol, 4-pentenol, 5-hexenol, 6-heptenol, 7-octenol, 8-nonenol, 9-decenol, 10-undecenol, 11-dodecenol, 12-tridecenol, 13-tetradecenol, 14-pentadecenol, 15-hexadecenol, 16-heptadecenol, 17-octadecenol, 18-nonadecenol, 19-eicosenol and larger alpha alkenols, 2-propenyl acetate, 3-butenyl acetate, 4-pentenyl acetate, 5-hexenyl acetate, 6-heptenyl acetate, 7-octenyl acetate, 8-nonenyl acetate, 9-decenyl acetate, 10-undecenyl acetate, 11-dodecenyl acetate, 12-tridecenyl acetate, 13-tetradecenyl acetate, 14-pentadecenyl acetate, 15-hexadecenyl acetate, 16-heptadecenyl acetate, 17-octadecenyl acetate, 18-nonadecenyl acetate, 19-eicosenyl acetate and larger alpha-alkenyl acetates, 2-propenyl chloride, 3-butenyl chloride, 4-pentenyl chloride, 5-hexenyl chloride, 6-heptenyl chloride, 7-octenyl chloride, 8-nonenyl chloride, 9-decenyl chloride, 10-undecenyl chloride, 11-dodecenyl chloride, 12-tridecenyl chloride, 13-tetradecenyl chloride, 14-pentadecenyl chloride, 15-hexadecenyl chloride, 16-heptadecenyl chloride, 17-octadecenyl chloride, 18-nonadecenyl chloride, 19-eicosenyl chloride and larger alpha-alkenyl chlorides, bromides, and iodides, allyl cyclohexane, allyl cyclopentane, and the like.

Examples of monosubstituted alpha olefins that may be used for the cross-metathesis partner include 1-propene, 1-butene, 1-hexene, 1-octene, 1-decene.

Examples of monosubstituted alpha olefins that may be used for the cross-metathesis partner include 1-propene and 1-butene. An example of a monosubstituted alpha olefin that may be used for the cross-metathesis partner includes 1-butene.

Examples of disubstituted alpha-olefins that may be used for the cross-metathesis partner include isobutylene, 2-methylbut-1-ene, 2-methylpent-1-ene, 2-methylhex-1-ene, 2-methylhept-1-ene, 2-methyloct-1-ene, and the like.

The components of the reactions of the present disclosure may be combined in any order, and it will be appreciated that the order of combining the reactants may be adjusted as needed. For example, the olefinic substrate may be added to the cross-metathesis partner, followed by addition of the catalyst. Alternatively, the olefinic substrate and cross-metathesis partner may be added to the catalyst. When one of the reactants is a gas, it may be necessary to add the catalyst to the liquid or solid reactant before introducing the gaseous reactant.

The catalyst may be added to the reaction either as a solid, dissolved in one of the reactants, or dissolved in a solvent. The catalyst may be added in any quantity and manner effective for the intended results of the reaction. For example, predetermined amounts of catalyst can be sequentially added to the reaction mixture at predetermined time intervals.

The reactions of the present disclosure may be carried out in a solvent, and any solvent that is inert towards cross metathesis may be employed. Generally, solvents that may be used in the cross-metathesis reactions include organic, protic, or aqueous solvents, such as aromatic hydrocarbons, chlorinated hydrocarbons, ethers, aliphatic hydrocarbons, alcohols, Water, or mixtures thereof. Example solvents include benzene, toluene, p-xylene, methylene chloride, 1,2-dichloroethane, dichlorobenzene, chlorobenzene, tetrahydrofuran, diethylether, pentane, methanol, ethanol, water, or mixtures thereof. In certain embodiments, the reactions of the present disclosure are carried out neat, i.e., without the use of a solvent.

The temperature at which a cross metathesis reaction according to the present disclosure is conducted can be adjusted as needed, and may be at least about -78° C, about -40° C, about -10° C, about 0° C, about 10° C, about 20° C, about 25° C, about 35° C, about 50° C, about 100° C, or about 150° C.

The product(s) of the reactions according to the present disclosure can be purified by any of the methods commonly known and used in the art, including, for example, distillation and crystallization. Any excess of alpha-olefin (which is a terminal olefin or alkene with a chemical formula $RR'C=CH_2$, where R and R' are each independently H, alkyl, aryl, heteroalkyl, heteroaryl, alkoxy, alkenyl, alkynyl, or acyl) after the reaction is completed can be separated from the final reaction mixture and recycled. For example, an alpha olefin of the formula $RR'C=CH_2$, where R and R' are each independently H, corresponds to the alpha olefin being ethylene. In other words, a particular alpha olefin for use in the present invention is ethylene. In one embodiment, the alpha olefin of the formula $RR'C=CH_2$, where R and R' are each independently H, has a purity of greater than 95%. In one embodiment, the alpha olefin of the formula $RR'C=CH_2$, where R and R' are each independently H, has a purity of greater than 99%. In another embodiment, the alpha olefin of the formula $RR'C=CH_2$, where R and R' are each independently H, has a purity selected from 99.95% and 99.995%. In another embodiment, the alpha olefin of the formula $RR'C=CH_2$, where R and R' are each independently H, has a purity of 99.95%. In another embodiment, the alpha olefin of the formula $RR'C=CH_2$, where R and R' are each independently H, has a purity of 99.995%. In another embodiment, the alpha olefin of the formula $RR'C=CH_2$, where R and R' are each independently H, has a purity of at least 99.95%. In another embodiment, the alpha olefin of the formula $RR'C=CH_2$, where R and R' are each independently H, has a purity of at least 99.995%. In another embodiment, the ethylene has a purity of greater than 95%. In another embodiment, the ethylene has a purity of greater than 99%. In another embodiment, the ethylene has a purity selected from 99.95% and 99.995%. In another embodiment, the ethylene has a purity of 99.95%. In another embodiment, the ethylene has a purity of 99.995%. In another embodiment, the ethylene has a purity of at least 99.95%. In another embodiment, the ethylene has a purity of at least 99.995%.

In another embodiment, the alpha olefin has a purity of greater than 90%. In another embodiment, the alpha olefin has a purity of greater than 95%. In another embodiment, the alpha olefin has a purity of greather than 99%.

Furthermore, any internal olefins present in the final reaction mixture can be separated, optionally purified, and recycled or used in a different reaction. A mixture of terminal olefins and internal olefins (e.g., terminally unsaturated esters and internally unsaturated esters) produced by the processes described herein can be separated from the final reaction mixture and used in a subsequent reaction. For example, such products may be used in another metathesis process (e.g., a metathesis process wherein the terminally unsaturated esters and internally unsaturated esters are converted into diesters).

In certain embodiments, the invention relates to any one of the aforementioned methods, wherein the alpha olefin and the olefinic substrate are part of the same molecule. In certain embodiments, the alpha olefin and the internal olefin are separated by a covalent linker.

In certain embodiments, the catalyst is present in an amount ranging from about 1 ppm to about 50 ppm relative to the number of olefinic substrate double bonds. Generally, preferred catalyst amounts of about 1 to 10 ppm can be used.

According to an aspect of the invention, the reaction is carried out by contacting, under an inert atmosphere and in the presence of a ruthenium alkylidene metathesis catalyst, an olefinic substrate comprising at least one internal olefin with a cross-metathesis partner comprising an alpha olefinic reactant, under reaction conditions effective to allow cross-metathesis to occur.

According to another aspect of the invention, the reaction is carried out by contacting, in an oxygen-containing atmosphere and in the presence of a ruthenium alkylidene metathesis catalyst, an olefinic substrate comprising at least one internal olefin with a cross metathesis partner comprising an alpha olefinic reactant, under reaction conditions effective to allow cross metathesis to occur.

In certain embodiments, the reaction is carried out by contacting, in the presence of a ruthenium alkylidene metathesis catalyst, an olefinic substrate comprising a mixture of monoglycerides, diglycerides, and triglycerides, with a cross metathesis partner comprising an alpha olefinic reactant, under reaction conditions effective to allow cross-metathesis to occur.

According to another aspect of the invention, the reaction is carried out by contacting, under an inert atmosphere and in the presence of a ruthenium alkylidene metathesis catalyst, an olefinic substrate comprising at least one internal olefin with a cross metathesis partner comprising an alpha olefinic reactant, under reaction conditions effective to allow cross-metathesis to occur, wherein the moles of the olefinic substrate is approximately equal to 1 to 9 times the moles of the cross metathesis partner.

According to a further aspect, the reaction is carried out by contacting, in the presence of a ruthenium alkylidene metathesis catalyst, an olefinic substrate comprising at least one internal olefin with a cross metathesis partner comprising an alpha olefinic reactant, with a cross metathesis partner comprising an alpha olefinic reactant, under reaction conditions effective and to allow cross-metathesis to occur, wherein the internal olefin has a molecular weight (MW) of at least 250 g/mol, or has at least 7 carbon atoms. Preferably the internal olefin has a molecular weight from about 300 g/mol to about 1000 g/mol, or has from 20 to 60 carbons. Such high-MW internal olefin substrates can include readily available, inexpensive olefins or mixtures of olefins such as unsaturated or polyunsaturated triacylglycerides obtained from plant or animal oils, high-boiling petrochemical fractions, or elastomeric or other unsaturated polymers (e.g., polybutadienes or polyisoprenes). Mixtures of such high-MW olefins are typically difficult to separate due to their high boiling points and relatively similar boiling point ranges. They can also be difficult to purify because of the high temperatures required for distillation. Cross-metathesis of these high-MW substrates can be efficiently performed with alpha olefins, especially lower alpha olefins, to produce lower-MW products that can be more easily separated or purified. In preferred embodiments, the high-MW internal olefin substrate is a triacylglyceride or a mixture of triacylglycerides. Such compounds could be saponified to mixtures of lower MW FAMEs, although such mixtures are still difficult to separate. If mixed FAMEs are used as substrates for cross metathesis, very complex mixtures of products are obtained. However, cross-metathesis of triacylglycerides can be efficiently performed with alpha olefins. By proper selection of the alpha-olefin, in particularly using lower alpha olefins such as propene or butene, relatively low-MW hydrocarbon olefins are produced that can be easily separated from the metathesized triacylglyceride fragment. In a subsequent step, the remaining triacylglyceride fragment can be saponified to yield unsaturated carboxylate compounds.

In a preferred embodiment, the turnover number (TON), defined as the moles of terminal olefin product formed per mol of catalyst, of the process is at least 50,000, preferably at least 100,000, preferably at least 500,000.

In a preferred embodiment, the yield, defined as the moles of terminal olefin product formed per mol of olefinic substrate, is 30% or more, preferably 40% or more, preferably 45% or more, preferably 50% or more, preferably 55% or more, preferably 60% or more.

## Exemplary Products

In certain embodiments, the invention relates to an olefin product produced by any one of the methods described herein.

## Further Definitions

The term "heteroaralkyl," as used herein, refers to an alkyl group substituted with a heteroaryl group.

Heteroaryl groups include, for example, pyrrole, furan, thiophene, imidazole, oxazole, thiazole, pyrazole, pyridine, pyrazine, pyridazine, quinoline, and pyrimidine, and the like.

The term "heteroatom," as used herein, means an atom of any element other than carbon or hydrogen. Preferred

heteroatoms are nitrogen, oxygen, and sulfur, most preferably oxygen and nitrogen.

The term "heterocyclyl" refers to substituted or unsubstituted non-aromatic ring structures, preferably 3- to 10-membered rings, more preferably 3- to 7-membered rings, whose ring structures include at least one heteroatom, preferably one to four heteroatoms, more preferably one or two heteroatoms. Heterocyclyl groups include, for example, piperidine, piperazine, pyrrolidine, morpholine, lactones, lactams, and the like.

Additional Embodiments

According to one embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula V, wherein the compound of Formula V has the structure:

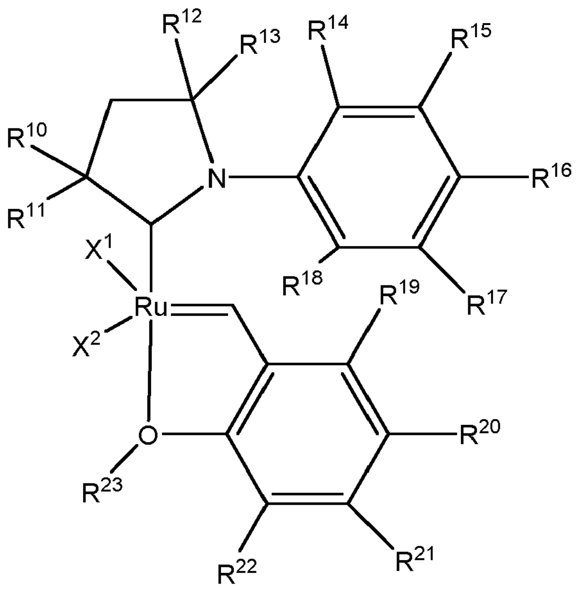

wherein:

$X^1$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsulfonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;
$X^2$ is an anionic ligand, $C_1$-$C_{20}$ alkoxy, halo, $C_1$-$C_{20}$ alkyl, $C_5$-$C_{24}$ aryl, $C_1$-$C_{20}$ alkoxy, $C_5$-$C_{24}$ aryloxy, $C_2$-$C_{20}$ alkoxycarbonyl, $C_6$-$C_{24}$ aryloxycarbonyl, $C_2$-$C_{24}$ acyl, $C_2$-$C_{24}$ acyloxy, $C_1$-$C_{20}$ alkylsulfonato, $C_5$-$C_{24}$ arylsul-

fonato, $C_1$-$C_{20}$ alkylsulfanyl, $C_5$-$C_{24}$ arylsulfanyl, $C_1$-$C_{20}$ alkylsulfinyl, $NO_3$, -N=C=O, -N=C=S, or $C_5$-$C_{24}$ arylsulfinyl;

$R^{10}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{11}$ can form a form a five-, six-, or ten-membered cycloalkyl or heterocyclyl ring, with the carbon atom to which they are attached;

$R^{11}$ is H, $C_1$-$C_{12}$ alkyl, $C_4$-$C_{12}$ cycloalkyl, $C_5$-$C_{24}$ aryl, $C_6$-$C_{24}$ aralkyl, $C_1$-$C_{20}$ heteroalkyl, or together with $R^{10}$ can form a form a five-, six-, or ten-membered cycloalkyl or heterocyclyl ring, with the carbon atom to which they are attached;

$R^{12}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{13}$ is H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl;

$R^{14}$, $R^{15}$, $R^{16}$, $R^{17}$ and $R^{18}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl (-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N(Ci-C24 alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl);

$R^{19}$, $R^{20}$, $R^{21}$ and $R^{22}$ are independently selected from H, $C_1$-$C_{12}$ alkyl, $C_4$-$C_{12}$ cycloalkyl, $C_5$-$C_{24}$ aryl, $C_5$-$C_{24}$ heteroaryl, $C_6$-$C_{24}$ aralkyl, or $C_6$-$C_{24}$ heteroaralkyl halo, hydroxyl, sulfhydryl, $C_1$-$C_{24}$ alkoxy, $C_2$-$C_{24}$ alkenyloxy, $C_2$-$C_{24}$ alkynyloxy, $C_5$-$C_{24}$ aryloxy, $C_6$-$C_{24}$ aralkyloxy, $C_6$-$C_{24}$ alkaryloxy, acyl (including $C_2$-$C_{24}$ alkylcarbonyl (-CO-alkyl) and $C_6$-$C_{24}$ arylcarbonyl (-CO-aryl)), acyloxy (-O-acyl, including $C_2$-$C_{24}$ alkylcarbonyloxy (-O-CO-alkyl) and $C_6$-$C_{24}$ arylcarbonyloxy (-O-CO-aryl)), $C_2$-$C_{24}$ alkoxycarbonyl (-(CO)-O-alkyl), $C_6$-$C_{24}$ aryloxycarbonyl (-(CO)-O-aryl), halocarbonyl (-CO)-X where X is halo), $C_2$-$C_{24}$ alkylcarbonato (-O-(CO)-O-alkyl), $C_6$-$C_{24}$ arylcarbonato (-O-(CO)-O-aryl), carboxy (-COOH), carboxylato ($-COO^-$), carbamoyl (-(CO)-$NH_2$), mono-($C_1$-$C_{24}$ alkyl)-substituted carbamoyl (-(CO)-NH($C_1$-$C_{24}$ alkyl)), di-($C_1$-$C_{24}$ alkyl)-substituted carbamoyl (-(CO)-N($C_1$-$C_{24}$ alkyl)$_2$), mono-($C_1$-$C_{24}$ haloalkyl)-substituted carbamoyl (-(CO)-NH($C_1$-$C_{24}$ haloalkyl)), di-($C_1$-$C_{24}$ haloalkyl)-substituted carbamoyl (-(CO)-N($C_1$-$C_{24}$ haloalkyl)$_2$), mono-($C_5$-$C_{24}$ aryl)-substituted carbamoyl (-(CO)-NH-aryl), di-($C_5$-$C_{24}$ aryl)-substituted carbamoyl (-(CO)-N($C_5$-$C_{24}$ aryl)$_2$), di-N-($C_1$-$C_{24}$ alkyl),N-($C_5$-$C_{24}$ aryl)-substituted carbamoyl (-(CO)-N($C_1$-$C_{24}$ alkyl)($C_5$-$C_{24}$ aryl), thiocarbamoyl (-(CS)-$NH_2$), mono-($C_1$-$C_{24}$ alkyl)-substituted thiocarbamoyl (-(CS)-NH($C_1$-$C_{24}$ alkyl)), di-($C_1$-$C_{24}$ alkyl)-substituted thiocarbamoyl (-(CS)-N($C_1$-$C_{24}$ alkyl)$_2$), mono-($C_5$-$C_{24}$ aryl)-substituted thiocarbamoyl (-(CS)-NH-aryl), di-($C_5$-$C_{24}$ aryl)-substituted thiocarbamoyl (-(CS)-N($C_5$-$C_{24}$ aryl)$_2$), di-N-($C_1$-$C_{24}$ alkyl), N-($C_5$-$C_{24}$ aryl)-substituted thiocarbamoyl

(-(CS)-N($C_1$-$C_{24}$ alkyl)($C_5$-$C_{24}$ aryl), carbamido (-NH-(CO)-$NH_2$), cyano (-C≡N), cyanato (-O-C≡N), thiocyanato (-S-C≡N), isocyanate (-N=C=O), thioisocyanate (-N=C=S), formyl (-(CO)-H), thioformyl (-(CS)-H), amino (-$NH_2$), mono-(Ci-$C_{24}$ alkyl)-substituted amino (-NH($C_1$-$C_{24}$ alkyl), di-($C_1$-$C_{24}$ alkyl)-substituted amino (-N($C_1$-$C_{24}$ alkyl)$_2$), mono-($C_5$-$C_{24}$ aryl)-substituted amino (-NH($C_5$-$C_{24}$ aryl), di-($C_5$-$C_{24}$ aryl)-substituted amino (-N($C_5$-$C_{24}$ aryl)$_2$), $C_2$-$C_{24}$ alkylamido (-NH-(CO)-alkyl), $C_6$-$C_{24}$ arylamido (-NH-(CO)-aryl), imino (-CR=NH where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), $C_2$-$C_{20}$ alkylimino (-CR=N(alkyl), where R includes without limitation hydrogen, $C_1$-$C_{24}$ alkyl, $C_5$-$C_{24}$ aryl, $C_6$-$C_{24}$ alkaryl, $C_6$-$C_{24}$ aralkyl, etc.), arylimino (-CR=N(aryl), where R includes without limitation hydrogen, $C_1$-$C_{20}$ alkyl, $C_5$-$C_{24}$ aryl, $C_6$-$C_{24}$ alkaryl, $C_6$-$C_{24}$ aralkyl, etc.), nitro (-$NO_2$), nitroso (-NO), sulfo (-$SO_2$-OH), sulfonato (-$SO_2$-$O^-$), $C_1$-$C_{24}$ alkylsulfanyl (-S-alkyl; also termed "alkylthio"), $C_5$-$C_{24}$ arylsulfanyl (-S-aryl; also termed "arylthio"), $C_1$-$C_{24}$ alkylsulfinyl (-(SO)-alkyl), $C_5$-$C_{24}$ arylsulfinyl (-(SO)-aryl), $C_1$-$C_{24}$ alkylsulfonyl (-$SO_2$-alkyl), $C_1$-$C_{24}$ monoalkylaminosulfonyl (-$SO_2$-N(H) alkyl), $C_1$-$C_{24}$ dialkylaminosulfonyl (-$SO_2$-N(alkyl)$_2$), $C_5$-$C_{24}$ arylsulfonyl (-$SO_2$-aryl), boryl (-$BH_2$), borono (-B(OH)$_2$), boronato (-B(OR)$_2$ where R includes without limitation alkyl or other hydrocarbyl), phosphono (-P(O)(OH)$_2$), phosphonato (-P(O)($O^-$)$_2$), phosphinato (-P(O)($O^-$)), phospho (-$PO_2$), and phosphino (-$PH_2$); and the hydrocarbyl moieties $C_1$-$C_{24}$ alkyl (preferably $C_1$-$C_{12}$ alkyl, more preferably $C_1$-$C_6$ alkyl), $C_2$-$C_{24}$ alkenyl (preferably $C_2$-$C_{12}$ alkenyl, more preferably $C_2$-$C_6$ alkenyl), $C_2$-$C_{24}$ alkynyl (preferably $C_2$-$C_{12}$ alkynyl, more preferably $C_2$-$C_6$ alkynyl), $C_5$-$C_{24}$ aryl (preferably $C_5$-$C_{14}$ aryl), $C_6$-$C_{24}$ alkaryl (preferably $C_6$-$C_{16}$ alkaryl), and $C_6$-$C_{24}$ aralkyl (preferably $C_6$-$C_{16}$ aralkyl); and

$R^{23}$ is H, $C_1$-$C_{12}$ alkyl, $C_4$-$C_{12}$ cycloalkyl, $C_5$-$C_{24}$ aryl or $C_6$-$C_{24}$ aralkyl.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula I, wherein the compound of Formula I has the structure:

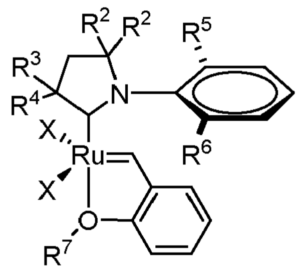

wherein,

X is independently for each occurrence a monovalent ligand;
$R^2$ is independently for each occurrence alkyl;
$R^3$ is alkyl;
$R^4$ is alkyl, aryl, heteroaryl, aralkyl, or heteroaralkyl, or $R^3$ and $R^4$, taken together with the carbon atom to which they are attached, form a five-, six-, or ten-membered cycloalkyl or heterocyclyl ring;
$R^5$ is alkyl;
$R^6$ is H or alkyl provided that $R^5$ and $R^6$ are not the same, and $R^6$ has fewer atoms than $R^5$; and
$R^7$ is alkyl.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;

providing at least one alpha olefin; and

contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,

wherein the ruthenium complex bearing a cyclic alkyl amino carbene ligand is a compound of Formula I, wherein the compound of Formula I is selected from

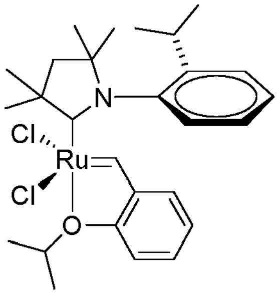
,
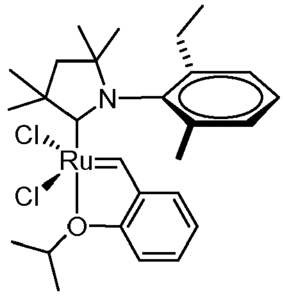
,
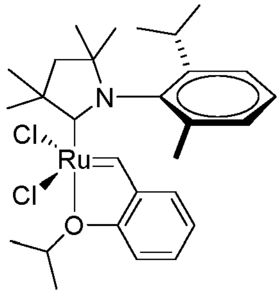
,
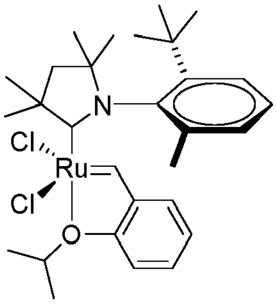
,
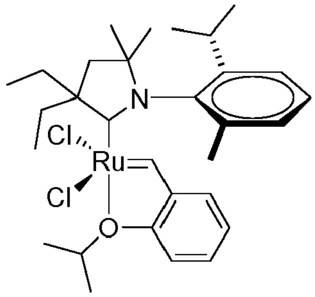
,
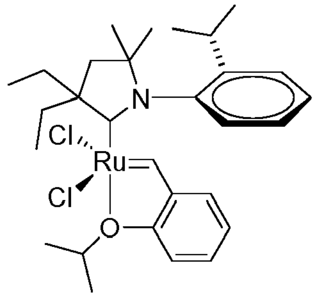
,
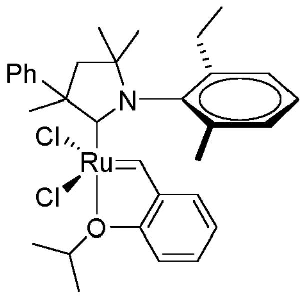
,
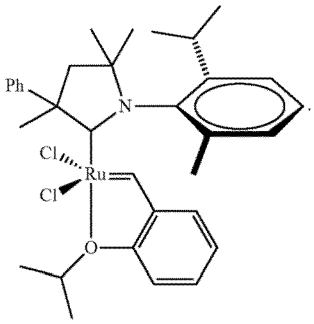

and

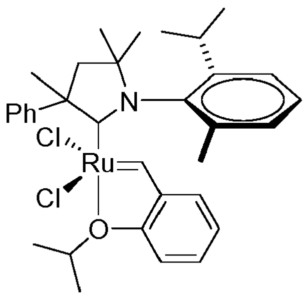
.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;

providing at least one alpha olefin; and

contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,

wherein the ruthenium complex bearing a cyclic alkyl amino carbene ligand is a compound of Formula I, wherein the compound of Formula I is selected from

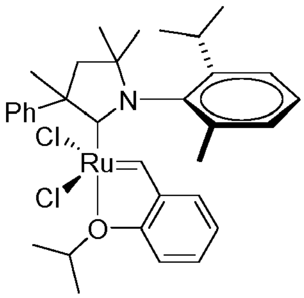
,
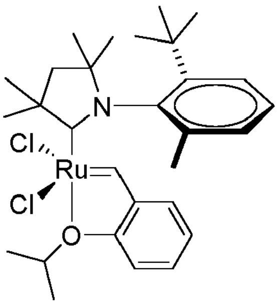
,
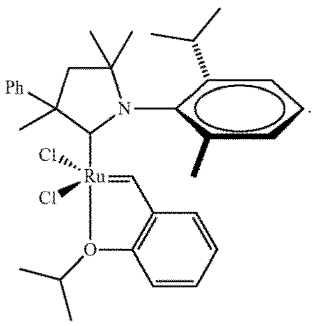
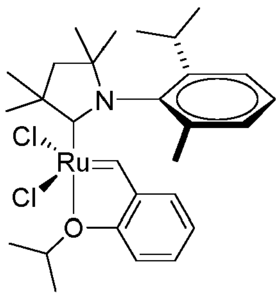
, and .

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;

providing at least one alpha olefin; and

contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,

wherein the ruthenium complex bearing a cyclic alkyl amino carbene ligand is a compound of Formula I, wherein the compound of Formula I is

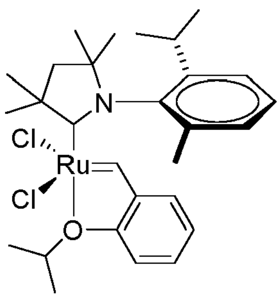
.

According to another embodiment, the invention is directed to a method of producing at least one olefin product

comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula II, wherein the compound of Formula II has the structure:

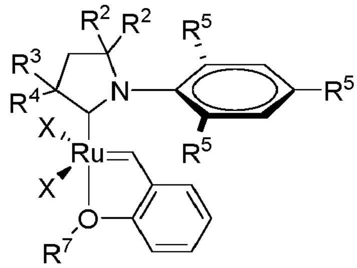

wherein,

X is, independently for each occurrence, a monovalent ligand;
$R^2$ is, independently for each occurrence, alkyl;
$R^3$ is alkyl;
$R^4$ is alkyl, aryl, heteroaryl, aralkyl, or heteroaralkyl, or $R^3$ and $R^4$, taken together with the carbon atom to which they are attached, form a five-, six-, or ten-membered cycloalkyl or heterocyclyl ring;
$R^5$ is, independently for each occurrence, alkyl; and
$R^7$ is alkyl.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula II, wherein the compound of Formula II is selected from

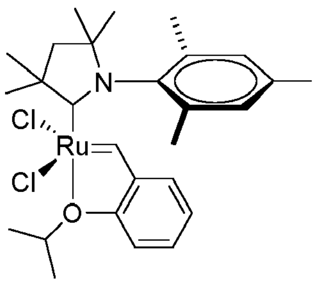
,
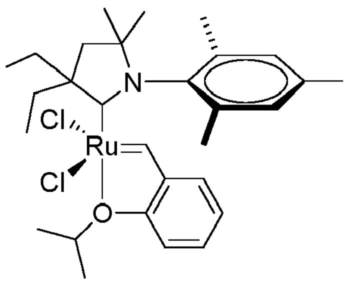
,
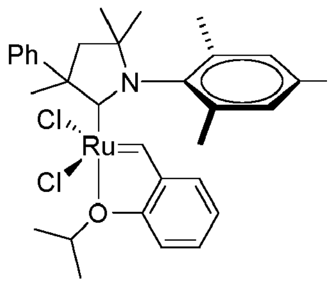

and

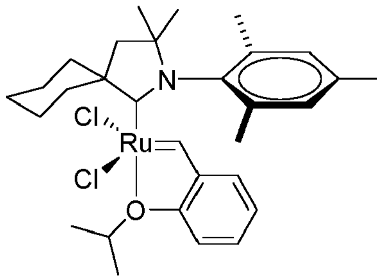

.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula III, wherein the compound of Formula III has the structure:

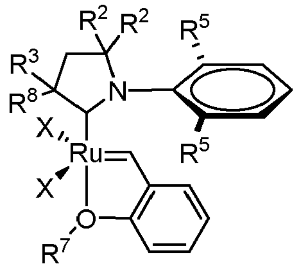

wherein,

X is, independently for each occurrence, a monovalent ligand;
$R^2$ is alkyl;
$R^3$ is alkyl;
$R^5$ is, independently for each occurrence, alkyl;
$R^7$ is alkyl; and
$R^8$ is aryl or heteroaryl.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula III, wherein the compound of Formula III is

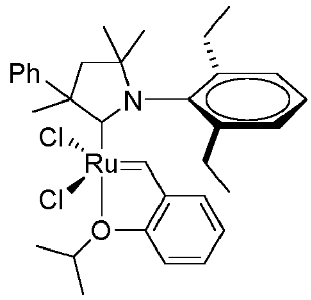

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula IV, wherein the compound of Formula IV has the structure:

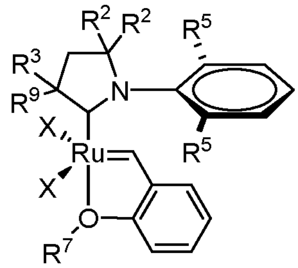

wherein,

X is, independently for each occurrence, a monovalent ligand;
$R^2$ is alkyl;
$R^3$ is alkyl;
$R^5$ is, independently for each occurrence, alkyl;
$R^7$ is alkyl; and
$R^9$ is $C_2$-$C_6$ alkyl, or $R^3$ and $R^9$, taken together with the carbon atom to which they are attached, form a five-, or ten-membered cycloalkyl or heterocyclyl ring.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is a compound of Formula IV, wherein the compound of Formula IV is selected from

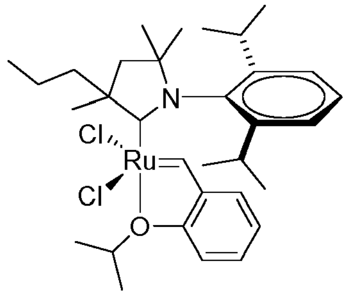, and 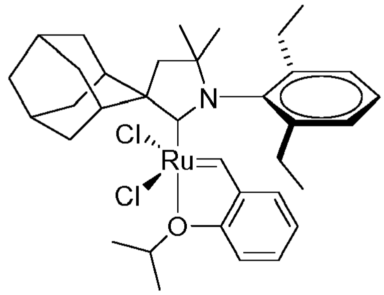.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is a monosubstituted alpha olefin.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is a monosubstituted alpha olefin selected from l-propene, 1-butene, 1-pentene, 1-hexene, 1-heptene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene and larger alpha olefins, 2-propenol, 3-butenol, 4-pentenol, 5-hexenol, 6-heptenol, 7-octenol, 8-nonenol, 9-decenol, 10-undecenol, 11-dodecenol, 12-tridecenol, 13-tetradecenol, 14-pentadecenol, 15-hexadecenol, 16-heptadecenol, 17-octadecenol, 18-nonadecenol, 19-eicosenol and larger alpha alkenols, 2-propenyl acetate, 3-butenyl acetate, 4-pentenyl acetate, 5-hexenyl acetate, 6-heptenyl acetate, 7-octenyl acetate, 8-nonenyl acetate, 9-decenyl acetate, 10-undecenyl acetate, 11-dodecenyl acetate, 12-tridecenyl acetate, 13-tetradecenyl acetate, 14-pentadecenyl acetate, 15-hexadecenyl acetate, 16-heptadecenyl acetate, 17-octadecenyl acetate, 18-nonadecenyl acetate, 19-eicosenyl acetate and larger alpha-alkenyl acetates, 2-propenyl chloride, 3-butenyl chloride, 4-pentenyl chloride, 5-hexenyl chloride, 6-heptenyl chloride, 7-octenyl chloride, 8-nonenyl chloride, 9-decenyl chloride, 10-undecenyl chloride, 11-dodecenyl chloride, 12-tridecenyl chloride, 13-tetradecenyl chloride, 14-pentadecenyl chloride, 15-hexadecenyl chloride, 16-heptadecenyl chloride, 17-octadecenyl chloride, 18-nonadecenyl chloride, 19-eicosenyl chloride and larger alpha-alkenyl chlorides, bromides, and iodides, allyl cyclohexane, allyl cyclopentane, and the like.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is a monosubstituted alpha olefin selected from 1-propene and 1-butene.

According to another embodiment, the invention is directed to a method of producing at least one olefin product

comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is a monosubstituted alpha olefin selected from 1-butene.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one olefinic substrate is soy FAME

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is of the formula $RR'C=CH_2$, wherein R and R' are each independently H, alkyl, aryl, heteroalkyl, heteroaryl, alkoxy, alkenyl, alkynyl, or acyl.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is of the formula $RR'C=CH_2$, wherein R and R' are each independently H.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is of the formula $RR'C=CH_2$, wherein R and R' are each independently H, and wherein the at least one alpha olefin has a purity of greater than 99%.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is of the formula $RR'C=CH_2$, wherein R and R' are each independently H, and wherein the at least one alpha olefin has a purity selected from 99.95% or 99.995%.

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one olefinic substrate is soy FAME

According to another embodiment, the invention is directed to a method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME, and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the at least one alpha olefin is of the formula $RR'C=CH_2$, wherein R and R' are each independently H, wherein the at least one alpha olefin has a purity selected from 99.95% and 99.995%, and wherein the at least one olefinic substrate is soy FAME

**Experimental**

Exemplification

The invention now being generally described, it will be more readily understood by reference to the following examples which are included merely for purposes of illustration of certain aspects and embodiments of the present invention, and are not intended to limit the invention.

General methods

All reactions were setup in a glovebox (Vacuum Atmospheres or mBraun) under a nitrogen atmosphere unless otherwise specified. All solvents were purified by passage through solvent purification columns, (Pangborn, A. B.; Giardello, M. A.; Grubbs, R. H.; Rosen, R. K.; Timmers, F. J. Organometallics 1996, 15, 1518.) and further degassed with argon. Ruthenium complex S1 was obtained from Materia Inc. CAAC salts 9a - 25a were prepared according to the methods reported in the literature.(Lavallo, V. ; Canac, Y. ; Prasang, C. ; Donnadieu, B. ; Bertrand, G. Angew. Chem. 2005, 117, 5851; Angew. Chem. Int. Ed. 2005, 44, 5705. Jazzar, R.; Dewhurst, R. D.; Bourg, J. B.; Donnadieu, B.; Canac, Y.; Bertrand, G. Angew. Chem. 2007, 119, 2957; Angew. Chem. Int. Ed. 2007, 46, 2899. Jazzar, R.; Bourg, J. B.; Dewhurst, R. D.; Donnadieu, B.; Bertrand, G. J. Org. Chem. 2007, 72, 3492. d) Anderson, D. R.; Lavallo, V.; O'Leary, D. J.; Bertrand, G.; Grubbs, R. H. Angew. Chem. 2007, 119, 7400; Angew. Chem. Int. Ed. 2007, 46, 7262. Mignani, G.; Pevere, V.; Olivier-Bourbigou, H.; Vallee, C.; Berthod, M.; Citadelle, C. (Rhodia Operations, IFP), WO2010/010290 A1, 2010.) CAAC catalysts 9 - 25 were prepared according to the literature procedures already established for previously reported catalysts 10, (Jazzar, R.; Dewhurst, R. D.; Bourg, J. B.; Donnadieu, B.; Canac, Y.; Bertrand, G. Angew. Chem. 2007, 119, 2957; Angew. Chem. Int. Ed. 2007, 46, 2899) 13, (Jazzar, R.; Dewhurst, R. D.; Bourg, J. B.; Donnadieu, B.; Canac, Y.; Bertrand, G. Angew. Chem. 2007, 119, 2957; Angew. Chem. Int. Ed. 2007, 46, 2899) 18, (Jazzar, R.; Dewhurst, R. D.; Bourg, J. B.; Donnadieu, B.; Canac, Y.; Bertrand, G. Angew. Chem. 2007, 119, 2957; Angew. Chem. Int. Ed.

2007, 46, 2899) and 25. (Jazzar, R.; Bourg, J. B.; Dewhurst, R. D.; Donnadieu, B.; Bertrand, G. J. Org. Chem. 2007, 72, 3492.) Methyl oleate was purchased from Nu-Chek-Prep, and subjected to further purification with activated neutral alumina. To activate, neutral alumina was heated to 200 °C for 24 h in a glassware drying oven, was then cooled to room temperature, and added to methyl oleate (1.5 wt%). The methyl oleate was then degassed, and stored over the activated neutral alumina for a minimum of one month before use. Ethylene gas was purchased and used as received from Matheson, and was either Ultra High Purity (99.95%) or Matheson Purity (99.995%). $^{1}H$-NMR spectra were acquired at 500 MHz and $^{13}C$-NMR spectra at 125 MHz as $CDCl_3$ solutions, on a Varian spectrometer equipped with an AutoX probe. ROESY-AD spectra were acquired at 600 MHz, using standard pulse sequences available in VNMR-J. Gas Chromatographic analyses were conducted using an Agilent 6850 GC equipped with a CTC-PAL autosampler. High-resolution mass spectrometry (HRMS) data was obtained on a JEOL MSRoute mass spectrometer. Elemental analysis was performed by Midwest Microlab, LLC.

GC Analytical Methods

Volatile products were analyzed using an Agilent 5890 gas chromatography (GC) instrument with a flame ionization detector (FID). The following conditions and equipment were used:

| | |
|---|---|
| Column: | HP-5, 30m x 0.25mm (ID) x 0.25μm film thickness. Manufacturer: J&W |
| GC and column conditions: | Injector temperature: 250°C |
| | Detector temperature: 280°C |
| Oven temperature: | Starting temperature: 100°C, hold time: 1 minute. |
| | Ramp rate 10°C/min to 250°C, hold time: 12 minutes Carrier gas: Helium |
| Mean gas velocity: | 31.3 ± 3.5% cm/sec (calculated) |
| Split ratio: | ~50:1 |

The products were characterized by comparing peaks with known standards, in conjunction with supporting data from mass spectrum analysis (GCMS-Agilent 5973N). GCMS analysis was accomplished with a second HP-5, 30m x 0.25mm (ID) x 0.25μm film thickness GC column, using the same method as above.

*General procedure 1:* Synthesis of catalysts 9-25

In a glovebox, a vial was charged with dichloro(o-isopropoxyphenylmethylene) (tricyclohexylphosphine)ruthenium(II) (**S1**) (1 equiv), CAAC salt 9a - 25a (2.4 equiv), KHMDS (2.8 equiv), and THF (0.03 M). The vial was capped, brought out of the glovebox, and let to stir at room temperature. After 24 hours, the mixture was filtered over a pad of celite, eluted with $CH_2Cl_2$, and concentrated. The residue was then washed with hexanes, to provide the pure catalyst **(9-25)** as a dark green solid. If desired, the catalyst can be further purified by column chromatography ($SiO_2$, using 10% EtOAc in hexanes to 25% EtOAc in hexanes). (This is only a necessary purification step for the lower yielding catalysts **9** and **10.** Residual **S1** elutes first as a reddish-brown band, followed by the desired catalyst as a bright green band.)

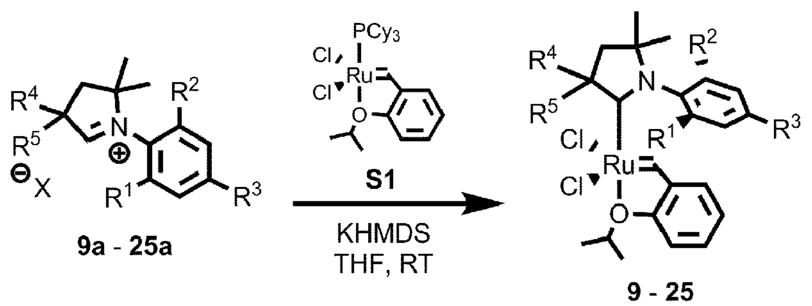


**9a** - **25a**

**9 - 25**

**Catalyst 9** ($R^1$ = Me, $R^2$ = Et, $R^3$ = H, $R^4$ = $R^5$ = Me)

According to *general procedure 1*: S1 (0.18 g, 0.30 mmol), 9a·$PF_6$ (0.28 g, 0.70 mmol), and KHMDS (0.17 g, 0.86 mmol) were reacted in THF (10 mL) to provide 9 (0.030 g, 15%) as a dark green solid; *(*Angew. Chem. Int. Ed. 2015, 54, 1919 -1923) $^{1}H$ NMR δ 16.25 (1H, s), 7.52 (1H, m), 7.50 (1H, m), 7.44 (1H, m), 7.28 (1H, m), 6.93 (1H, m), 6.86 (2H, m), 5.17 (1H, m), 2.60 (1H, m), 2.51 (1H, m), 2.29 (3H, s), 2.20 (2H, s), 2.16 (3H, s), 2.06 (3H, s), 1.80 (3H,

d, $J$ = 6.1 Hz), 1.72 (3H, d, $J$ = 6.1 Hz), 1.41 (3H, s), 1.34 (3H, s), 0.89 (3H, t, $J$ = 7.4 Hz); $^{13}C$ NMR δ 286.4, 266.5, 152.4, 144.3, 144.1, 139.5, 138.1, 130.9, 129.7, 128.9, 127.2, 123.8, 122.1, 113.2, 78.7, 75.1, 56.3, 52.2, 30.0, 29.5, 29.4, 28.3, 25.0, 22.3, 22.1, 20.8, 14.7; HRMS (FAB+) 563.1286, $[C_{27}H_{37}RuONCl_2]^+$ requires 563.1296.

**Catalyst 11** ($R^1$ = Me, $R^2$ = *i*Pr, $R^3$ = H, $R^4$ = $R^5$ = Me)

According to *general procedure 1*: S1 (0.72 g, 1.2 mmol), 11a·$BF_4$ (0.50 g, 1.4 mmol), and KHMDS (0.34 g, 1.7 mmol) were reacted in THF (40 mL) to provide 11 (0.54 g, 78%) as a dark green solid; *(*Angew. Chem. Int. Ed. 2015, 54, 1919 -1923) $^1H$ NMR δ 16.25 (1H, , s), 7.52 (2H, m), 7.47 (1H, m), 7.27 (1H, m), 6.93 (1H, m), 6.84 (2H, m), 5.17 (1H, m), 2.99 (1H, m), 2.28 (3H, s), 2.22 (1H, d, $J$ = 12 Hz), 2.18 (1H, d, $J$ = 12 Hz), 2.15 (3H, s), 2.07 (3H, s), 1.81 (3H, d, $J$ = 6.1 Hz), 1.74 (3H, d, $J$ = 6.1 Hz), 1.41 (3H, s), 1.37 (3H, s), 1.28 (3H, d, $J$ = 6.6 Hz), 0.69 (3H, d, $J$ = 6.6 Hz); $^{13}C$ NMR δ 297.2, 267.1, 152.6, 148.9, 143.8, 138.4, 138.3, 130.9, 129.7, 129.1, 125.8, 123.8, 122.1, 113.3, 78.4, 75.1, 56.3, 52.1, 29.8, 29.7, 29.6, 28.7, 28.5, 26.3, 24.2, 22.4, 22.1, 21.6; HRMS (FAB+) 577.1468, $[C_{28}H_{39}RuONCl_2]^+$ requires 577.1453.

**Catalyst 12** ($R^1$ = Me, $R^2$ = tBu, $R^3$ = H, $R^4$ = $R^5$ = Me)

According to *general procedure 1*: S1 (0.14 g, 0.24 mmol), 12a·$BF_4$ (0.10 g, 0.28 mmol), and KHMDS (0.068 g, 0.34 mmol) were reacted in THF (8 mL) to provide 12 (0.12 g, 86%) as a dark green solid; *(*Angew. Chem. Int. Ed. 2015, 54, 1919 -1923) $^1H$ NMR δ 17.06 (1H, s), 7.70 (1H, m), 7.52 (1H, m), 7.45 (1H, m), 7.24 (1H, m), 6.94 (1H, m), 6.88 (1H, m), 6.85 (1H, m), 5.17 (1H, m), 2.23 (1H, d, $J$ = 12 Hz), 2.18 (3H, s), 2.10 (1H, d, $J$= 12 Hz), 1.97 (3H, s), 1.83 (3H, d, $J$ = 6.1 Hz), 1.77 (3H, d, $J$ = 6.1 Hz), 1.37 (6H, s), 1.34 (9H, s); $^{13}C$ NMR δ 297.5, 266.1, 152.6, 148.4, 144.3, 138.8, 138.1, 131.0, 130.1, 130.1, 128.2, 123.9, 122.0, 113.4, 79.1, 75.2, 55.5, 52.7, 38.6, 33.8, 32.2, 32.2, 30.9, 28.6, 23.0, 22.5, 22.2 (2C); HRMS (FAB+) 591.1599, $[C_{29}H_{41}RuONCl_2]^+$ requires 591.1609.

**Catalyst 14** ($R^1$ = H, $R^2$ = *i*Pr, $R^3$ = H, $R^4$ = $R^5$ = Et)

According to *general procedure 1*: **S1** (0.18 g, 0.30 mmol), 14a·$PF_6$ (0.29 g, 0.70 mmol), and KHMDS (0.17 g, 0.86 mmol) were reacted in THF (10 mL) to provide 14 (0.080 g, 44%) as a dark green solid; *(*Angew. Chem. Int. Ed. 2015, 54, 1919 -1923) $^1H$ NMR δ 16.03 (1H, s), 7.65 (1H, m), 7.59 (1H, m), 7.53 (1H, m), 7.25 (1H, m), 7.21 (1H, m), 6.94 (1H, m), 6.86 (1H, m), 6.80 (1H, m), 5.12 (1H, m), 3.23 (1H, m), 2.87 (1H, m), 2.64 (1H, m), 2.44 (1H, m), 2.34 (1H, d, $J$ = 13 Hz), 2.03 (1H, d, $J$ = 13 Hz), 1.89 (1H, m), 1.70 (3H, d, $J$ = 6.1 Hz), 1.62 (3H, t, $J$ = 7.4 Hz), 1.37 (3H, s), 1.35 (3H, d, $J$ = 6.7 Hz), 1.34 (3H, s), 1.09 (3H, t, $J$ = 7.4 Hz), 1.04 (3H, d, $J$ = 6.7 Hz); $^{13}C$ NMR δ 301.7, 265.8, 152.7, 148.1, 144.2, 139.7, 131.7, 131.1, 129.4, 127.1, 127.0, 123.8, 122.2, 113.3, 75.3, 74.8, 65.6, 45.0, 33.3, 32.0, 28.6, 28.5, 27.4, 25.1, 23.3, 22.4, 22.4, 14.0, 10.0; HRMS (FAB+) 591.1584, $[C_{29}H_{41}RuONCl_2]^+$ requires 591.1609.

**Catalyst 15** ($R^1$ = $R^2$ = $R^3$ = Me, $R^4$ = $R^5$ = Et)

According to *general procedure 1*: S1 (0.21 g, 0.35 mmol), 15a·$BF_4$ (0.25 g, 0.70 mmol), and KHMDS (0.14 g, 0.70 mmol) were reacted in THF (10 mL) to provide 15 (0.10 g, 48%) as a dark green solid; *(*Angew. Chem. Int. Ed. 2015, 54, 1919 -1923) $^1H$ NMR δ 16.41 (1H, s), 7.55 (1H, m), 7.11 (2H, s), 6.95 (1H, m), 6.88 (1H, m), 6.79 (1H, m), 5.12 (1H, m), 2.86 (2H, m), 2.47 (3H, s), 2.19 (6H, s), 2.16 (2H, s), 1.80 (2H, m), 1.68 (6H, d, $J$ = 6.1 Hz), 1.52 (6H, t, $J$ = 7.4 Hz), 1.36 (6H, s); $^{13}C$ NMR δ 306.3, 266.7, 152.2, 145.2, 138.5, 138.2 (2C), 138.1, 131.4, 130.5 (2C), 124.4, 122.2, 113.5, 77.8, 74.6, 66.2, 45.5, 29.0 (2C), 27.7 (2C), 22.5 (2C), 21.2, 20.7 (2C), 13.1; HRMS (FAB+) 591.1608, $[C_{29}H_{41}RuONCl_2]^+$ requires 591.1609.

**Catalyst 16** ($R^1$ = Me, $R^2$ = *i*Pr, $R^3$ = H, $R^4$ = $R^5$ = Et)

According to *general procedure 1*: S1 (0.21 g, 0.35 mmol), 16a·$BF_4$ (0.26 g, 0.70 mmol), and KHMDS (0.14 g, 0.70 mmol) were reacted in THF (10 mL) to provide 16 (0.17 g, 82%) as a dark green solid; *(*Angew. Chem. Int. Ed. 2015, 54, 1919 -1923) $^1H$ NMR δ 16.45 (1H, s), 7.53 (2H, m), 7.27 (1H, m), 7.26 (1H, m), 6.94 (1H, m), 6.84 (1H, m), 6.76 (1H, m), 5.12 (1H, m), 2.93 (2H, m), 2.86 (1H, m), 2.25 (3H, s), 2.17 (2H, s), 1.87 (1H, m), 1.75 (3H, d, $J$ = 6.2 Hz), 1.74 (1H, m), 1.67 (3H, d, $J$ = 6.2 Hz), 1.53 (3H, t, $J$ = 7.4 Hz), 1.36 (3H, s), 1.31 (3H, s), 1.28 (3H, d, $J$ = 6.5 Hz), 0.68 (3H, d, $J$ = 6.5 Hz); $^{13}C$ NMR δ 303.3, 267.3, 152.6, 148.5, 144.2, 138.9, 138.1, 131.4, 129.8, 129.1, 125.8, 124.6, 122.1, 113.5, 77.5, 74.7, 66.1, 45.5, 29.9, 28.6, 28.5, 28.1, 27.8, 26.5, 24.3, 22.8, 22.3, 21.7, 13.1, 13.0; HRMS (FAB+) 605.1751, $[C_{30}H_{43}RuONCl_2]^+$ requires 605.1766.

**Catalyst 17** ($R^1 = R^2 = R^3$ = Me, $R^4/R^5 = -(CH_2)_5-$)

According to *general procedure 1*: S1 (0.18 g, 0.30 mmol), 17a·$PF_6$ (0.26 g, 0.70 mmol), and KHMDS (0.17 g, 0.86 mmol) were reacted in THF (10 mL) to provide 17 (0.070 mg, 39%) as a dark green solid; *(*Angew. Chem. Int. Ed. 2015, 54, 1919 -1923) $^1$H NMR $\delta$ 16.35 (1H, s), 7.55 (1H, m), 7.10 (2H, s), 6.93 (1H, m), 6.88 (1H, m), 6.86 (1H, m), 5.16 (1H, m), 3.24 (2H, m), 2.46 (3H, s), 2.29 (2H, s), 2.25 (2H, m), 2.20 (6H, s), 1.93 (2H, m), 1.80 (1H, m), 1.75 (6H, d, *J* = 6.1 Hz), 1.52 (3H, m), 1.40 (6H, s); $^{13}$C NMR $\delta$ 301.0, 266.3, 152.3, 144.8, 138.5, 138.2 (2C), 137.8, 131.0, 130.5 (2C), 123.9, 122.2, 113.3, 78.6, 74.9, 62.1, 44.9, 35.7 (2C), 29.5 (2C), 25.8, 23.2 (2C), 22.2 (2C), 21.2, 20.8 (2C); HRMS (FAB+) 603.1631, $[C_{30}H_{41}RuONCl_2]^+$ requires 603.1609.

**Catalyst 19** ($R^1 = R^2$ = *i*Pr, $R^3$ = H, $R^4$ = Me, $R^5$ = *n*Pr)

According to *general procedure 1*: S1 (0.18 g, 0.30 mmol), 19a·$BF_4$ (0.28 g, 0.70 mmol), and KHMDS (0.17 g, 0.86 mmol) were reacted in THF (10 mL) to provide 19 (0.15 g, 79%) as a dark green solid; *(*Angew. Chem. Int. Ed. 2015, 54, 1919 -1923) $^1$H NMR $\delta$ 16.50 (1H, s), 7.61 (1H, m), 7.50 (1H, m), 7.45 (2H, m), 6.94 (1H, m), 6.83 (1H, m), 6.78 (1H, m), 5.15 (1H, m), 3.02 (2H, m), 2.97 (1H, m), 2.21 (1H, d, *J* = 12 Hz), 2.14 (1H, d, *J*= 12 Hz), 1.97 (1H, m), 1.92 (3H, s), 1.81 (3H, d, *J* = 6.1 Hz), 1.75 (3H, d, *J* = 6.1 Hz), 1.60 (1H, m), 1.37 (3H, s), 1.29 (3H, d, *J* = 6.6 Hz), 1.28 (3H, s), 1.24 (3H, d, *J* = 6.6 Hz), 1.23 (3H, d, *J* = 6.6 Hz), 1.14 (3H, t, *J* = 7.2 Hz), 0.77 (3H, d, *J* = 6.4 Hz), 0.61 (3H, d, *J* = 6.4 Hz); $^{13}$C NMR $\delta$ 296.3, 267.5, 153.0, 148.6, 148.4, 143.1, 136.7, 130.8, 129.5, 125.9, 125.8, 123.9, 122.0, 113.4, 77.8, 75.0, 60.7, 49.7, 44.3, 31.4, 28.8, 28.6, 28.3, 27.2, 26.4, 25.2, 24.5, 24.4, 22.5, 22.2, 19.9, 14.9; HRMS (FAB+) 633.2080, $[C_{32}H_{47}RuONCl_2]^+$ requires 633.2079.

**Catalyst 20** ($R^1 = R^2$ = Et, $R^3$ = H, $R^4/R^5$ = bicyclo[3.3.1]nonane)

According to *general procedure 1*: S1 (0.15 g, 0.25 mmol), 20a·$BF_4$ (0.12 g, 0.30 mmol), and KHMDS (0.070 g, 0.35 mmol) were reacted in THF (10 mL) to provide 20 (0.13 g, 81%) as a dark green solid; *(*Angew. Chem. Int. Ed. 2015, 54, 1919 -1923) $^1$H NMR $\delta$ 16.90 (1H, s), 7.58 (2H, m), 7.46 (2H, m), 6.91 (2H, m), 6.86 (1H, m), 5.05 (1H, m), 3.91 (2H, m), 2.80 (2H, m), 2.52 (4H, m), 2.38 (1H, m), 2.36 (2H, s), 2.30 (2H, m), 2.17 (2H, m), 2.01 (1H, m), 1.99 (2H, m), 1.90 (2H, m), 1.66 (6H, d, *J* = 6.1 Hz), 1.28 (6H, s), 1.00 (6H, t, *J* = 7.4 Hz); $^{13}$C NMR $\delta$ 314.5, 271.7, 152.4, 145.6, 143.3 (2C), 140.1, 132.1, 128.9, 126.8 (2C), 125.3, 122.2, 113.8, 76.5, 74.5, 71.5, 48.0, 40.4 (2C), 39.3, 36.7 (2C), 35.3 (2C), 29.2 (2C), 27.7, 27.5, 24.9 (2C), 22.6 (2C), 14.3 (2C); HRMS (FAB+) 669.2066, $[C_{35}H_{47}RuONCl_2]^+$ requires 669.2079.

**Catalyst 21** ($R^1 = R^2 = R^3$ = Me, $R^4$ = Me, $R^5$ = Ph)

According to *general procedure 1*: S1 (0.18 g, 0.30 mmol), 21a·$BF_4$ (0.28 g, 0.70 mmol), and KHMDS (0.17 g, 0.86 mmol) were reacted in THF (10 mL) to provide 21 (0.055 g, 29%) as a dark green solid. *(*Angew. Chem. Int. Ed. 2015, 54, 1919 -1923) The $^1$H NMR spectrum shows two diagnostic benzylidene resonances at 17.72 and 16.34 ppm, in a ratio of 2.0:1.0 (due to the presence of conformational and rotational isomers, the $^1$H- and $^{13}$C-spectra of catalyst 21 are highly complex, which precluded further assignment);(ROESY spectra confirmed that these benzylidene resonances undergo chemical exchange) HRMS (FAB+) 625.1435, $[C_{32}H_{39}RuONCl_2]^+$ requires 625.1453. Elemental analysis (%) C 61.19, H 6.24, N 2.13, $[C_{32}H_{39}RuONCl_2]$ requires C 61.43, H 6.28, N 2.24.

**Catalyst 22** ($R^1$ = Me, $R^2$ = Et, $R^3$ = H, $R^4$ = Me, $R^5$ = Ph)

According to *general procedure 1*: S1 (0.18 g, 0.30 mmol), 22a·$BF_4$ (0.28 g, 0.70 mmol), and KHMDS (0.17 g, 0.86 mmol) were reacted in THF (10 mL) to provide 22 (0.060 g, 32%) as a dark green solid. *(*Angew. Chem. Int. Ed. 2015, 54, 1919 -1923) The $^1$H NMR spectrum shows two diagnostic benzylidene resonances at 17.71 and 16.32 ppm, in a ratio of 1.0:1.7 (due to the presence of conformational and rotational isomers, the $^1$H- and $^{13}$C-spectra of catalyst 22 are highly complex, which precluded further assignment); (ROESY spectra confirmed that these benzylidene resonances undergo chemical exchange) HRMS (FAB+) 625.1436, $[C_{32}H_{39}RuONCl_2]^+$ requires 625.1453. Elemental analysis (%) C 61.02, H 6.19, N 1.94, $[C_{32}H_{39}RuONCl_2]$ requires C 61.43, H 6.28, N 2.24.

**Catalyst 23** ($R^1 = R^2$ = Et, $R^3$ = H, $R^4$ = Me, $R^5$ = Ph)

According to *general procedure 1*: S1 (0.18 g, 0.30 mmol), 23a·$BF_4$ (0.29 g, 0.70 mmol), and KHMDS (0.17 g, 0.86 mmol) were reacted in THF (10 mL) to provide 23 (0.085 g, 45%) as a dark green solid. *(*Angew. Chem. Int. Ed. 2015, 54, 1919 -1923) The $^1$H NMR spectrum shows two diagnostic benzylidene resonances at 17.72 and 16.43 ppm,

in a ratio of 1.0:3.6. Due to the presence of conformational and rotational isomers, the $^{13}C$-spectra of catalyst 23 was highly complex, which precluded further assignment. Although the $^{1}H$-spectrum was also complex and exhibited significant line broadening, resonances corresponding to the major benzylidene were able to be extracted as follows: δ 16.43 (1H), 8.26 (2H), 7.56 (2H), 7.43 (5H), 6.86 (1H), 6.81 (1H), 6.74 (1H), 4.98 (1H), 3.15 (1H), 2.72 (2H), 2.53 (2H), 2.38 (1H), 2.36 (3H), 1.55 (3H), 1.52 (3H), 1.42 (3H), 1.41 (3H), 1.38 (3H), 1.07 (3H), 0.83 (3H);); (ROESY spectra confirmed that these benzylidene resonances undergo chemical exchange) HRMS (FAB+) 639.1598, $[C_{33}H_{41}RuONCl_2]^+$ requires 631.1609. Elemental analysis (%) C 62.46, H 6.41, N 1.94, $[C_{33}H_{41}RuONCl_2]$ requires C 61.96, H 6.46, N 2.19.

**Catalyst 24** ($R^1$ = Me, $R^2$ = *i*Pr, $R^3$ = H, $R^4$ = Me, $R^5$ = Ph)

According to *general procedure 1*: S1 (0.18 g, 0.30 mmol), 24a·$BF_4$ (0.29 g, 0.70 mmol), and KHMDS (0.17 g, 0.86 mmol) were reacted in THF (10 mL) to provide 24 (0.11 g, 58%) as a dark green solid. *(*Angew. Chem. Int. Ed. 2015, 54, 1919 -1923) The $^{1}H$ NMR spectrum shows two diagnostic benzylidene resonances at 16.36 and 16.34 ppm, in a ratio of 1.0:2.9. Due to the presence of conformational and rotational isomers, the $^{13}C$-spectra of catalyst 24 was highly complex, which precluded further assignment. Although the $^{1}H$-spectrum was also complex and exhibited significant line broadening, resonances corresponding to the major benzylidene were able to be extracted as follows: δ 16.34 (1H), 8.24 (2H), 7.51 (5H), 7.38 (1H), 7.29 (1H), 6.85 (1H), 6.80 (1H), 6.74 (1H), 4.93 (1H), 3.18 (1H), 3.09 (1H), 2.37 (1H), 2.31 (3H), 2.25 (3H), 1.61 (3H), 1.52 (3H), 1.36 (3H), 1.34 (3H), 0.84 (3H); HRMS (FAB+) 631.1609, $[C_{33}H_{41}RuONCl_2]^+$ requires 631.1609. Elemental analysis (%) C 62.27, H 6.44, N 2.00, $[C_{33}H_{41}RuONCl_2]$ requires C 61.96, H 6.46, N 2.19.

**Catalyst 25** ($R^1$ = $R^2$ = *i*Pr, $R^3$ = H, $R^4$ = Me, $R^5$ = Ph)

According to *general procedure 1*: S1 (0.18 g, 0.30 mmol), 25a·$BF_4$ (0.30 g, 0.70 mmol), and KHMDS (0.17 g, 0.86 mmol) were reacted in THF (10 mL) to provide 25 (0.14 g, 70%) as a dark green solid; *(*Angew. Chem. Int. Ed. 2015, 54, 1919 -1923) $^{1}H$ NMR δ 16.53 (1H, s), 8.26 (2H, m), 7.63 (1H, m), 7.55 (2H, m), 7.47 (2H, m), 7.44 (1H, m), 7.37 (1H, m), 6.85 (1H, m), 6.78 (1H, m), 6.69 (1H, m), 4.95 (1H, m), 3.15 (1H, d, *J* = 13 Hz), 3.09 (1H, m), 2.99 (1H, m), 2.35 (3H, s), 1.56 (3H, d, *J* = 6.1 Hz), 1.51 (3H, s), 1.41 (3H, d, *J* = 6.1 Hz), 1.40 (3H, s), 1.34 (3H, d, *J* = 6.6 Hz), 1.25 (1H, d, *J* = 6.6 Hz), 0.83 (3H, d, *J* = 6.4 Hz), 0.52 (1H, d, *J* = 6.4 Hz); $^{13}C$ NMR δ 298.0, 264.8, 153.0, 148.6, 148.4, 143.1, 142.8, 136.9, 131.0, 129.6, 129.4 (2C), 129.2 (2C), 127.5, 126.1, 125.8, 124.1, 121.8, 113.5, 77.6, 74.7, 63.3, 48.3, 29.0, 28.7, 28.3, 27.8, 27.4, 26.4, 24.6, 24.4, 22.7, 22.3; HRMS (FAB+) 668.2027, $[C_{35}H_{46}RuONCl_2]^+$ requires 668.2000.

Determination of Initiation Rates

All reactions were set up as described in the literature. (Keitz, B. K.; Endo, K.; Patel, P. R.; Herbert, M. B.; Grubbs, R. H. J. Am. Chem .Soc. 2012, 134, 693. Sanford, M. S.; Love, J. A.; Grubbs, R. H. J. Am. Chem. Soc. 2001, 123, 6543.) In brief, conditions were catalyst (0.003 mmol) and *n*-butylvinylether (0.09 mmol) in $C_6D_6$ (0.6 mL), at the given temperature.

| catalyst | temp (°C) | Initiation rate constant, $10^{-3}$ $s^{-1}$ |
|---|---|---|
| 9 | 60 | 3.2 ± 0.3 |
| 10 | 60 | 1.3 ± 0.1 |
| 11 | 60 | 0.29 ± 0.03 |
| 12 | 60 | 0.24 ± 0.02 |
| 13 | 60 | 0.0016 ± 0.0002 |
| 14 | 60 | >20 [a] |
| 14 | 30 | 1.1 ± 0.1 |
| 24 | 60 | 0.23 ± 0.02 |
| [a] complete initiation had occurred within 90s, and an accurate reading could not be determined via NMR. As such this value represents the theoretical minimum of $k_{obs}$. | | |

Ring Closing Metathesis Studies

All reactions were setup as in the standardized study, as described in the literature (except 2 mol% catalyst loading was utilized instead of 1 mol%). (Ritter, T.; Hejl, A.; Wenzel, A. G.; Funk T. W.; Grubbs, R. H. Organometallics. 2006, 25, 5740.)

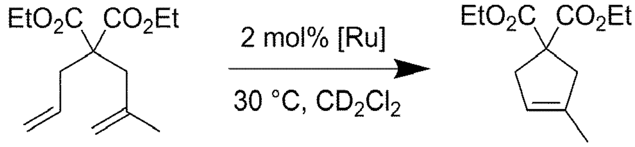


*General procedure 2:* Ethenolysis of methyl oleate using catalysts 9-25 (FIG. 1)

Reactions were setup analogously to those described in the literature. (Nickel, A.; Ung, T.; Mkrtumyan, G.; Uy, J.; Lee, C.- W.; Stoianova, D.; Papazian, J.; Wei, W.-H.; Mallari, A.; Schrodi, Y.; Pederson, R. L. Top. Catal. 2012, 55, 518. Schrodi, Y.; Ung, T.; Vargas, A.; Mkrtumyan, G.; Lee, C.- W.; Champagne, T. M.; Pederson, R. L.; Hong, S. H. Clean. 2008, 36, 669.)

As a representative procedure for reactions run at 3 ppm, a Fisher-Porter bottle equipped with a stir bar was charged with methyl oleate (15 g, 0.0506 mole) and neat dodecane (0.3 - 0.5 g) as an internal standard in a glovebox. A solution of olefin metathesis catalyst in toluene (0.076 mL, 0.002 M) was then added, the head of the Fisher-Porter bottle was connected via a T-adapter to a pressure gauge and a dip-tube fashioned from 0.17 mm hplc tubing was connectedm to a manual hplc valve. The system was sealed and taken out of the glove box to an ethylene line. The vessel was then purged three times with ethylene, pressurized to 150 psi, and placed in an oil bath at 40 °C. The reaction was monitored by withdrawing samples into 20 mL vials at sequential reaction times via the dip-tube. Immediately after collecting a sample (*ca.* 0.05 mL), it was diluted with hexanes (*ca.*20 mL), then kept in an ice bath until analysis by GC. The samples were analyzed sequentially by GC using an Agilent 6850 system equipped with an HP-1 column (30 m x 0.32 mm) using an oven gradient (60 °C, 2 min; 60 - 250 °C, 21 min) and a flame ionization detector. Response factors were determined for MO (1) as well as synthetic samples of 3, 4, 3a and 4a relative to the internal standard dodecane. A standard mixture of these compounds was used as a calibration standard to confirm consistency of response factors during each analysis run. Representative values for the response factors are shown in the table below.

GC retention times and response factors relative to internal standard (dodecane):

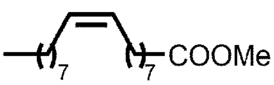
**1**

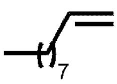
**3**

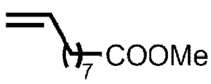
**4**

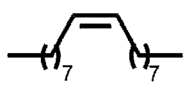
**3a**

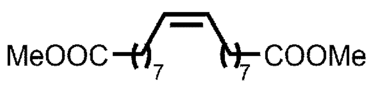
**4a**

| Compound | Dodecane | 1 | 3 | 4 | 3a | 4a |
|---|---|---|---|---|---|---|
| Time (min) | 6.8 | 17.4 | 3.5 | 8.3 | 14.2 | 20.1 |
| Response Factor | 1.000 | 1.33 | 0.83 | 0.79 | 1.40 | 1.09 |

For each timepoint obtained from an ethenolysis reaction, the GC areas of each component of the reaction mixture were converted to the percentages of each component using the response factors above. The turnover numbers for the production of terminal olefins (3 + 4) were calculated as follows:

$$\text{Conversion} = 100 - [(\text{final moles } 1) \text{ X } 100/[\text{initial moles } 1)]$$

$$\text{Selectivity} = 100 \text{ X } (\text{moles } 3 + 4)/[(\text{moles } 3 + 4) + (2 \text{ X moles } 3a + 4a)]$$

$$\text{Yield} = \text{Conversion X Selectivity}/100$$

$$TON = Yield\ X\ (initial\ moles\ 1/moles\ catalyst)/100$$

The activity, and selectivity, of catalyst **C578 (Me, *i*Pr) Ru-17 or 16** in ethenolysis reactions was assayed with methyl oleate **1**[a] (*cf.* **Scheme 1**[a]). In summary, the best conditions to date employ 150 psig ethylene and 3 ppm catalyst loading, with stirring for 1 hour at 40 °C in neat methyl oleate. This results in a 32% yield of ethenolysis products, with 92% selectivity (selectivity = ethenolysis products / (ethenolysis products + self-metathesis products)), and a TON of 106,000. This represents the best result for the ethenolysis of methyl oleate to date. Complex **16** was readily accessed in three steps from MIPP complex **C578 (11).** Monitoring the stability and kinetics of this complex as a function of time and temperature might yield mechanistic insight into the unprecedented activity and selectivity of **11**.

Example 1

**Ru-9-Ru-11 (Scheme 2)** were synthesized in moderate to excellent yields (20-90%). These catalysts all displayed unprecedented activity in the ethenolysis of methyl oleate **(Scheme 3).** Specificially, catalysts **Ru-9** and **Ru-10,** provided *TONs >100, 000* at only 3 ppm catalyst loading under optimized reaction conditions **(Table 1).** High purity ethylene (>99%) was used in this experiment; previous studies had utilized lower quality ethylene (>95%), containing significant amounts (>5 ppm) of unsaturated impurities including acetylene. These impurities may lead to premature catalyst death at lower catalyst loadings.

The backbone substitution of the CAAC ligands was varied. The *gem*-dimethyl groups of **Ru-10** may be replaced with only a slight loss in activity (as in **Ru-13).** This small change in the steric environment $\alpha$ to the carbene carbon afforded access to catalysts bearing previously unaccessible *N*-aryl substituents, such as **Ru-14** and **Ru-15** in good conversion (60-80%). Although these catalysts performed well, TONs were still not as high as those observed for **Ru-9, Ru-10,** and **Ru-13.** Too much substitution at the $\alpha$-carbon (cyclohexyl, adamantyl) appeared to impede reactivity (as in **Ru-16** and **Ru-17);** this was especially prevalent in catalysts bearing bulky *N*-ortho substituents **(Ru-12).**

Replacement of one of the *gem*-dimethyl substituents alpha to the carbene carbon with an aromatic ring, as in catalysts **Ru-18-Ru-21,** also had a profound effect. The trend for TON for various *N*-aryl substituents was similar to that found for the other catalyst systems **(Ru-7-Ru-17).** (FIG. 2a, 2b, and 2c).

Scheme 3

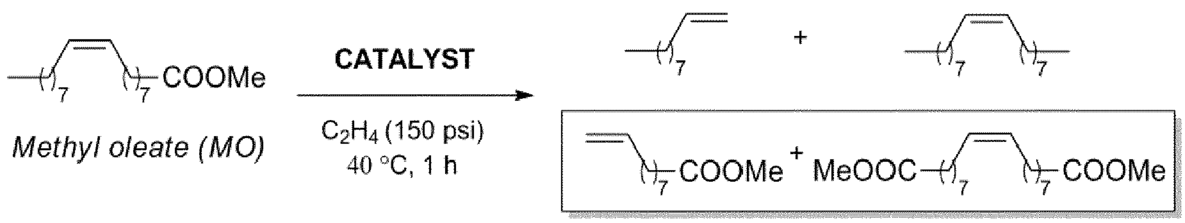


Table 1

| CATALYST | LOADING (PPM)[a] | TON[b] | Conversion (%)[c] | SELECTIVITY (%)[d] |
|---|---|---|---|---|
| Ru-4 | 3 | 125,000 | 42 | 88 |
| Ru-6 | 3 | 60,000 | 19 | 97 |
| Ru-9 | 3 | 110,000 | 37 | 86 |
| Ru-10 | 2 | 240,000 | 51 | 92 |
| | 3 | 180,000 | 59 | 92 |
| Ru-11 | 3 | 50,000 | 17 | 94 |
| Ru-12 | 3 | ND | <10 | N/A |
| Ru-13 | 3 | 130,000 | 42 | 92 |
| Ru-14 | 3 | 45,000 | 22 | 63 |
| Ru-15 | 3 | 74,000 | 26 | 86 |

(continued)

| CATALYST | LOADING (PPM)[a] | TON[b] | Conversion (%)[c] | SELECTIVITY (%)[d] |
|---|---|---|---|---|
| Ru-16 | 3 | 48,000 | 19 | 78 |
| Ru-17 | 3 | ND | <10 | N/A |
| Ru-18 | 3 | 114,000 | 41 | 83 |
| Ru-19 | 3 | 129,000 | 46 | 85 |
| Ru-20 | 3 | 142,000 | 48 | 88 |
| Ru-21 | 3 | 175,000 | 56 | 94 |

[a]loading = (moles catalyst/starting moles MO), reactions were generally conducted using 15 g MO;
[b]TON = (1/2 * moles ethenolysis products/starting moles of MO)*(1/loading);
[c]Conversion = 100 * (1/2 * moles ethenolysis products + moles self-metathesis products)/(starting moles of MO), determined via GC (Agilent technologies, HP1 column) using dodecane as an internal standard;
[d]Selectivity = 100 * (moles ethenolysis products)/(moles ethenolysis products + 2 * self-metathesis products)

Example ethenolysis of methyl oleate (1) using 3 ppm catalyst **11** ($C_2H_4$, 99.95%)

| | Decene **(3)** | dodecane | **4** | **3a** | MO (**1**) | **4a** |
|---|---|---|---|---|---|---|
| **GC Areas for:** Retention Times: | 3.5 min | 6.8 min | 8.3 min | 14.2 min | 17.4 min | 20.1 min |
| t = 0 min | 0.0 | 2324.1 | 0.0 | 0.0 | 50046.8 | 0.0 |
| t = 10 min | 6369.9 | 4680.3 | 6137.3 | 494.6 | 88271.1 | 380.4 |
| t = 20 min | 17103.7 | 4242.6 | 16471.7 | 1195.1 | 61345.2 | 900.5 |
| t = 30 min | 23494.1 | 4447.0 | 22815.3 | 1759.9 | 60842.8 | 1422.5 |
| t = 45 min | 26489.4 | 4273.1 | 25472.7 | 1941.3 | 48418.1 | 1466.7 |
| t = 60 min | 37452.0 | 5613.3 | 35995.3 | 2722.8 | 57701.6 | 2141.2 |
| t = 80 min | 36078.4 | 5135.9 | 34673.5 | 2674.8 | 50245.9 | 2117.6 |
| t = 110 min | 27171.0 | 3744.3 | 26229.7 | 2077.7 | 35108.2 | 1612.0 |
| t = 150 min | 41195.0 | 5626.2 | 40269.0 | 3192.5 | 50647.3 | 2565.6 |
| t = 210 min | 34327.6 | 4675.0 | 33616.0 | 2683.8 | 40758.2 | 2059.4 |
| t = 300 min | 34618.5 | 4711.5 | 33907.8 | 2683.4 | 40740.3 | 2053.4 |

Contributions of reaction products in ethenolysis of methyl oleate (1) using 3 ppm catalyst 11

| **GC % Yield** | **3**, % | **4**, % | **3a,** % | **1**, % | **4a,** % | total | %conv | % sel. | TON |
|---|---|---|---|---|---|---|---|---|---|
| 0 | 0.0 | 0.0 | 0.0 | 93.2 | 0.0 | 93.2 | 0.0 | | 0 |
| 10 | 9.7 | 9.6 | 0.9 | 81.6 | 0.9 | 92.2 | 10.5 | 91.8 | 32553 |
| 20 | 28.9 | 28.4 | 2.3 | 62.6 | 2.3 | 93.5 | 30.9 | 92.6 | 96405 |
| 30 | 37.8 | 37.5 | 3.3 | 59.2 | 3.4 | 100.2 | 41.0 | 91.9 | 126862 |
| 45 | 44.4 | 43.6 | 3.8 | 49.0 | 3.6 | 96.7 | 47.7 | 92.2 | 148132 |
| 60 | 47.8 | 46.9 | 4.0 | 44.5 | 4.1 | 95.9 | 51.4 | 92.2 | 159391 |
| 80 | 50.3 | 49.4 | 4.3 | 42.4 | 4.4 | 96.5 | 54.2 | 92.0 | 167813 |
| 110 | 52.0 | 51.2 | 4.6 | 40.6 | 4.6 | 96.8 | 56.2 | 91.8 | 173737 |
| 150 | 52.4 | 52.3 | 4.7 | 39.0 | 4.8 | 96.1 | 57.1 | 91.7 | 176398 |
| 210 | 52.6 | 52.6 | 4.7 | 37.7 | 4.7 | 95.0 | 57.3 | 91.8 | 177058 |
| 300 | 52.6 | 52.6 | 4.7 | 37.4 | 4.6 | 94.7 | 57.3 | 91.9 | 177193 |

Example ethenolysis of methyl oleate (1) using 3 ppm catalyst **24** ($C_2H_4$, 99.95%)

| GC Areas for: Retention Times: | Decene (3) 3.5 min | dodecane 6.8 min | 4 8.3 min | 3a 14.2 min | MO (1) 17.4 min | 4a 20.1 min |
|---|---|---|---|---|---|---|
| t = 0 min | 0.0 | 1260.8 | 0.0 | 0.0 | 30818.8 | 0.0 |
| t = 10 min | 10236.8 | 2058.6 | 9870.2 | 489.3 | 30166.0 | 380.5 |
| t = 20 min | 13959.8 | 2050.1 | 13483.1 | 721.0 | 23376.9 | 556.8 |
| t = 30 min | 18933.0 | 2607.5 | 18408.5 | 1042.2 | 28713.0 | 822.8 |
| t = 45 min | 22842.7 | 3042.5 | 22093.3 | 1255.2 | 31578.1 | 1007.4 |
| t = 60 min | 13660.9 | 1803.4 | 13259.3 | 765.8 | 18862.5 | 617.0 |
| t = 80 min | 20192.3 | 2643.2 | 19607.8 | 1136.6 | 26633.5 | 891.8 |
| t = 110 min | 21474.4 | 2786.2 | 20796.2 | 1216.9 | 28066.6 | 961.4 |
| t = 150 min | 18706.0 | 2435.6 | 18264.1 | 1064.3 | 23444.9 | 826.8 |
| t = 210 min | 20533.5 | 2650.7 | 19902.7 | 1164.4 | 26071.2 | 923.2 |
| t = 300 min | 20537.7 | 2655.2 | 19939.7 | 1176.5 | 26170.1 | 927.6 |

Contributions of reaction products in ethenolysis of methyl oleate (1) using 3 ppm catalyst 24

| **GC % Yield** | **3**, % | **4**, % | **3a,** % | **1**, % | **4a,** % | total | %conv | % sel. | TON |
|---|---|---|---|---|---|---|---|---|---|
| 0 | 0.0 | 0.0 | 0.0 | 104.0 | 0.0 | 104.0 | 0.0 | | 0 |
| 10 | 34.0 | 34.6 | 1.9 | 62.3 | 1.9 | 98.6 | 36.3 | 94.7 | 115156 |
| 20 | 46.6 | 47.5 | 2.9 | 48.5 | 2.8 | 98.4 | 49.9 | 94.3 | 157826 |
| 30 | 49.7 | 51.0 | 3.2 | 46.9 | 3.3 | 100.5 | 53.6 | 93.9 | 168861 |
| 45 | 51.4 | 52.4 | 3.4 | 44.2 | 3.4 | 99.5 | 55.3 | 93.9 | 174138 |
| 60 | 51.9 | 53.1 | 3.5 | 44.5 | 3.6 | 100.5 | 56.0 | 93.7 | 176010 |
| 80 | 52.3 | 53.5 | 3.5 | 42.9 | 3.5 | 99.3 | 56.4 | 93.8 | 177545 |
| 110 | 52.8 | 53.9 | 3.5 | 42.9 | 3.6 | 99.8 | 56.9 | 93.7 | 178881 |
| 150 | 52.6 | 54.1 | 3.6 | 41.0 | 3.5 | 97.9 | 56.9 | 93.8 | 178990 |
| 210 | 53.0 | 54.2 | 3.6 | 41.8 | 3.6 | 99.1 | 57.2 | 93.7 | 179867 |
| 300 | 53.0 | 54.2 | 3.6 | 41.9 | 3.6 | 99.1 | 57.2 | 93.7 | 179750 |

Remarkably, at 3 ppm catalyst loading, *most catalysts surpassed a TON of 100,000!* Specifically, catalysts 11 and 24 emerged as the most efficient, with TONs of 180,000. Catalyst activity correlates with *N*-aryl substitution, with larger substituents at $R^1$ and $R^2$ generally resulting in higher TONs, although this can also have a deleterious effect (as in 12 and 13, compared to 9 - 11). The ideal combination thus far appears to be when $R^1$ is small and $R^2$ is large, as in catalysts 11 and 24 ($R^1$ = Me, $R^2$ = *i*Pr). Interestingly, substitution at $R^5$ by a phenyl ring resulted in an overall improvement of activity, especially for *N*-2,6-diisopropylphenyl catalyst 25. Replacement of $R^4$ and/or $R^5$ by ethyl, propyl, or cyclohexyl did not result in a significant change in the TON (as in 15 - 19), although an adamantyl substituent (20) resulted in complete loss of activity. Interestingly, while consumption of MO appears to be the most important determining factor in the overall yield of ethenolysis products, increased *N*-aryl substitution on the catalyst appears to strongly favour selectivity for terminal olefins. These trends in selectivity and TON are most evident in the series of catalysts bearing a phenyl ring on the backbone at $R^5$ **(21 - 25)**.

Once in hand, catalysts **9 - 25** were examined in the ethenolysis of 1 using ethylene **(Table 1[a]).**

**Table 1[a].** Ethenolysis of methyl oleate (1) using catalysts 9 - 25.[a]

| catalyst | conversion (%)[b,c] | selectivity (%)[b,d,e] | yield (%)[f] | TON[g] |
|---|---|---|---|---|
| 9 | 37 | 86 | 32 | 110,000 |
| 10 | 42 | 88 | 37 | 120,000 |
| 11 | 59 | 92 | 54 | 180,000 |
| 12 | 18 | 94 | 17 | 57,000 |
| 13 | 19 | 97 | 18 | 60,000 |

(continued)

| catalyst | conversion (%)[b,c] | selectivity (%)[b,d,e] | yield (%)[f] | TON[g] |
|---|---|---|---|---|
| 14 | 22 | 63 | 14 | 47,000 |
| 15 | 26 | 86 | 22 | 73,000 |
| 16 | 42 | 92 | 39 | 130,000 |
| 17 | 19 | 78 | 14 | 47,000 |
| 18 | 13 | 97 | 13 | 43,000 |
| 19 | 16 | 97 | 15 | 50,000 |
| 20 | <5% | --- | --- | --- |
| 21 | 41 | 83 | 34 | 110,000 |
| 22 | 46 | 85 | 39 | 130,000 |
| 23 | 48 | 88 | 43 | 140,000 |
| 24 | 57 | 94 | 54 | 180,000 |

[a]Reaction conditions: catalyst (3 ppm), $C_2H_4$ (150 psi, 99.95% purity), 40 °C, 3 h.
[b]Determined via GC, using dodecane as an internal standard. [c]Conversion = 100 - [(final moles 1) X 100/[initial moles 1)] [d]Selectivity for ethenolysis products (3 and 4) over self-metathesis products (3a and 4a). [e]Selectivity = 100 X (moles 3 + 4)/[(moles 3 + 4) + (2 X moles 3a + 4a)]. [f]Yield = Conversion X Selectivity/100. [g]TON = Yield X (initial moles 1/moles catalyst)/100.

Reaction conditions were adapted from those that were previously reported in the literature, and were initially re-optimized using benchmark catalyst 10 (neat MO, 40 °C, 150 psi ethylene). (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669.)

The only deviation from the published procedures was the use of higher purity ethylene (99.95%) than previously reported (99.9%). We were pleased to find that this simple modification appeared to already result in a substantial increase in activity: 10 ppm loading of catalyst 10 resulted in a TON of 67,000, whereas the benchmark value for 10 published in the literature is a TON 35,000. (Schrodi, Y.; Ung, T.; Vargas, A.; Mkrtumyan, G.; Lee, C.- W.; Champagne, T. M.; Pederson, R. L.; Hong, S. H. Clean. 2008, 36, 669.) Intuitively, this marked effect might be expected, as at such low catayst loadings any trace impurities that would result in catalyst decomposition, such as acetylene gas, would exhibit a greater effect.

Methyl oleate (Nu-Check-Prep, >99%, see Experimental) was purified through filtration over alumina (see ESI). As disclosed previously this purification step is essential for high TON (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518; Schrodi, Y.; Ung, T.; Vargas, A.; Mkrtumyan, G.; Lee, C.- W.; Champagne, T. M.; Pederson, R. L.; Hong, S. H. Clean. 2008, 36, 669.) For example, when methyl oleate purchased from Nu-Check-Prep (>99%) or Sigma Aldrich (>99.0%, Fluka analytical standard) was used without further purification, TONs were only 8,500 and 230 respectively for catalyst 11 (3 ppm). Interestingly, a similar increase was not noted for catalyst 5. For example, under the optimized reaction conditions 5 (100 ppm) generated a TON of only 4600 (*cf.* literature value of 5400, (Schrodi, Y.; Ung, T.; Vargas, A.; Mkrtumyan, G.; Lee, C.- W.; Champagne, T. M.; Pederson, R. L.; Hong, S. H. Clean. 2008, 36, 669).

Cross metathesis of Soy FAME or Soybean Oil with 1-butene (butenolysis) using Grubbs's catalyst bearing a cyclic (alkyl) (amino) carbene (CAAC).

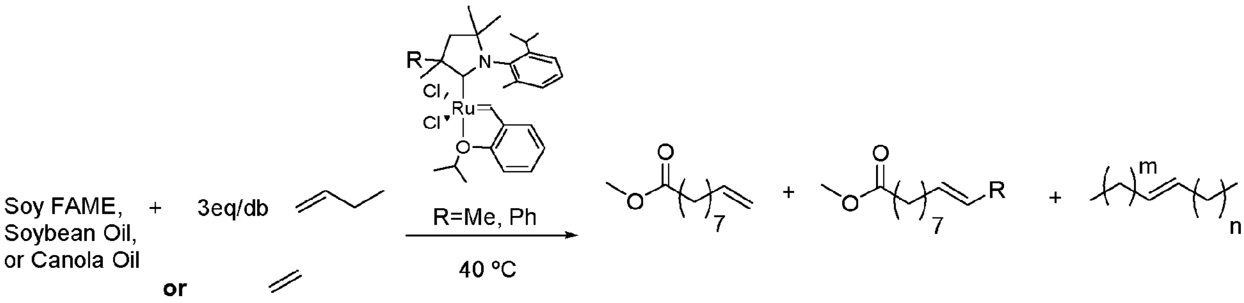


R=Me (C578), R=Ph (C640)

Ethenolysis of Soy FAME

Reactions were setup analogously to those described in the literature. (Nickel, A.; Ung, T.; Mkrtumyan, G.; Uy, J.; Lee, C.- W.; Stoianova, D.; Papazian, J.; Wei, W.- H.; Mallari, A.; Schrodi, Y.; Pederson, R. L. Top. Catal. 2012, 55, 518. Schrodi, Y.; Ung, T.; Vargas, A.; Mkrtumyan, G.; Lee, C.- W.; Champagne, T. M.; Pederson, R. L.; Hong, S. H. Clean. 2008, 36, 669.) As a representative procedure for reactions run at 10 ppm, a Fisher-Porter bottle equipped with a stir bar was charged with Soy FAME (15g, 0.050 mole) and neat dodecane (0.30 g) as an internal standard in a glovebox. A solution of olefin metathesis catalyst in toluene (0.36 mL, 0.0014 M) was then added. The head of the Fisher-Porter bottle was connected via a T-adapter to a pressure gauge and a dip-tube fashioned from 0.17 mm hplc tubing was connected to a manual hplc valve. The system was sealed and taken out of the glove box to an ethylene line. The vessel was then purged three times with ethylene, pressurized to 150 psi, and placed in an oil bath at 40 °C. The reaction was monitored by withdrawing samples into 20 mL vials at sequential reaction times via the dip-tube. Immediately after collecting a sample (*ca.* 0.05 mL), it was diluted with hexanes (*ca.*20 mL), then kept in an ice bath until analysis by GC. The samples were analyzed sequentially by GC using an Agilent 6850 system equipped with an HP-1 column (30 m x 0.32 mm) using an oven gradient (60 °C, 2 min; 60 - 250 °C, 21 min) and a flame ionization detector.

Representative Example of Ethenolysis of Soy Fame with ethylene (99.995% purity) in the presence of a ruthenium complex bearing a cyclic alkyl amino carbene ligand of the invention.

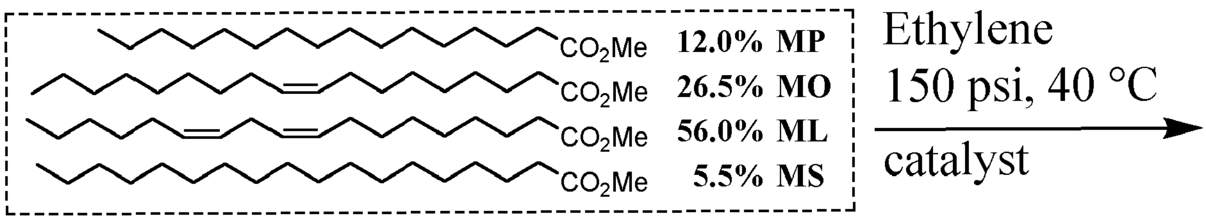


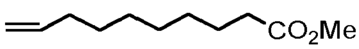

**Methyl 9-Decenoate ($C_{11}$) (product)**

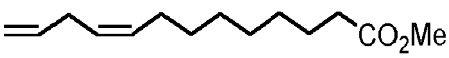

**Methyl 9,12-Tridecadienoate ($C_{14}$) (product)**

Results of soy fame ethenolysis using ethylene (99.995% purity)

| Catalyst** | Catalyst Loading (ppm) | TON* |
|---|---|---|
| 711 (comparative) | 10 | 22,000 |
| 11 | 10 | 60,000 |
| 11 | 3 | 92,000 |
| 12 | 3 | 60,000 |

(continued)

| Catalyst** | Catalyst Loading (ppm) | TON* |
|---|---|---|
| 24 | 3 | 55,000 |
| *TON = Turn Over Number<br>**Catalyst | | |

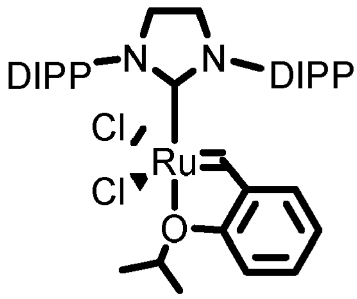

711

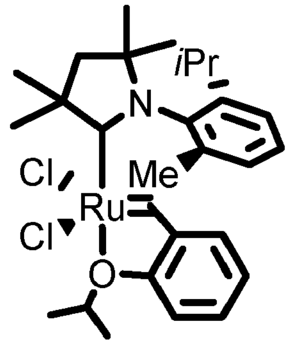

11

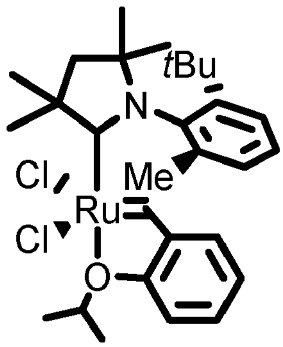

12

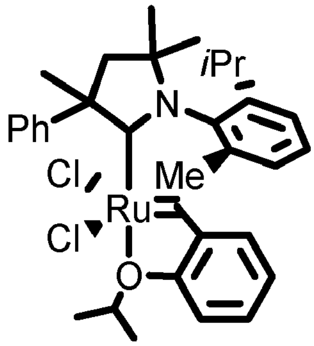

24

*Soy FAME Butenolysis:*

Inside an argon filled glovebox, a fisher porter vessel equipped with a stir bar and pressure head setup was loaded with Soy FAME (10.0 g; 34.2 mmol) and catalysts solution (148 μL, 1.28 μmol, 0.00865 M). The reaction vessel was sealed, removed from the glovebox and attached to a 1-butene source. Then, 1-butene (8.65 g; 154 mmol) was condensed into the reaction mixture. The reaction was allowed to proceed at 40 °C. An aliquot sample of the reaction mixture was collected via the equipped dip tube into a vial containing tris(hydroxymethyl)phosphine (THMP) solution (0.2 mL, 1M), heated to 60 °C for 1 hour, and then cooled to room temperature. Water was added to the sample reaction mixture and the sample was extracted with hexane. The organic layer was analyzed by GC/GCMS.

**Table 2:** GC percentage of Soy FAME butenolysis products over time.

| **Catalysts** | **C578** | **C578** | **C578** | **C578** | **C640** | **C640** | **C640** | **C640** |
|---|---|---|---|---|---|---|---|---|
| **Time (hr)** | **0.5** | **1** | **2** | **3** | **0.5** | **1** | **2** | **3** |
| **C9** | 8.3 | 7.9 | 7.2 | 7.8 | 7.8 | 9.2 | 8.0 | 7.4 |
| **C10** | 8.0 | 7.7 | 7.3 | 7.5 | 7.7 | 8.1 | 7.3 | 7.2 |
| **C12** | 4.7 | 4.7 | 4.7 | 4.7 | 4.9 | 4.8 | 4.9 | 4.9 |
| **C13** | 1.6 | 1.6 | 1.6 | 1.6 | 1.0 | 1.0 | 1.2 | 1.0 |

(continued)

| Catalysts | C578 | C578 | C578 | C578 | C640 | C640 | C640 | C640 |
|---|---|---|---|---|---|---|---|---|
| C10E | 20.2 | 20.7 | 21.0 | 20.8 | 20.4 | 20.1 | 20.6 | 20.9 |
| C15 | 1.3 | 0.9 | 0.9 | 1.4 | 0.9 | 0.9 | 0.9 | 1.0 |
| C12E | 15.4 | 15.8 | 16.1 | 16.0 | 14.8 | 14.5 | 15.0 | 15.4 |
| C13E | 4.2 | 4.0 | 3.9 | 3.9 | 4.6 | 3.5 | 3.4 | 3.5 |
| C18 | 0.2 | 0.2 | 0.2 | 0.2 | 0.2 | 0.2 | 0.2 | 0.2 |
| C15E | 5.7 | 5.7 | 5.7 | 5.7 | 5.2 | 5.1 | 5.2 | 5.3 |
| C16E | 11.1 | 11.3 | 11.2 | 11.2 | 11.1 | 10.9 | 11.1 | 11.3 |
| Oleates | 8.3 | 8.0 | 7.6 | 7.6 | 11.5 | 11.1 | 11.1 | 11.1 |
| C18E | 5.4 | 5.5 | 5.5 | 5.5 | 5.5 | 5.3 | 5.4 | 5.5 |
| C20E | 0.4 | 0.4 | 0.4 | 0.4 | 0.4 | 0.4 | 0.4 | 0.4 |
| 1,18DE | 2.1 | 2.3 | 2.3 | 2.3 | 1.4 | 1.5 | 1.5 | 1.6 |
| **Conversion(%)** | **91.7** | **92.0** | **92.3** | **92.4** | **88.5** | **88.9** | **88.9** | **88.9** |
| Conversion = 100 - oleates; Oleates are unsaturated esters of methyl oleate, methyl linoleate, and methyl linolenate; C16E and C18E are saturated esters that are non-metathesis active. Products distribution of Soy FAME butenolysis equilibrium, wherein Cn is an alkene with n number of carbons, E is a methyl ester, C16E is palmitate, C18E is stearate and 1,18DiE is C18-diester. | | | | | | | | |

*Soybean Oil Butenolysis:*

Inside an argon filled glovebox, a fisher porter vessel equipped with a stir bar and pressure head setup was loaded with Soybean Oil (10.0 g; 11.5 mmol) and catalysts solution (149 μL, 1.29 μmol, 0.00865 M). The reaction vessel was sealed, removed from the glovebox and attached to a 1-butene source. Then, 1-butene (8.69 g; 155 mmol) was condensed into the reaction mixture. The reaction was allowed to proceed at 40 °C. An aliquot sample of the reaction mixture was collected via the equipped dip tube into a vial containing tris(hydroxymethyl)phosphine (THMP) solution (0.2 mL, 1M) and sodium methoxide solution (1.0 mL, 1 wt % solution in methanol). The reaction mixture was heated to 60 °C for 30 minutes, and then cooled to room temperature. Water was added to the sample reaction mixture and the sample was extracted with hexane. The organic layer was analyzed by GC/GCMS.

**Table 3:** GC percentage of Soybean oil butenolysis products over time.

| Catalysts | C578 | C578 | C578 | C578 | C640 | C640 | C640 | C640 |
|---|---|---|---|---|---|---|---|---|
| **Time (hr)** | **0.5** | **1** | **2** | **3** | **0.5** | **1** | **2** | **3** |
| C9 | 9.3 | 8.9 | 9.1 | 9.4 | 9.2 | 9.1 | 9.4 | 8.2 |
| C10 | 8.4 | 8.2 | 7.7 | 7.6 | 8.2 | 8.2 | 8.1 | 7.5 |
| C12 | 6.4 | 6.8 | 6.8 | 6.9 | 6.4 | 6.8 | 6.5 | 6.5 |
| C13 | 0.6 | 0.7 | 0.7 | 0.7 | 0.5 | 0.5 | 0.4 | 0.4 |
| C10E | 20.1 | 19.5 | 21.1 | 20.7 | 18.9 | 17.9 | 18.7 | 18.6 |
| C15 | 1.1 | 1.2 | 1.3 | 1.1 | 0.5 | 0.8 | 0.9 | 0.9 |
| C12E | 13.9 | 14.9 | 16.3 | 16.8 | 12.6 | 12.9 | 12.7 | 13.4 |
| C13E | 4.3 | 3.7 | 3.2 | 2.9 | 4.6 | 4.3 | 4.4 | 4.4 |
| C18 | 0 | 0 | 0.2 | 0.3 | 0 | 0 | 0.2 | 0.2 |
| C15E | 5.3 | 5.3 | 5.0 | 5.0 | 5.3 | 5.3 | 5.2 | 5.4 |
| C16E | 11.5 | 12.4 | 11.7 | 11.8 | 11.4 | 12.4 | 11.5 | 11.9 |

(continued)

| Catalysts | C578 | C578 | C578 | C578 | C640 | C640 | C640 | C640 |
|---|---|---|---|---|---|---|---|---|
| **Time (hr)** | **0.5** | **1** | **2** | **3** | **0.5** | **1** | **2** | **3** |
| **Oleates** | 11.0 | 9.7 | 7.0 | 6.3 | 14.5 | 15.0 | 13.6 | 13.8 |
| **C18E** | 4.7 | 5.1 | 4.7 | 4.8 | 4.7 | 5.1 | 4.7 | 4.9 |
| **C20E** | 0.4 | 0.4 | 0.4 | 0.4 | 0.4 | 0.4 | 0.4 | 0.4 |
| **1,18DE** | 1.4 | 1.7 | 1.8 | 1.9 | 1.0 | 0.5 | 1.1 | 1.2 |
| **Conversion(%)** | **89.0** | **90.3** | **93.0** | **93.7** | **85.5** | **85.0** | **86.4** | 86.2 |
| Conversion = 100 - oleates; Oleates are unsaturated esters of methyl oleate, methyl linoleate, and methyl linolenate; C16E and C18E are saturated esters that are non-metathesis active. Products distribution of Soybean Oil butenolysis equilibrium, wherein Cn is an alkene with n number of carbons, E is a methyl ester, C16E is palmitate, C18E is stearate and 1,18DiE is C18-diester. | | | | | | | | |

*Canola Oil Ethenolysis:*

Inside an argon filled glovebox, a fisher porter vessel equipped with a stir bar and pressure head setup was loaded with Canola Oil (10.0 g; 11.4 mmol) and catalysts solution (148 μL, 1.28 μmol, 0.00865 M). The reaction vessel was sealed, removed from the glovebox and attached to an ethylene source. Then, the reaction mixture was sparged with ethylene 3 times and the reaction vessel was pressurized with ethylene to 150 psig. The reaction was allowed to proceed at 40 °C. An aliquot sample of the reaction mixture was collected via the equipped dip tube into a vial containing tris(hydroxymethyl)phosphine (THMP) solution (0.2 mL, 1M) and sodium methoxide solution (1.0 mL, 1 wt % solution in methanol). The reaction mixture was heated to 60 °C for 30 minutes, and then cooled to room temperature. Water was added to the sample reaction mixture and the sample was extracted with hexane. The organic layer was analyzed by GC/GCMS.

**Table 4:** GC percentage of Canola Oil ethenolysis products over time.

| Catalysts | C578 | C578 | C578 | C578 | C640 | C640 | C640 | C640 |
|---|---|---|---|---|---|---|---|---|
| **Time (hr)** | **0.5** | **1** | **2** | **3** | **0.5** | **1** | **2** | **3** |
| **C9** | 0.4 | 0.4 | 0.5 | 0.0 | 0.8 | 0.8 | 0.6 | 0.6 |
| **C10** | 3.1 | 6.5 | 10.4 | 12.4 | 2.6 | 2.8 | 6.5 | 7.5 |
| **C12** | 1.8 | 2.2 | 2.3 | 2.5 | 3.9 | 4.2 | 3.4 | 3.1 |
| **C13** | 0.6 | 1.6 | 2.4 | 2.8 | 0.9 | 1.1 | 2.0 | 2.2 |
| **C10E** | 5.3 | 2.4 | 23.3 | 25.9 | 4.8 | 5.6 | 14.7 | 16.4 |
| **C15** | 3.4 | 3.0 | 2.6 | 2.7 | 5.4 | 5.2 | 3.8 | 3.45 |
| **C12E** | 0.8 | 1.1 | 1.1 | 1.2 | 1.1 | 1.3 | 1.6 | 1.5 |
| **C13E** | 3.7 | 6.4 | 8.0 | 8.1 | 2.4 | 2.6 | 5.3 | 5.8 |
| **C15E** | 6.1 | 6.5 | 6.7 | 6.6 | 11.5 | 12.1 | 11.2 | 11.9 |
| **C16E** | 11.6 | 11.8 | 12.3 | 12.0 | 12.8 | 26.2 | 11.9 | 12.3 |
| **Oleates** | 44.5 | 24.3 | 14.1 | 11.1 | 26.2 | 22.0 | 14.1 | 13.3 |
| **C18E** | 4.6 | 4.7 | 4.9 | 4.7 | 5.2 | 5.0 | 4.7 | 4.9 |
| **C20E** | 0.4 | 0.4 | 0.4 | 0.4 | 0.5 | 0.4 | 0.4 | 0.4 |
| **1,18DE** | 3.2 | 4.4 | 4.8 | 3.9 | 8.3 | 8.9 | 8.0 | 3.5 |
| **1,21DE** | 4.5 | 3.0 | 2.2 | 1.4 | 5.9 | 5.7 | 7.7 | 3.4 |

(continued)

| Catalysts | C578 | C578 | C578 | C578 | C640 | C640 | C640 | C640 |
|---|---|---|---|---|---|---|---|---|
| Time (hr) | 0.5 | 1 | 2 | 3 | 0.5 | 1 | 2 | 3 |
| Conversion(%) | 55.5 | 75.7 | 85.9 | 88.9 | 73.8 | 78.0 | 85.9 | 86.7 |
| Conversion = 100 - oleates; Oleates are unsaturated esters of methyl oleate, methyl linoleate, and methyl linolenate; C16E and C18E are saturated esters that are non-metathesis active. Products distribution of Canola Oil ethenolysis equilibrium, wherein Cn is an alkene with n number of carbons, E is a methyl ester, C16E is palmitate, C18E is stearate and 1,18DiE is C18-diester. | | | | | | | | |

The TON of catalyst 10 further increased to 120,000 upon reduction of the catalyst loading to 3 ppm. Thus, all subsequent reactions were run at 3 ppm catalyst loading, which was also expected to provide greater differentiation in activity between promising catalysts than at 10 ppm.

A plausible explanation for the high activity exhibited by CAAC catalysts in ethenolysis transformations might be a result of increased stabilization of the ruthenium methylidene intermediate generated in the presence of ethylene gas. Ruthenium methylidenes are known to decompose rapidly via insertion of the *N*-aryl substituent into the methylidene carbene, which subsequently generates various ruthenium hydrides that are inactive in metathesis transformations. (Hong, S. H.; Wenzel, A. G.; Salguero, T. T.; Day, M. W.; Grubbs. R. H. J. Am. Chem. Soc. 2007, 129, 7961; Sanford, M. S.; Love, J. A.; Grubbs, R. H. J. Am. Chem. Soc. 2001, 123, 6543; Lehman, S. B.; Wagener, K, B.; Organometallics 2005, 24, 1477; Ulman, M.; Grubbs, R. H. J. Org. Chem. 1999, 64, 7202.)

CAAC ligands are known to be more electron donating than their *N*-heterocyclic carbene (NHC) counterparts (Occhipinti, G.; Jensen, V. R. Organometallics 2011, 30, 3522.).

Thus, when used in place of NHCs, it is expected that the increased electron density at ruthenium might somewhat stabilize the otherwise highly reactive and electron deficient methylidene intermediate. (Lavallo, V.; Canac, Y.; Prasang, C.; Donnadieu, B.; Bertrand, G. Angew. Chem. 2005, 117, 5851; Angew. Chem. Int. Ed. 2005, 44, 5705; Melaimi, M.; Soleilhavoup, M.; Bertrand, G. Angew. Chem. 2010, 122, 8992; Angew. Chem. Int. Ed. 2010, *49*, 8810 and experimental).

Substitution of the ortho *N*-aryl substituents with a larger sterically encumbered group would also be expected to significantly decrease the rate of termination by insertion into the $[Ru]=CH_2$ bond. However, increased substitution can also hinder coordination of olefins to the ruthenium metal center. Diminished reactivity with increasing *N*-aryl substitution was indeed noted when initiation rates of selected catalysts (9 - 14) were measured following exposure to *n*-butylvinylether. (See experimental for further details.) When initiation rates are compared to TONs, it is clear that both the slowest initiating catalysts (12, 13) and the fastest initiating catalyst (14) exhibit the lowest TONs (FIG. 4).

This is likely a result of diminished catalytic rate for the former group and an increased susceptibility to decomposition for the latter. This study illustrates the importance of this delicate balance, as reflected in the superior TONs exhibited by catalysts 11 and 24, possessing asymmetric *N*-aryl substituents. In these systems, the smaller substituent ($R^1$ = Me) faciltates rapid coordination of the incoming olefin substrate, whereas the larger substituent ($R^2$ = *i*Pr) prevents decomposition of the methylidene intermediate. This asymmetry might be expected to exhibit a greater effect in CAAC ligated catalysts, as the steric interaction of the ortho substituents on the *N*-aryl ring with the two adjacent geminal methyl substituents would be expected to influence the conformation of the *N*-aryl ring. If the larger substituent resides closer to the ruthenium metal center, the methyl group which is inherently more susceptible to CH insertion would be directed away from the reactive ruthenium methylidene.

It has been postulated that high activity in ethenolysis might be correlated to the tendency of a catalyst to undergo degenerative metathesis events, through the preferential formation of 2,4-metallacycles rather then 2,3-metallacycles, which would result in increased selectivity for terminal olefins in the product distribution. (Stewart, I. C.; Keitz, B. K.; Kuhn, K. M.; Thomas, R. M.; Grubbs, R. H. J. Am. Chem. Soc. 2010, 132, 8534.) This is a powerful design principle in the context of achieving high kinetic selectivity for terminal olefins, when employing higher olefins such as propene and 1-butene gas as ethylene surrogates. However, when ethylene gas is employed, it is more likely that the lower selectivities for terminal olefins exhibited by previous generations of catalysts (5 - 8) is primarily a result of rapid catalyst death in the presence of ethylene. This would translate to products reflecting the kinetic distribution of rapid unselective cross metathesis reactions of 5 - 8 with both ethylene and terminal olefins. (For similar correlations regarding catalyst lifetime and approach to equilibrium for *E*/*Z* diastereoselectivity see: Lee, C.-W.; Grubbs, R. H. Org. Lett. 2000, 2, 2145.) Although CAAC-ligated ruthenium-based catalysts have been demonstrated to engage in metathesis reactions more slowly than phosphine or NHC-ligated catalysts, (Anderson, D. R.; Ung, T.; Mkrtumyan. G.; Bertrand, G.; Grubbs, R. H.; Schrodi, Y. Organometallics. 2008, 27, 563. See ESI for further details.) they also appear to persist for a much longer time in the presence of ethylene. (Keitz, B. K.; Grubbs, R. H. J. Am. Chem. Soc. 2011, 133, 16277.)

This would allow for the ethenolysis reaction to proceed closer to completion in order to achieve the equilibrium ratio of terminal olefin products, and provides a feasible explanation for the notable increase in activity exhibited by this family of catalysts.

Finally, given the notable dependance on the TON with respect to the purity of the ethylene employed, as seen earlier for catalyst 10, we briefly explored the effect of utilizing an even higher purity ethylene source (99.995% vs. 99.95%) at different loadings of catalyst 11 **(Table 3)**. A dramatic increase in TON was noted at 1 ppm catalyst loading. To the best of our knowledge, *this represents the highest value available in the literature for any ethenolysis catalyst to date (TON 340,000).*

**Table 3.** Effect of $C_2H_4$ **(2)** source on activity of catalyst 11[(a)]

| Loading | purity of 2[(b)] | yield (%)[(a)] | TON[(a)] |
|---|---|---|---|
| 3 | 99.95% | 54 | 180,000 |
| 3 | 99.995% | 53 | 180,000 |
| 2 | 99.95% | 48 | 240,000 |
| 2 | 99.995% | 49 | 245,000 |
| 1 | 99.95% | 13 | 130,000 |
| 1 | 99.995% | 34 | 340,000 |
| [(a)]Reaction conditions, and calculations, are as listed in **Table 1a.** [(b)]See experimental for further details regarding ethylene purity, grade, and source. | | | |

While not wishing to be bound by any particular theory, the exceptional activity displayed by the most active catalysts is hypothesized to be a result of a balance between catalyst initiation and decomposition, both of which increase with decreasing substitution about the CAAC carbene carbon. Asymmetrical *ortho*-substitution about the *N*-aryl ring of the CAAC ligand appears to be beneficial in achieving this desired increase in propensity towards substrate turnover *in lieu* of catalyst decomposition.

Example 2

Initiation rates were measured by reacting catalysts with n-butylvinylether. As a comparison, the initiation rate constants of known catalysts are also included (i.e., Ru4 and SD-Hoveyda (i.e., the second-generation Grubbs-Hoveyda catalyst)). As depicted in **Table 2a**, the less bulky catalysts initiating much faster. The mono-substituted isopropyl derivative Ru8 initiates especially rapidly, and in fact could not be monitored at 60 °C for direct comparison to the others because the reaction was complete by the time the first data point was obtained at 90 s. It is interesting to note that the MIPP catalyst Ru10 barely reacts until about 50 °C.

Table 2a

| CATALYST | Catalyst #, Scheme 5 | TEMP (°C) | INITIATION RATE CONSTANT, $10^{-3}$ $s^{-1}$ |
|---|---|---|---|
| Ru-21 | 1 | 60 | 0.23 ± 0.02 |
| Ru-11 | 2 | 60 | 0.24 ± 0.02 |
| Ru-10 | 3 | 60 | 0.29 ± 0.03 |
| Ru-9 | 5 | 60 | 3.2 ± 0.3 |
| Ru-8 | 6 | 60 | [b] |
| Ru-8 | 6 | 30 | 1.1 ± 0.1 |
| Ru-4 | 4 | 60 | 1.3 ± 0.1 |
| SD-Hov | | 30 | 7.2 ± 0.2 |
| [a] Initiation rate constants were determined by measuring the decrease in the benzylidene resonance using $^1H$ NMR spectroscopy following addition of BVE (measurements conducted in triplicate). Conditions were catalyst (0.003 mmol) and BVE (0.09 mmol) in $C_6D_6$ (0.6 mL) at given temperature.<br>[b] Complete initiation had occurred within 90s. | | | |

Example 3

The steady-state cross-metathesis (CM) of 1-hexene (sealed in an NMR tube)

(Scheme 4)

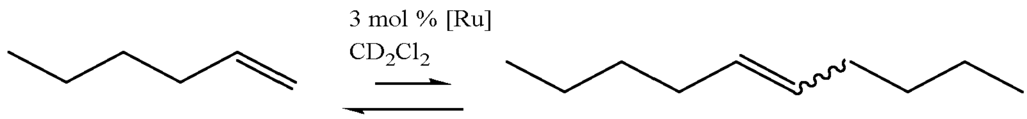


**Scheme 4** was examined to see if a correlation could be made to the selectivity noted in the ethenolysis of methyl oleate. In general, the CAACs seem to exhibit similar selectivity to previously reported N-alkyl N-aryl catalysts (which range from 10-20% conversion). It is interesting that except for the disubstituted N-aryl derivatives, N-aryl substitution does not appear to influence the preference of the catalysts for terminal olefins, however phenyl substitution on the backbone as in 1 (i.e., Ru21) decreases the selectivity somewhat. Also, the monounsubstituted derivative 6 (i.e., Ru8) did not reach a steady state after 6 hours. (FIG. 2).

In summary, a new series of ruthenium metathesis catalysts, bearing CAAC ligands, is presented that displays exceptional activity in ethenolysis reactions. In the cross metathesis reaction of the seed oil derivative methyl oleate (1) and ethylene gas (2), TONs >100,000 are generated in many cases, which surpasses the minimum value of 50,000 required to be considered economically sustainable on an industrial scale. Furthermore, even higher TONs (180,000 - 340,000) were obtained in some cases. These are the highest values recorded in the literature to date for an ethenolysis reaction, and the only reported TONs >50,000 using ethylene gas specifically. As a result, it is envisioned that this work will find substantial application in the continued development of new methodologies and processes directed towards the economically and environmentally sustainable production of LAOs, as well as other valuable terminal olefins, especially through the transformation of seed oils and their derivatives.

All publications and patents cited herein are hereby incorporated by reference in their entirety.

Those skilled in the art will recognize, or be able to ascertain using no more than routine experimentation, many equivalents to the specific embodiments of the invention described herein. Such equivalents are intended to be encompassed by the following claims.

## Claims

**1.** A ruthenium complex bearing a cyclic alkyl amino carbene ligand, which is represented by Formula III:

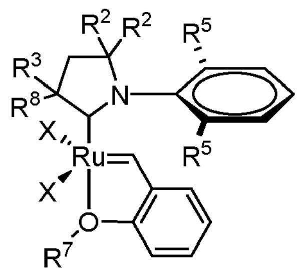

Formula III

wherein:

X is, independently for each occurrence, a monovalent ligand,
$R^2$ is alkyl;
$R^3$ is alkyl;
$R^5$ is, independently for each occurrence, methyl or ethyl;
$R^7$ is alkyl; and
$R^8$ is aryl or heteroaryl.

**2.** The ruthenium complex according to claim 1, wherein:

X is alkoxy or halo, preferably halo.

**3.** The ruthenium complex according to claim 1 or 2, wherein: $R^2$ is methyl.

**4.** The ruthenium complex according to any of claims 1 to 3, wherein: $R^3$ is methyl.

**5.** The ruthenium complex according to any of claims 1 to 4, wherein: $R^5$ is ethyl.

**6.** The ruthenium complex according to any of claims 1 to 5, wherein: $R^7$ is 2-propyl.

**7.** The ruthenium complex according to any of claims 1 to 6, wherein: $R^8$ is substituted or unsubstituted phenyl, preferably unsubstituted phenyl.

**8.** The ruthenium complex according to claim 1 having the following formula:

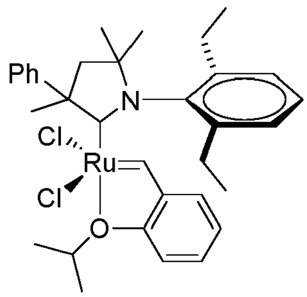

.

**9.** A method of producing at least one olefin product comprising:

providing at least one olefinic substrate, wherein the at least one olefinic substrate is a fatty acid methyl ester selected from sunflower FAME, safflower FAME, soy FAME, algal FAME, palm FAME and canola FAME;
providing at least one alpha olefin; and
contacting the at least one olefinic substrate with the at least one alpha olefin in the presence of a ruthenium complex bearing a cyclic alkyl amino carbene ligand under reaction conditions effective to allow a metathesis reaction to occur, thereby producing the at least one olefin product,
wherein the ruthenium complex bearing a cyclic alkyl amino carbene ligand is the ruthenium complex according to any one of claims 1 to 8.

**10.** The method according to claim 9, wherein the at least one alpha olefin is a monosubstituted alpha olefin.

**11.** The method according to claim 9, wherein the at least one alpha olefin is of the formula RR'C=$CH_2$, wherein R and R' are each independently H, alkyl, aryl, heteroalkyl, heteroaryl, alkoxy, alkenyl, alkynyl, or acyl.

**12.** The method according to claim 9, wherein the at least one alpha olefin is of the formula RR'C=$CH_2$, wherein R and R' are each independently H.

**13.** The method according to any one of claims 9 to 12, wherein the at least one olefinic substrate is soy FAME

**14.** The method according to any one of claims 9 to 13, wherein the at least one alpha olefin has a purity of greater than 99%.

**15.** The method of claim 5, wherein the at least one alpha olefin has a purity selected from 99.95% and 99.995%.

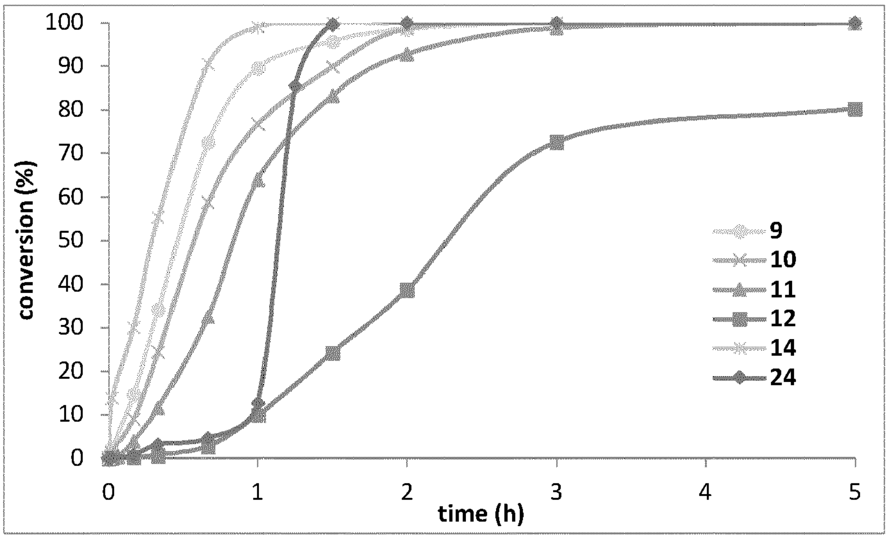
conversion (%)
100
90
80
70
60
50
40
30
20
10
0
0
1
2
3
4
5
time (h)
9
10
11
12
14
24

FIG. 1

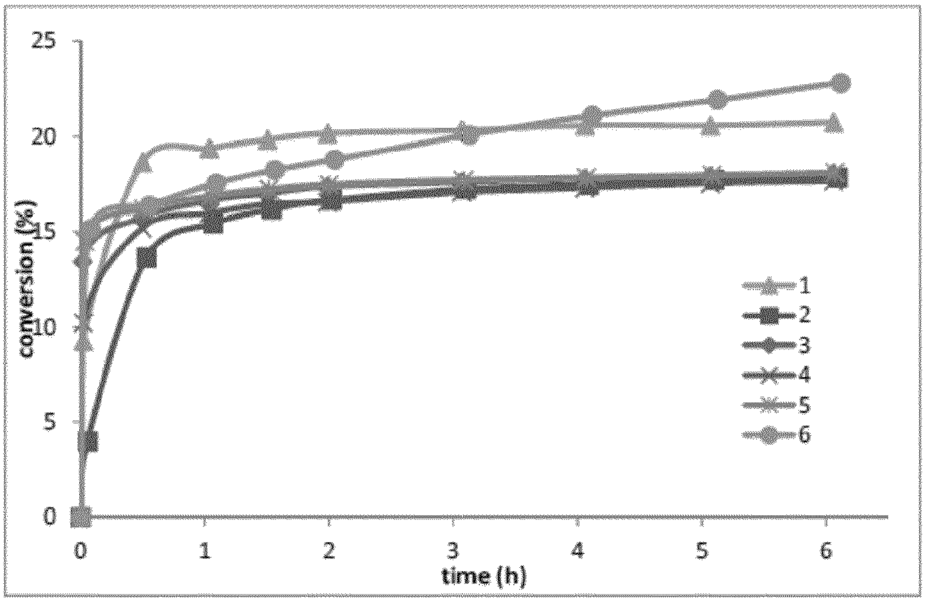


FIG. 2

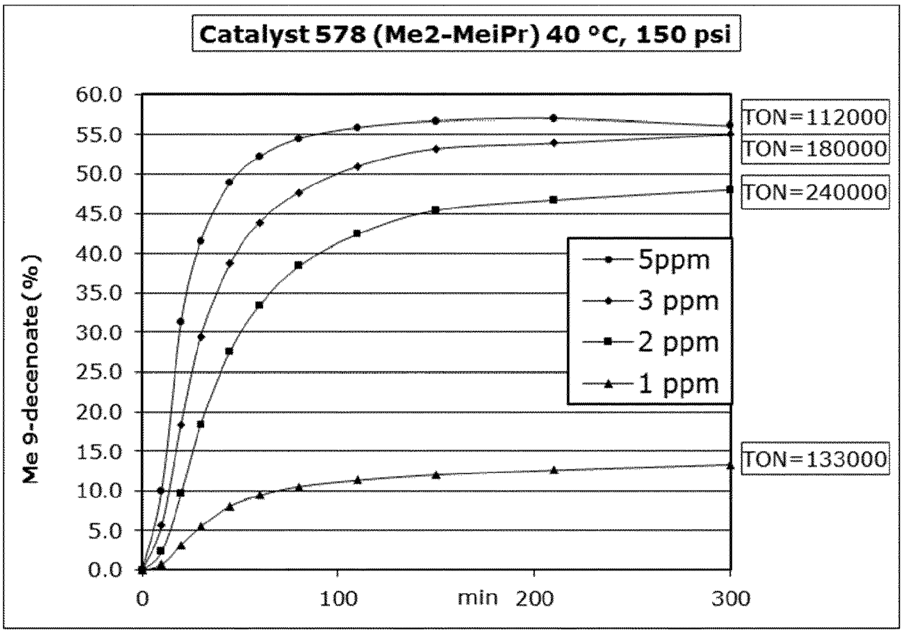


| | 1ppm | 2ppm | 3ppm | 5ppm |
|---|---|---|---|---|
| 0 | 0.0 | 0.0 | 0.0 | 0.0 |
| 10 | 0.7 | 2.3 | 5.6 | 10.0 |
| 20 | 3.2 | 9.6 | 18.4 | 31.3 |
| 30 | 5.5 | 18.4 | 29.5 | 41.5 |
| 45 | 8.1 | 27.6 | 38.7 | 48.9 |
| 60 | 9.4 | 33.4 | 43.9 | 52.2 |
| 80 | 10.5 | 38.4 | 47.7 | 54.5 |
| 110 | 11.3 | 42.4 | 51.0 | 55.9 |
| 150 | 12.0 | 45.4 | 53.2 | 56.7 |
| 210 | 12.6 | 46.7 | 53.9 | 57.0 |
| 300 | 13.3 | 48.0 | 55.0 | 56.1 |

FIG. 3a

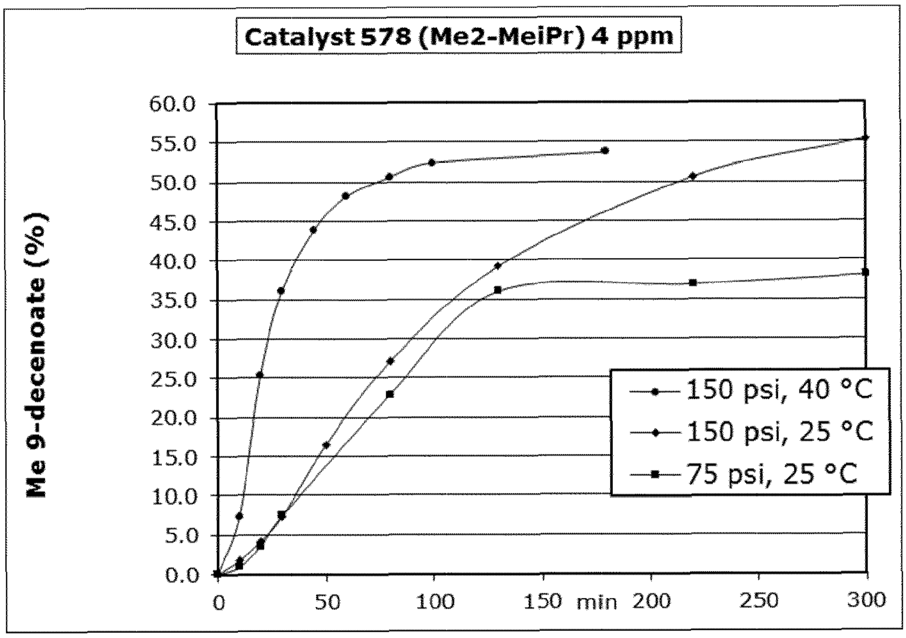


| psi | 150 | psi | 150 | psi | 75 |
|---|---|---|---|---|---|
| °C | 40.0 | °C | 25.0 | °C | 25.0 |
| 0 | 0.0 | 0 | 0.0 | 0 | 0.0 |
| 10 | 7.4 | 10 | 1.7 | 10 | 0.9 |
| 20 | 25.4 | 20 | 4.2 | 20 | 3.6 |
| 30 | 36.1 | 30 | 7.4 | 30 | 7.5 |
| 45 | 43.9 | 50 | 16.5 | 80 | 22.9 |
| 60 | 48.2 | 80 | 27.1 | 130 | 36.0 |
| 80 | 50.6 | 130 | 39.2 | 220 | 36.9 |
| 100 | 52.4 | 220 | 50.6 | 300 | 38.1 |
| 180 | 53.7 | 300 | 55.4 | | |

FIG. 3b

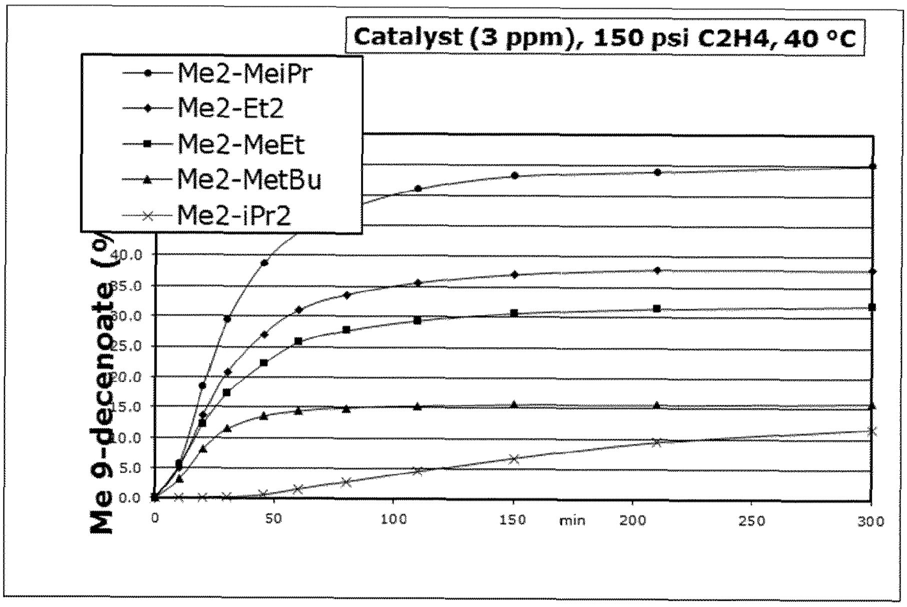


| | Me2 MeiPr | Me2 Et2 | Me2 MeEt | Me2 MetB | Me2 iPr2 |
|---|---|---|---|---|---|
| 0 | 0.0 | 0.0 | 0.0 | 0.0 | 0.0 |
| 10 | 5.6 | 5.1 | 5.4 | 3.1 | 0.0 |
| 20 | 18.4 | 13.6 | 12.2 | 8.1 | 0.1 |
| 30 | 29.5 | 20.9 | 17.4 | 11.5 | 0.3 |
| 45 | 38.7 | 27.1 | 22.1 | 13.5 | 0.6 |
| 60 | 43.9 | 31.1 | 25.7 | 14.4 | 1.6 |
| 80 | 47.7 | 33.6 | 27.7 | 14.9 | 2.8 |
| 110 | 51.0 | 35.6 | 29.3 | 15.3 | 4.6 |
| 150 | 53.2 | 37.0 | 30.6 | 15.6 | 6.7 |
| 210 | 53.9 | 37.8 | 31.4 | 15.6 | 9.5 |
| 300 | 55.0 | 37.8 | 31.9 | 15.9 | 11.6 |

FIG. 3c

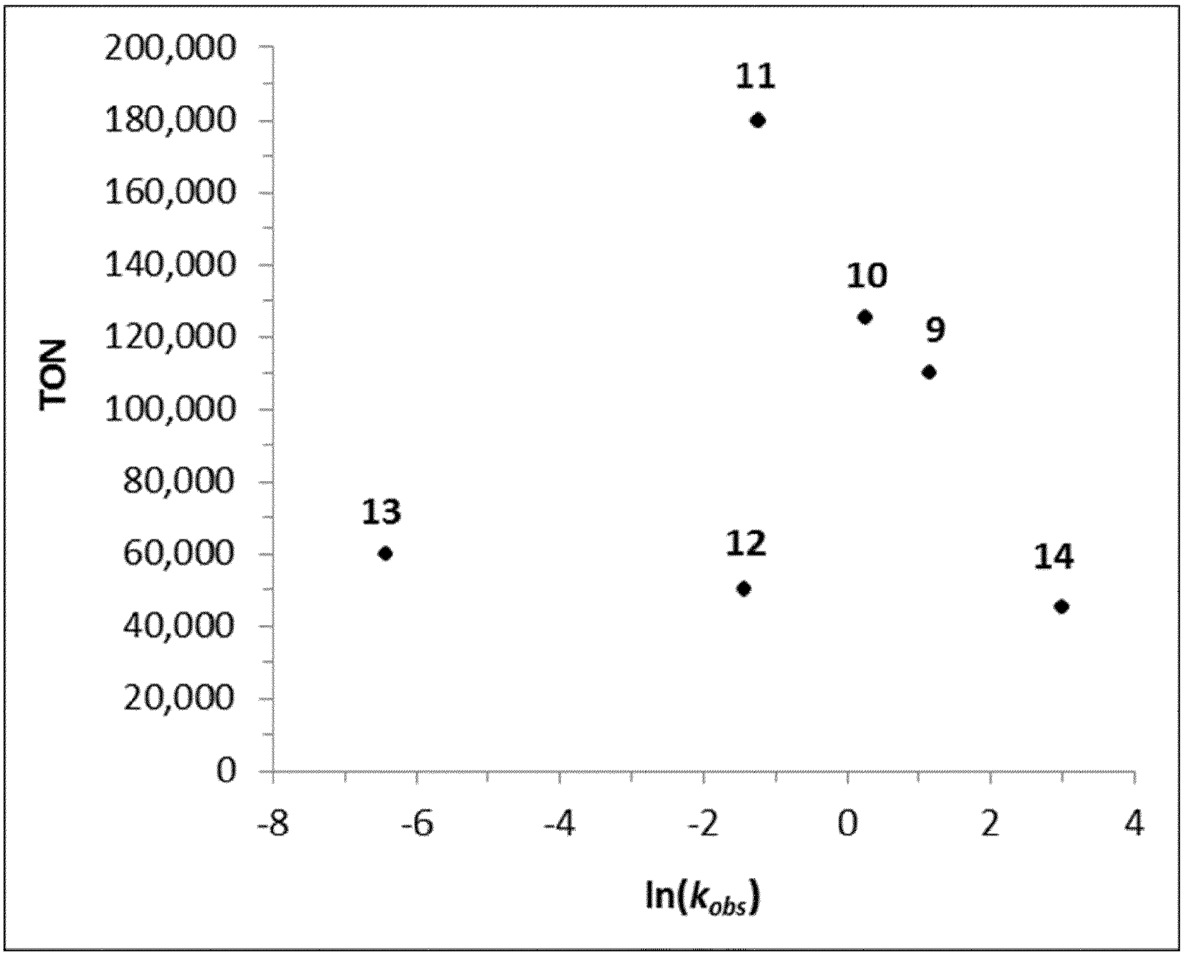
200,000
180,000
160,000
140,000
120,000
100,000
80,000
60,000
40,000
20,000
0
TON
11
10
9
13
12
14
-8
-6
-4
-2
0
2
4
ln($k_{obs}$)

FIG. 4

Europäisches Patentamt
European Patent Office
Office européen des brevets

# EUROPEAN SEARCH REPORT

Application Number
EP 21 17 9435

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2010/010290 A1 (RHODIA OPERATIONS [FR]; INST FRANCAIS DU PETROLE [FR] ET AL.) 28 January 2010 (2010-01-28) * abstract * * page 9 - page 10; example 1 * * claim 1 * ----- | 1-15 | INV. C07F9/66 C07C67/05 |
| | | | TECHNICAL FIELDS SEARCHED (IPC) |
| | | | C07C C07F |

The present search report has been drawn up for all claims

1

| Place of search | Date of completion of the search | Examiner |
|---|---|---|
| Munich | 23 September 2021 | Bissmire, Stewart |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 17 9435

This annex lists the patent family members relating to the patent documents cited in the above-mentioned European search report. The members are as contained in the European Patent Office EDP file on
The European Patent Office is in no way liable for these particulars which are merely given for the purpose of information.

23-09-2021

| Patent document cited in search report | Publication date | Patent family member(s) | Publication date |
|---|---|---|---|
| WO 2010010290 A1 | 28-01-2010 | FR 2934178 A1 | 29-01-2010 |
| | | US 2010022789 A1 | 28-01-2010 |
| | | WO 2010010290 A1 | 28-01-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011046872 A2
- WO 2011100022 A3
- WO 2010010290 A1
- WO 2008046106 A2
- US 20100145086 A, Schrodi, Y.; Pederson, R.L.; Kaido, H.; Tupy, M.J.


### Non-patent literature cited in the description


- **BIERMANN, U. ; BORNSCHEUER, U. ; MEIER, M. A. R. ; METZGER, J. O. ; SCHÄFER, H. J.** *Angew. Chem.,* 2011, vol. 123, 3938
- *Angew. Chem. Int. Ed.,* 2011, vol. 50, 3854
- **MONTERO DE ESPINOSA, L. ; MEIER, M. A. R.** *Eur. Polym. J.,* 2011, vol. 47, 837
- **MARSHALL, A.- L. ; ALAIMO, P. J.** *Chem. Eur. J.,* 2010, vol. 16, 4970
- **MEIER, M. A. R. ; METZGER, J. O. ; SCHUBERT. U. S.** *Chem. Soc. Rev.,* 2007, vol. 36, 1788
- **SHELDON, R. A.** *Green Chem.,* 2007, vol. 9, 1273
- Green Chemistry and Catalysis. Wiley-VCH. Weinheim, 2007
- **CORMA, A. ; IBORRA, S. ; VELTY, A.** *Chem. Rev.,* 2007, vol. 107, 2411
- Metathesis in Natural Product Synthesis. Wiley-VCH, 2010
- **LOZANO-VILA, A. M. ; MONSAERT, S. ; BAJEK, A. ; VERPOORT, F.** *Chem. Rev.,* 2010, vol. 110, 4865
- **VOUGIOUKALAKIS, G. ; GRUBBS, R. H.** *Chem. Rev.,* 2010, vol. 110, 1746
- **SAMOJLOWICZ, C. ; BIENIEK, M. ; GRELA, K.** *Chem. Rev.,* 2009, vol. 109, 3708
- **SCHRODI, Y. ; PEDERSON, R. L.** *Aldrichim. Acta.,* 2007, vol. 40, 45
- **NICOLAOU, K. C. ; BULGER, P. G. ; SARLAH, D.** *Angew. Chem.,* 2005, vol. 117, 4564
- *Angew. Chem. Int. Ed.,* 2005, vol. 44, 4490
- Handbook of Metathesis. Wiley-VCH, 2003, vol. 2
- **SCHROCK R. R. ; HOVEYDA A. H.** *Angew. Chem.,* 2003, vol. 115, 4740
- *Angew. Chem. Int. Ed.,* 2003, vol. 42, 4592
- **SCHROCK, R. R.** *Chem. Rev,* 2002, vol. 102, 145
- **TRNKA T. M. ; GRUBBS R. H.** *Acc. Chem. Res.,* 2001, vol. 34, 18
- **FÜRSTNER A.** *Angew. Chem.,* 2000, vol. 112, 3140
- *Angew. Chem. Int. Ed.,* 2000, vol. 39, 3012
- **PEDERSON, R. L. ; HONG, S. K.** *Clean,* 2008, vol. 36, 669-673
- **MARINESCU, S. C. ; SCHROCK, R. R. ; MÜLLER, P. ; HOVEYDA, A. H.** *J. Am. Chem. Soc.,* 2009, vol. 131, 10840-10841
- **THOMAS, R. M. ; KEITZ, B. K. ; CHAMPAGNE, T. M. ; GRUBBS, R. H.** *J. Am. Chem. Soc.,* 2011, vol. 133, 7490-7496
- **SCHRODI, Y. ; UNG, T. ; VARGAS, A. ; MKRTUMYAN, G. ; LEE, C. -W. ; CHAMPAGNE, T. ; PEDERSON, R. L. ; HONG, S. K.** *Clean.,* 2008, vol. 36, 669-673
- **CHIKKALI, S. ; MECKLING, S.** *Angew. Chem.,* 2012, vol. 124, 5902
- *Angew. Chem. Int. Ed.,* 2012, vol. 51, 5802
- **MONTERO DE ESPINOSA, L. ; MEIER, M. A. R.** *Top. Organomet. Chem.,* 2012, vol. 39, 1
- **RALUCA, M. ; DIXNEUF, P. H.** *Green Metathesis Chemistry,* 185
- Mol, J. C. Green Chem. Springer, 2002, vol. 4, 5
- **NICKEL, A. ; UNG, T. ; MKRTUMYAN, G. ; UY, J. ; LEE, C.- W. ; STOIANOVA, D. ; PAPAZIAN, J. ; WEI, W.- H. ; MALLARI, A. ; SCHRODI, Y.** *Top. Catal.,* 2012, vol. 55, 518
- **SCHRODI, Y. ; UNG, T. ; VARGAS, A. ; MKRTUMYAN, G. ; LEE, C.- W. ; CHAMPAGNE, T. M. ; PEDERSON, R. L. ; HONG, S. H.** *Clean.,* 2008, vol. 36, 669
- **ANDERSON, D. R. ; UNG, T. ; MKRTUMYAN. G. ; BERTRAND, G. ; GRUBBS, R. H. ; SCHRODI, Y.** *Organometallics,* 2008, vol. 27, 563
- **ANDERSON, D. R. ; LAVALLO, V. ; O'LEARY, D. J. ; BERTRAND, G. ; GRUBBS, R. H.** *Angew. Chem.,* 2007, vol. 119, 7400
- *Angew. Chem. Int. Ed.,* 2007, vol. 46, 7262

- **ZHANG, J. ; SONG, S. ; WANG, X. ; JIAOA, J. ; SHI, M.** *Chem. Commun.,* 2013, vol. 49, 9491
- **VAN DER KLIS, F. ; LE NÔTRE, J. ; BLAAUW, R. ; VAN HAVEREN, J. ; VAN ES, D. S.** *Eur. J. Lipid. Sci. Technol.,* 2012, vol. 114, 911
- **COHEN, S. A. ; LUETKENS, M. L. ; BALAKRISHNAN, C. ; SNYDER, R.** *Elevance Renewable Sciences*
- **THOMAS, R. M. ; KEITZ, B. K. ; CHAMPAGNE, T. ; GRUBBS, R. H.** *J. Am. Chem. Soc.,* 2011, vol. 133, 7490
- **HAGADORN, J. R. ; HOLTCAMP, M. W. ; BEDOYA, M. S.** *Exxonmobile*
- **MIGNANI, G. ; PEVERE, V. ; OLIVIER-BOURBIGOU, H. ; VALLEE, C. ; BERTHOD, M. ; CITADELLE, C.** *Rhodia Operations, IFP*
- **MARINESCU, S. C. ; SCHROCK, R. R. ; MÜLLER, P. ; HOVEYDA, A. H.** *J. Am. Chem. Soc.,* 2009, vol. 131, 10840
- **SCHRODI, Y. ; PEDERSON, R. L. ; KAIDO, H. ; TUPY, M. J.** *Elevance Renewable Sciences*
- **BURDETT, K. A. ; HARRIS, L. D. ; MARGL, P. ; MAUGHON, B. R. ; MOKHTAR-ZADEH, T. ; SAUCIER, P. C. ; WASSERMAN, E. P.** *Organometallics,* 2004, vol. 23, 2027
- **SCHRODI, Y. ; UNG, T. ; VARGAS, A. ; MKRTUMYAN, G. ; LEE, C.- W. ; CHAMPAGNE, T. M. ; PEDERSON, R. L. ; HONG, S. H.** *Clean,* 2008, vol. 36, 669
- **PATEL, J. ; MUJCINOVIC, S. ; JACKSON, W. R. ; ROBINSON, A. J. ; SERELIS, A. K. ; SUCH, C.** *Green Chem.,* 2006, vol. 8, 450
- **NICKEL, A. ; UNG, T. ; MKRTUMYAN, G. ; UY, J. ; LEE, C.- W. ; STOIANOVA, D. ; PAPAZIAN, J. ; WEI, W.- H. ; MALLARI, A. ; SCHRODI, Y.** *Top. Catal,* 2012, vol. 55, 518
- **SCHRODI, Y. ; UNG, T. ; VARGAS, A. ; MKRTUMYAN, G.; ; LEE, C.- W. ; CHAMPAGNE, T. M. ; PEDERSON, R. L. ; HONG, S. H.** *Clean,* 2008, vol. 36, 669
- **RALUCA, M. ; DIXNEUF, P. H.** Green Metathesis Chemistry. Springer, 2010, 185
- **MOL, J. C.** *Green Chem.,* 2002, vol. 4, 5
- **JAZZAR, R. ; DEWHURST, R. D. ; BOURG, J.- B ; DONNADIEU, B.; CANAC, Y. ; BERTRAND, G.** *Angew. Chem.,* 2007, vol. 119, 2957
- *Angew. Chem Int. Ed.,* 2007, vol. 46, 2899
- **BURDETT, K.A. ; HARRIS, L.D. ; MARGL, P. ; MAUGHON, B.R. ; MOKHTAR-ZADEH, T. ; SAUCIER, P.C. ; WASSERMAN, E.P.** *Organometallics,* 2004, vol. 23, 2027
- **NICKEL, A. ; UNG, T. ; MKRTUMYAN, G. ; UY, J. ; LEE, C.H. ; STOIANOVA, D. ; PAPAZIAN, J. ; WEI,W.-H. ; MALLARI, A. ; SCHRODI, Y.** *Topic in Catalysis,* 2012, vol. 55, 518-523
- **WARWEL, S. ; BRÜSE, F. ; DEMES, C. ; KUNZ, M. ; RÜSCH GEN. KLAAS M.** *Chemosphere,* 2001, vol. 43, 39
- **NDERSON, D.R. ; UNG, T. ; MKRTUMYAN, G. ; BERTRAND, G. ; GRUBBS, R.H. ; SCHRODI, Y.** *Organometallics,* 2008, vol. 27, 563
- **SCHRODI, Y. ; UNG, T. ; VARGAS, A. ; MKRTUMYAN, G. ; LEE, C.W. ; CHAMPAGNE, T.M. ; PEDERSON, R.L. ; HONG, S.H.** *Clean - Soil, Air, Water,* 2008, vol. 36, 669
- **PANGBORN, A. B. ; GIARDELLO, M. A. ; GRUBBS, R. H. ; ROSEN, R. K. ; TIMMERS, F. J.** *Organometallics,* 1996, vol. 15, 1518
- **LAVALLO, V. ; CANAC, Y. ; PRASANG, C. ; DONNADIEU, B. ; BERTRAND, G.** *Angew. Chem.,* 2005, vol. 117, 5851
- *Angew. Chem. Int. Ed.,* 2005, vol. 44, 5705
- **JAZZAR, R. ; DEWHURST, R. D. ; BOURG, J. B. ; DONNADIEU, B. ; CANAC, Y. ; BERTRAND, G.** *Angew. Chem.,* 2007, vol. 119, 2957
- *Angew. Chem. Int. Ed.,* 2007, vol. 46, 2899
- **JAZZAR, R. ; BOURG, J. B. ; DEWHURST, R. D. ; DONNADIEU, B. ; BERTRAND, G.** *J. Org. Chem.,* 2007, vol. 72, 3492
- **MIGNANI, G. ; PEVERE, V. ; OLIVIER-BOURBIGOU, H. ; VALLEE, C. ; BERTHOD, M. ; CITADELLE, C.** *Rhodia Operations, IFP*
- **JAZZAR, R. ; DEWHURST, R. D. ; BOURG, J. B. ; DONNADIEU, B. ; CANAC, Y. ; BERTRAND, G.** *Angew. Chem.,* 2007, vol. 119, 2957
- *Angew. Chem. Int. Ed.,* 2015, vol. 54, 1919-1923
- **KEITZ, B. K. ; ENDO, K. ; PATEL, P. R. ; HERBERT, M. B. ; GRUBBS, R. H.** *J. Am. Chem .Soc.,* 2012, vol. 134, 693
- **SANFORD, M. S. ; LOVE, J. A. ; GRUBBS, R. H.** *J. Am. Chem. Soc.,* 2001, vol. 123, 6543
- **RITTER, T. ; HEJL, A. ; WENZEL, A. G. ; FUNK T. W. ; GRUBBS, R. H.** *Organometallics,* 2006, vol. 25, 5740
- **NICKEL, A. ; UNG, T. ; MKRTUMYAN, G. ; UY, J. ; LEE, C.- W. ; STOIANOVA, D. ; PAPAZIAN, J. ; WEI, W.-H. ; MALLARI, A. ; SCHRODI, Y.** *Top. Catal.,* 2012, vol. 55, 518
- **HONG, S. H. ; WENZEL, A. G. ; SALGUERO, T. T. ; DAY, M. W. ; GRUBBS. R. H.** *J. Am. Chem. Soc.,* 2007, vol. 129, 7961
- **LEHMAN, S. B. ; WAGENER, K, B.** *Organometallics,* 2005, vol. 24, 1477
- **ULMAN, M. ; GRUBBS, R. H.** *J. Org. Chem.,* 1999, vol. 64, 7202
- **OCCHIPINTI, G. ; JENSEN, V. R.** *Organometallics,* 2011, vol. 30, 3522
- **MELAIMI, M. ; SOLEILHAVOUP, M. ; BERTRAND, G.** *Angew. Chem.,* 2010, vol. 122, 8992
- *Angew. Chem. Int. Ed.,* 2010
- **STEWART, I. C. ; KEITZ, B. K. ; KUHN, K. M. ; THOMAS, R. M. ; GRUBBS, R. H.** *J. Am. Chem. Soc.,* 2010, vol. 132, 8534

- **LEE, C.-W. ; GRUBBS, R. ; H. ORG.** *Lett.,* 2000, vol. 2, 2145
- **KEITZ, B. K. ; GRUBBS, R. H.** *J. Am. Chem. Soc.,* 2011, vol. 133, 16277